(19) Europäisches Patentamt
European Patent Office
Office européen des brevets

(11) EP 0 501 693 B1

(12) **EUROPEAN PATENT SPECIFICATION**

(45) Date of publication and mention of the grant of the patent:
**28.08.1996 Bulletin 1996/35**

(51) Int. Cl.[6]: **C07D 217/20**, A61K 31/47, C07D 401/06, C07D 401/12

(21) Application number: **92301467.4**

(22) Date of filing: **21.02.1992**

(54) **Isoquinoline derivatives**

Isoquinolinderivate

Dérivés d'isoquinoleine

(84) Designated Contracting States:
**AT BE CH DE DK ES FR GB GR IT LI LU MC NL PT SE**

(30) Priority: **27.02.1991 JP 55915/91**

(43) Date of publication of application:
**02.09.1992 Bulletin 1992/36**

(73) Proprietor: **BANYU PHARMACEUTICAL CO., LTD.**
**Chuo-ku Tokyo 103 (JP)**

(72) Inventors:
- **Ishikawa, Kiyofumi**
  **Chofu-shi, Tokyo 182 (JP)**
- **Hayama, Takashi**
  **Kawasaki-shi, Kanagawa 211 (JP)**
- **Nishikibe, Masaru**
  **Urayasu-shi, Chiba 279 (JP)**
- **Yano, Mitsuo**
  **Yokohama-shi, Kanagawa 240 (JP)**

(74) Representative: **Votier, Sidney David**
**CARPMAELS & RANSFORD**
**43, Bloomsbury Square**
**London WC1A 2RA (GB)**

(56) References cited:
**WO-A-90/02119** **US-A- 4 613 606**

- **CHEMICAL ABSTRACTS, vol. 61, no. 2, July 20, 1964, column 1829c-1830d, Columbus, Ohio, US; I. KAZUYOSHI et al.: "Influence of substituents on the direction of ring-closure in the Bischler-Napieralski isoquinoline synthesis" & YAKUGAKU ZASSHI, 84(4), 329-33(1964)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.13.4/3.4

## Description

BACKGROUND OF THE INVENTION

Field of the Invention

The present invention relates to new isoquinoline derivatives having an anti-arrhythmic activity and bradycardiac activity and their medical use. The compounds of the present invention can be utilized as therapeutic agents in the medical field, especially, as agents for the treatment of arrhythmia, myocardiac infarction or angina pectoris.

Statement of the Related Art

As compounds having a selective bradycardiac activity, there are known alinidine, benzazepine derivatives (UL-FS49) [cf., Drugs of the Future, 10, 639 (1985)]. Specification WO 90/02119 describes 1,2,3,4-tetrahydroisoquinoline derivatives and their use as anti-tumour effect potentiators. However, it has not been reported that isoquinoline derivatives analogous to the compounds of the present invention would have a bradycardiac activity and anti-arrhythimic activity.

Currently, heart diseases have been a serious problem as clinical causes for death. It has thus been desired to develop an excellent agent for the treatment of heart diseases.

SUMMARY OF THE INVENTION

Accordingly, an object of the present invention is to provide a new isoquinoline derivative having an anti-arrhythimic activity and bradycardiac activity without adversely affecting blood pressure which is useful for the treatment of heart diseases, particularly, arrhythmia, myocardiac infarction or angina pectoris.

Another object of the present invention is to provide the medical use of the isoquinoline derivative.

According to the present invention, there is provided a compound represented by general formula (I):

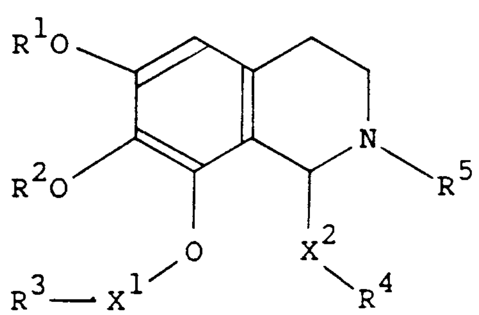

(I)

wherein each of $R^1$ and $R^2$ independently represents a lower alkyl group or both are combined together to form a methylene group;

$X^1$ represents a diva lent alkylene chain having 1 to 5 carbon atoms which may be substituted with a lower alkyl group (one optional methylene group in which alkylene chain may be replaced by one group selected from the group consisting of an oxy group, a thio group, a sulfinyl group, a sulfonyl group or a group shown by formula: $-NR^6-$ wherein $R^6$ represents a hydrogen atom or a lower alkyl group); provided that the said methylene-group is not the methylene group adjacent to the oxygen atom at the 8-position of the isoquinoline ring;

$X^2$ represents a divalent alkylene chain having 1 to 4 carbon atoms which may be substituted with a lower alkyl group;

each of $R^3$ and $R^4$ represents independently an aryl group or a heteroaryl group, each of which groups may be substituted with 1 to 3 substituents, which may be the same or different and are selected from the group consisting of a lower alkyl group, a lower alkoxy group, a methylenedioxy group, a halogen atom, a nitro group, a hydroxy group, a cyano group, a lower alkoxycarbonyl group, a lower alkanoyl group, an amino group, an N-mono-lower alkylamino group, an N,N-di-lower alkylamino group, a carbamoyl group, an N-mono-lower alkylcarbamoyl group, an N,N-di-lower alkylcarbamoyl group, an amino-lower alkyl group, an N-mono-lower alkylamino-lower alkyl group, an N,N-di-lower alkylamino-lower alkyl group, an N-(hydroxy-lower alkyl)amino-lower alkyl group, an N-lower-alkyl-N-(hydroxy-lower alkyl)amino-lower alkyl group, an N,N-di(hydroxy-lower alkyl)amino-lower alkyl group, an N-(lower alkoxy-lower

alkyl)amino-lower alkyl group, an N-lower alkyl-N-(lower alkoxy-lower alkyl)amino-lower alkyl group, an N,N-di(lower alkoxy-lower alkyl)amino-lower alkyl group and a nitrogen-containing saturated heterocyclic lower alkyl group; and

$R^5$ represents a lower alkoxycarbonyl group, a lower alkylsulfonyl group, a group shown by formula:

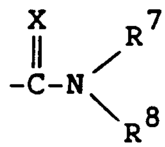

wherein each of $R^7$ and $R^8$ independently represents a hydrogen atom or a lower alkyl group which may be substituted with 1 or 2 substituents, which may be the same or different and are selected from the group consisting of a hydroxy group and a lower alkoxy group, or both are combined together with the nitrogen atom adjacent thereto to form a saturated nitrogen-containing heterocyclic group; and X represents an oxygen atom or a sulfur atom; or

a lower alkanoyl group which may be substituted with 1 or 2 substituents, which may be the same or different and are selected from the group consisting of a lower alkylsulfinyl group, a group shown by formula: $R^9S$- wherein $R^9$ represents a hydrogen atom, a lower alkyl group, a lower alkanoyl group, a carbamoyl group, an N-mono-lower alkylcarbamoyl group, an N,N-di-lower alkylcarbamoyl group or a lower alkoxycarbonyl group; a group shown by formula: $R^{10}O$- wherein $R^{10}$ represents a hydrogen atom, a lower alkyl group or a lower alkanoyl group which may be substituted with a hydroxy group; and a group shown by formula:

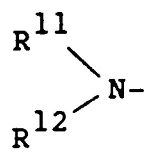

wherein each $R^{11}$ and $R^{12}$ independently represents a hydrogen atom, a lower alkyl group or a lower alkanoyl group, or both are combined together to form a 5- to 7-membered nitrogen-containing saturated heterocyclic ring having 3 to 6 carbon atoms together with the nitrogen atom adjacent thereto, wherein one methylene group not adjacent to the nitrogen atom for forming the ring may be replaced by an oxy group or a thio group and one methylene group adjacent to the nitrogen atom may be replaced by a carbonyl group,

and a pharmacentically acceptable salt thereof.

DETAILED DESCRIPTION OF PREFERRED EMBODIMENTS

Various terms referred to throughout the specification and embraced in the scope of the invention are defined below and specific examples are also given below.

The term "lower" is used to mean that the group or compound expressed by the term has carbon atoms of 6 or less, unless otherwise indicated. Therefore, the term "lower alkyl group" refers to a straight or branched alkyl group having 1 to 6 carbon atoms. Specific examples of the lower alkyl group include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethyl-propyl, 1-ethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

Examples of the "lower alkoxy group" include methoxy, ethoxy, propoxy, isopropoxy, butoxy, isobutoxy, sec-butoxy, tert-butoxy, pentyloxy, isopentyloxy, neopentyloxy, tert-pentyloxy, hexyloxy and isohexyloxy.

Examples of the "aryl group" include phenyl and naphthyl.

The term "heteroaryl group" is used to mean a monocyclic heteroaryl group containing hetero atom(s) such as an oxygen atom, a sulfur atom and a nitrogen atom. Examples of the heteroaryl group include pyridyl, furyl, thienyl, pyrrolyl and thiazolyl.

Examples of the "halogen atom" include a fluorine atom, a chlorine atom, a bromine atom and an iodine atom.

Examples of the "lower alkoxycarbonyl group" include methoxycarbonyl, ethoxycarbonyl, propoxy-carbonyl, isopropoxycarbonyl, butoxycarbonyl, isobutoxycarbonyl, sec-butoxycarbonyl, tert-butoxy-carbonyl and pentyloxycarbonyl.

Examples of the "lower alkanoyl group" include formyl, acetyl, propionyl, butyryl, valeryl, pivaloyl and hexanoyl.

The term "N-mono-lower alkylamino group" is used to mean an amino group substituted with one lower alkyl group defined as described above and specific examples include N-methylamino, N-ethylamino, N-propylamino, N-isopropylamino and N-butylamino.

The term "N,N-di-lower alkylamino group" is used to mean an amino group substituted with two lower alkyl groups defined as described above which may be the same or different. Specific examples include N,N-dimethylamino, N-ethyl-N-methylamino, N,N-diethylamino, N-ethyl-N-propylamino, N,N-dipropylamino and N,N-dibutylamino.

The term "N-mono-lower alkylcarbamoyl group" is used to mean a carbamoyl group substituted with one lower alkyl group defined as described above. Specific examples include N-methylcarbamoyl, N-ethylcarbamoyl, N-propylcarbamoyl and N-butylcarbamoyl.

The term "N,N-di-lower alkylcarbamoyl group" is used to mean a carbamoyl group substituted with two lower alkyl groups defined as above which may be the same or different. Specific examples include N,N-dimethylcarbamoyl, N-ethyl-N-methylcarbamoyl, N,N-diethylcarbamoyl and N-ethyl-N-propylcarbamoyl.

The term "amino-lower alkyl group" is used to mean a lower alkyl group defined above which is substituted with an amino group. Specific examples include aminomethyl, 1-aminoethyl, 2-aminoethyl, 1-aminopropyl, 2-aminopropyl, 3-aminopropyl, 1-amino-1-methylethyl, 2-amino-1-methylethyl, 1-aminobutyl, 2-aminobutyl, 3-aminobutyl and 4-aminobutyl.

The term "N-mono-lower alkylamino-lower alkyl group" is used to mean a lower alkyl group as defined above which is substituted with the N-mono-lower alkylamino group defined above. Specific examples include N-methylaminomethyl, 1-(N-methylamino)ethyl, 2-(N-methylamino)ethyl, 1-(N-methylamino)propyl, 2-(N-methylamino)propyl, 3-(N-methylamino)propyl, N-ethylaminomethyl, 1-(N-ethylamino)ethyl, 2-(N-ethyl-amino)ethyl, 1-(N-ethylamino)propyl, N-propylaminomethyl and N-isopropylaminomethyl.

The term "N,N-di-lower alkylamino-lower alkyl group" is used to mean a lower alkyl group as defined above which is substituted with the N,N-di-lower alkylamino group as described above. Specific examples include N,N-dimethylaminomethyl, 1-(N,N-dimethylamino)ethyl, 2-(N,N-dimethylamino)ethyl, 1-(N,N-dimethyl-amino)propyl, 2-(N,N-dimethylamino)propyl, 3-(N,N-dimethylamino)propyl, N-ethyl-N-methylaminomethyl, 1-(N-ethyl-N-methylamino)ethyl, 2-(N-ethyl-N-methylamino)ethyl, N,N-diethylaminomethyl, 1-(N,N-diethylamino)ethyl, 2-(N,N-diethylamino)ethyl, N-ethyl-N-propylaminomethyl, N,N-dipropylaminomethyl and N,N-diisopropylaminomethyl.

The term "hydroxy-lower alkyl group" is used to mean a lower alkyl group defined as above which is substituted with one hydroxy group at the position other than 1-position thereof. Specific examples include 2-hydroxyethyl, 2-hydroxypropyl and 3-hydroxypropyl.

The term "N-(hydroxy-lower alkyl)amino-lower alkyl group" is used to mean a lower alkyl group as defined above which has an amino group substituted with one hydroxy-lower alkyl group as defined above. Specific examples include N-(2-hydroxyethyl)aminomethyl, 1-(N-(2-hydroxyethyl)amino)ethyl, 2-(N-(2-hydroxyethyl)amino)ethyl, N-(2-hydroxypropyl)aminomethyl and N-(3-hydroxypropyl)aminomethyl.

The term "N-lower alkyl-N-(hydroxy-lower alkyl)amino-lower alkyl group" is used to mean a lower alkyl group as defined above which contains an amino group substituted with the lower alkyl group defined as above and the hydroxy-lower alkyl group defined as above. Specific examples include N-(2-hydroxyethyl)-N-methylaminomethyl, 1-(N-(2-hydroxyethyl)-N-methylamino)ethyl, 2-(N-(2-hydroxyethyl)-N-methylamino)ethyl, and 3-(N-(2-hydroxyethyl)-N-methylamino)propyl.

The term "N,N-di(hydroxy-lower alkyl)amino-lower alkyl group" is used to mean a lower alkyl group as defined above which contains an amino group substituted with the two hydroxy-lower alkyl groups defined above which may be the same or different. Specific examples include N,N-di(2-hydroxyethyl)aminomethyl, 2-(N,N-di(2-hydroxyethyl)amino)ethyl, 3-(N,N-di(2-hydroxyethyl)amino)propyl and N-2-hydroxyethyl-N-3-hydroxypropylaminomethyl.

The term "lower alkoxy-lower alkyl group" is used to mean a lower alkyl group as defined above in which the hydrogen atom other than the 1-position is replaced by one lower alkoxy group as defined above. Specific examples include 2-methoxyethyl, 2-methoxypropyl and 3-methoxypropyl,

The term "N-(lower alkoxy-lower alkyl)amino-lower alkyl group" is used to mean a lower alkyl group as defined above which contains an amino group substituted with one lower alkoxy-lower alkyl group defined as described above. Specific examples include N-(2-methoxyethyl)aminomethyl, 1-(N-(2-methoxyethyl)amino)ethyl, 2-(N-(2-methoxyethyl)amino)ethyl, N-(3-methoxypropyl)aminomethyl and N-(2-ethoxyethyl)aminomethyl.

The term "N-lower alkyl-N-(lower alkoxy-lower alkyl)amino-lower alkyl group" is used to mean a lower alkyl group as defined above which contains an amino group substituted with the lower alkyl group defined above and the lower alkoxy-lower alkyl group defined above. Specific examples include N-(2-methoxyethyl)-N-methylaminomethyl, 1-(N-(2-methoxyethyl)-N-methyl-amino)ethyl, 2-(N-(2-methoxyethyl)-N-methylamino)ethyl and N-ethyl-N-(2-methoxyethyl)aminomethyl.

The term "N,N-di(lower alkoxy-lower alkyl)-amino-lower alkyl group" is used to mean a lower alkyl group as defined above which contains an amino group substituted with two lower alkoxy-lower alkyl groups defined above which may be the same or different. Specific examples include N,N-di(2-methoxyethyl)aminomethyl, 2-(N,N-di(2-methoxyethyl)amino)ethyl, 3-(N,N-di(2-methoxyethyl)aminopropyl and N-(2-methoxyethyl)-N-(3-methoxypropyl)aminomethyl.

The term "nitrogen-containing saturated heterocyclic lower alkyl group" is used to mean a lower alkyl group as defined above which contains a 5- to 9-membered saturated heterocyclic ring having 3 to 8 carbon atoms which has at least one nitrogen atom in the ring thereof and the methylene group not adjacent to the nitrogen atom may be replaced by oxy, thio, sulfinyl or sulfonyl. Specific examples include pyrrolidinomethyl, pyrrolidin-2-ylmethyl, N-methylpyrrolidin-2-ylmethyl, 1-pyrrolidinoethyl, 2-pyrrolidinoethyl, 2-(pyrrolidin-2-yl)ethyl, 2-(N-methylpyrrolidin-2-yl)ethyl, 1,3-thiazolidin-3-ylmethyl, 1,3-thiazolidin-2-ylmethyl, N-methyl-1,3-thiazolidin-2-ylmethyl, 1-(1,3-thiazolidin-3-yl)ethyl, 2-(1,3-thiazolidin-3-yl)ethyl, 2-(N-methyl-1,3-thiazolidin-2-yl)ethyl, piperidinomethyl, piperidin-2-ylmethyl, N-methylpiperidin-2-ylmethyl, 1-piperidinoethyl, 2-piperidinoethyl, 2-(piperidin-2-yl)ethyl, 2-(N-methylpiperidin-2-yl)ethyl, morpholinomethyl, morpholin-3-ylmethyl, N-methylmorpholin-3-ylmethyl, 1-morpholinoethyl, 2-morpholinoethyl, 2-(morpholin-3-yl)ethyl, 2-(N-methylmorpholin-3-yl)ethyl, perhydro-1,4-thiazin-4-ylmethyl, perhydro-1,4-thiazin-3-ylmethyl, perhydro-N-methyl-1,4-thiazin-3-ylmethyl, 1-(perhydro-1,4-thiazin-4-yl)ethyl, 2-(perhydro-1,4-thiazin-4-yl)ethyl, 2-(perhydro-1,4-thiazin-3-yl)ethyl, 2-(perhydro-N-methyl-1,4-thiazin-3-yl)ethyl, perhydro-1,4-thiazin-1-oxid-4-ylmethyl, perhydro-1,4-thiazin-1-oxid-3-ylmethyl, perhydro-N-methyl- 1,4-thiazin-1-oxid-3-ylmethyl, 1-(perhydro-1,4-thiazin-1-oxid-4-yl)ethyl, 2-(perhydro-1,4-thiazin-1-oxid-4-yl)ethyl, perhydro-1,4-thiazin-1,1-dioxid-4-ylmethyl, 2-(perhydro-1,4-thiazin-1,1-dioxid-4-yl)ethyl, piperazin-1-ylmethyl, 2-(piperazin-1-yl)ethyl, N-methylpiperazin-1-ylmethyl, 2-(N-methylpiperazin-1-yl)ethyl, N,N′-dimethylpiperazin-2-ylmethyl, 2-(N,N′-dimethylpiperazin-2-yl)ethyl, perhydroazepin-1-ylmethyl, 1-(perhydroazepin-1-yl)ethyl, perhydroazocin-1-ylmethyl and perhydroazonin-1-ylmethyl.

Examples of the "lower alkylsulfonyl group" include mesyl, ethylsulfonyl, propylsulfonyl, isopropylsulfonyl, butylsulfonyl, isobutylsulfonyl, sec-butylsulfonyl, tert-butylsulfonyl and pentylsulfonyl.

Examples of the "lower alkylsulfinyl group" include methylsulfinyl, ethylsulfinyl, propylsulfinyl, isopropylsulfinyl and butylsulfinyl.

Examples of the "lower alkylthio group" include methylthio, ethylthio, propylthio, isopropylthio, butylthio, isobutylthio and sec-butylthio.

Specific examples of the group shown by formula:

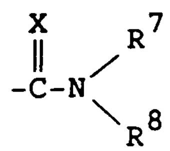

wherein each of $R^7$ and $R^8$ independently represents a hydrogen atom or a lower alkyl group which may be substituted with 1 or 2 substituents, which may be the same or different and are selected from the group consisting of a hydroxy group and a lower alkoxy group, or both $R^7$ and $R^8$ are combined together with the nitrogen atom adjacent thereto to form a saturated nitrogen-containing heterocyclic group; and X represents an oxygen atom or a sulfur atom, include carbamoyl, N-methylcarbamoyl, N-ethylcarbamoyl, N-(2-hydroxyethyl)carbamoyl, N-(2-methoxyethyl)carbamoyl, N-(2-hydroxypropyl)carbamoyl, N-(2-methoxypropyl)carbamoyl, N-N-dimethylcarbamoyl, N-ethyl-N-methylcarbamoyl, N-(2-hydroxyethyl)-N-methylcarbamoyl, N-(2-methoxyethyl)-N-methylcarbamoyl, N,N-diethylcarbamoyl, N,N-dipropylcarbamoyl, N-ethyl-N-(2-hydroxyethyl)carbamoyl, N-ethyl-N-(2-methoxyethyl)carbamoyl, N,N-di(2-hydroxyethyl)carbamoyl, N,N-di(2-methoxyethyl)carbamoyl, pyrrolidinocarbonyl, piperidinocarbonyl, morpholinocarbonyl, thiocarbamoyl, N-methylthiocarbamoyl, N-ethylthiocarbamoyl, N-(2-hydroxyethyl)thiocarbamoyl, N-(2-methoxyethyl)-thiocarbamoyl, N-(2-methoxypropyl)thiocarbamoyl, N,N-dimethylthiocarbamoyl, N-ethyl-N-methylthiocarbamoyl, N-(2-hydroxyethyl)-N-methylthiocarbamoyl, N-(2-methoxyethyl)-N-methylthiocarbamoyl, N,N-diethylthiocarbamoyl and pyrrolidinothiocarbonyl.

Specific examples of the group shown by formula:

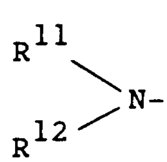

wherein each $R^{11}$ and $R^{12}$ independently represents a hydrogen atom, a lower alkyl group or a lower alkanoyl group, or both are combined together to form a 5- to 7-membered nitrogen-cntaining saturated heterocyclic ring having 3 to 6 carbon atoms together with the nitrogen atom adjacent thereto, wherein one methylene group not adjacent to the nitrogen atom for forming the ring may be replaced by an oxy group or a thio group and one methylene group adjacent to the nitrogen atom may be replaced by a carbonyl group,

include amino, N-methylamino, N,N-dimethylamino, N-ethyl-N-methylamino, N,N-diethylamino, N-acetylamino, N-acetyl-N-methylamino, N-propionylamino, N-methyl-N-propionylamino, 1,3-thiazolidin-3-yl, pyrrolidino, piperidino, morpholino, perhydro-1,4-thiazin-4-yl, perhydroazepin-1-yl, 2-pyrrolidon-1-yl and 2-piperidon-1-yl.

Examples of the "divalent alkylene chain having 1 to 5 carbon atoms which may be substituted with a lower alkyl group (one optional methylene group in which alkylene chain may be replaced by one group selected from the group consisting of an oxy group, a thio group, a sulfinyl group, a sulfonyl group or a group shown by formula: $-NR^6-$ wherein $R^6$ represents a hydrogen atom or a lower alkyl group); provided that the said methylene group is not the methylene group adjacent to the oxygen atom at the 8-position of the isoquinoline ring" include
$-CH_2-$, $-CH(CH_3)-$, $-CH(CH_2-CH_3)-$,
$-(CH_2)_2-$, $-CH(CH_3)-CH_2-$, $-CH_2-CH(CH_3)-$, $-O-CH_2-$,
$-S-CH_2-$, $-N(CH_3)-CH_2-$,
$-(CH_2)_3-$, $-CH(CH_3)-(CH_2)_2-$, $-CH_2-CH(CH_3)-CH_2-$,
$-(CH_2)_2-CH(CH_3)-$, $-O-(CH_2)_2-$, $-S-(CH_2)_2-$, $-SO-(CH_2)_2-$,
$-SO_2-(CH_2)_2-$, $-NH-(CH_2)_2-$, $-NCH_3-(CH_2)_2-$,
$-O-CH(CH_3)-CH_2-$, $-S-CH(CH_3)-CH_2-$, $-SO-CH(CH_3)-CH_2-$,
$-SO_2-CH(CH_3)-CH_2-$, $-NH-CH(CH_3)-CH_2-$, $-N(CH_3)-CH(CH_3)-CH_2-$,
$-(CH_2)_4-$, $-CH(CH_3)-(CH_2)_3-$, $-CH_2-CH(CH_3)-(CH_2)_2-$,
$-(CH_2)_2-CH(CH_3)-CH_2-$, $-(CH_2)_3-CH(CH_3)-$,
$-O-(CH_2)_3-$, $-S-(CH_2)_3-$, $-SO-(CH_2)_3-$, $-SO_2-(CH_2)_3-$,
$-NH-(CH_2)_3-$, $-NCH_3-(CH_2)_3-$,
$-CH_2-O-(CH_2)_2-$, $-CH_2-S-(CH_2)_2-$, $-CH_2-SO-(CH_2)_2-$,
$-CH_2-SO_2-(CH_2)_2-$, $-CH_2-NH-(CH_2)_2-$, $-CH_2-N(CH_3)-(CH_2)_2-$,
$-(CH_2)_2-O-CH_2-$, $-(CH_2)_2-S-CH_2-$, $-(CH_2)_2-N(CH_3)-CH_2-$,
$-O-CH(CH_3)-(CH_2)_2-$, $-S-CH(CH_3)-(CH_2)_2-$,
$-N(CH_3)-CH(CH_3)-(CH_2)_2-$,
$-(CH_2)_5-$, $-CH(CH_3)-(CH_2)_4-$, $-(CH_2)_4-CH(CH_3)-$,
$-O-(CH_2)_4-$, $-S-(CH_2)_4-$, $-SO-(CH_2)_4-$, $-SO_2-(CH_2)_4-$,
$-NH-(CH_2)_4-$, $-NCH_3-(CH_2)_4-$,
$-CH_2-O-(CH_2)_3-$, $-CH_2-S-(CH_2)_3-$, $-CH_2-SO-(CH_2)_3-$,
$-CH_2-SO_2-(CH_2)_3-$, $-CH_2-NH-(CH_2)_3-$, $-CH_2-N(CH_3)-(CH_2)_3-$.

Examples of the "divalent alkylene chain having 1 to 4 carbon atoms which may be substituted with a lower alkyl group" include
$-CH_2-$, $-CH(CH_3)-$, $-CH(CH_2-CH_3)-$,
$-(CH_2)_2-$, $-CH(CH_3)-CH_2-$, $-CH_2-CH(CH_3)-$,
$-(CH_2)_3-$, $-CH(CH_3)-(CH_2)_2-$, $-CH_2-CH(CH_3)-CH_2-$,
$-(CH_2)_2-CH(CH_3)-$,
$-(CH_2)_4-$, $-CH(CH_3)-(CH_2)_3-$, $-CH_2-CH(CH_3)-(CH_2)_2-$,
$-(CH_2)_2-CH(CH_3)-CH_2-$, $-(CH_2)_3-CH(CH_3)-$.

Preferred compounds of the present invention are the isoquinoline derivatives of formula (I), wherein each of $R^1$ and $R^2$ independently represents a lower alkyl group or both are combined together to form a methylene group; $R^4$ represents a phenyl group wherein 1 to 3 optional hydrogen atoms on the benzene ring may be replaced by 1 to 3 substituents selected from the group consisting of a lower alkoxy group and a methylenedioxy group, or a pyridyl group; $X^2$ represents $CH^2$ or $CH^2CH^2$; $R^3$ represents a group represented by formula:

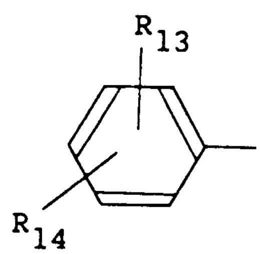

wherein $R^{13}$ represents a hydrogen atom or a lower alkoxy group; and $R^{14}$ represents an amino group, an N-mono-lower alkylamino group or an N,N-di-lower alkylamino group or represents a group shown by formula:

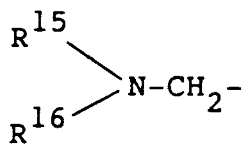

wherein each of $R^{15}$ and $R^{16}$ independently represents a hydrogen atom or a lower alkyl group wherein a hydrogen atom on the carbon atom not adjacent to the nitrogen atom may be replaced by a hydroxy group or a lower alkoxy group; or both $R^{15}$ and $R^{16}$ are combined together with the nitrogen atom adjacent thereto to form a 5- to 7-membered nitrogen-containing saturated heterocyclic ring having 3 to 6 carbon atoms and in this case, one optional methylene group not adjacent to the nitrogen atom may be replaced by one group selected from the group consisting of oxy, thio, sulfinyl, sulfonyl or a group shown by formula: $-NR^{17}-$ wherein $R^{17}$ represents a hydrogen atom or a lower alkyl group, or

a pyridyl group wherein 1 or 2 hydrogen atoms on the pyridine ring may be replaced by 1 or 2 substituents selected from the group consisting of a lower alkoxy group and an N,N-di-lower alkylaminomethyl group.

Processes for preparing the compounds (I) of the present invention are explained below.

The compounds of the present invention represented by general formula (I) described above can be prepared by alkanoylation, alkoxycarbonylation, carbamoylation or sulfonylation of 1,2,3,4-tetrahydroisoquinolin-8-ol derivatives (II) represented by general formula (II):

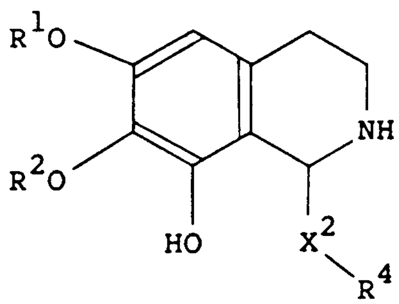

(II)

wherein $R^1$, $R^2$, $X^2$ and $R^4$ are as described above;
and then condensing the resulting derivatives (III):

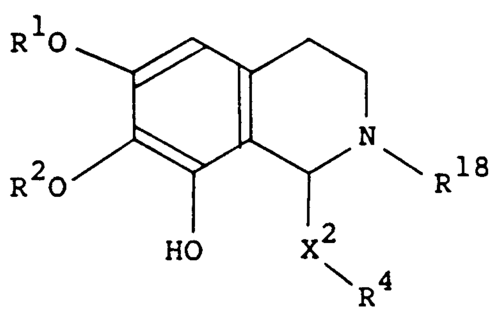

(III)

wherein $R^1$, $R^2$, $X^2$ and $R^4$ have the same significations as described above; and $R^{18}$ represents a lower alkoxycarbonyl group, a lower alkylsulfonyl group, a group represented by formula:

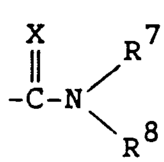

wherein $R^7$ and $R^8$ have the same significances as described above, or a lower alkanoyl group wherein the lower

alkanoyl group may be substituted with 1 or 2 substituents selected from the group consisting of a lower alkylsulfinyl group, a lower alkylthio group, a hydroxy group, a lower alkoxy group and an N,N-di-lower alkylamino group, on the hydroxy group at the 8-position of the isoquinoline ring, with compounds (IV) represented by general formula (IV):

$$R^3\text{-}X^1\text{-}Y \qquad (IV)$$

wherein $R^3$ and $X^1$ are as described above; and Y represents a splitting off group which is generally replacable by an aryloxy group,
in the presence of a base (referred to as Process 1).

Alternatively, the compounds of the present invention can be prepared by performing substitution of one halogen atom in $\alpha,\alpha'$-dihaloxylenes (o-, m-, p-) with the compounds (III) in a manner similar to Process 1 and then substituting another halogen atom remained with primary or secondary amines (referred to as Process 2).

Furthermore, compounds obtained in Process 1 or Process 2 in which $R^{18}$ is formyl can be converted into the desired compounds by subjecting the formyl compounds to alkali hydrolysis to remove the formyl, if necessary, then subjecting the resulting derivatives (V):

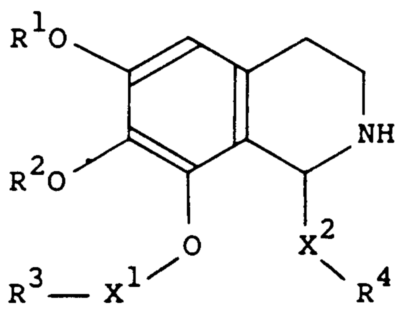
(V)

wherein $R^1$, $R^2$, $R^3$, $R^4$, $X^1$ and $X^2$ have the same significances as described above,
to alkanoylation, alkoxycarbonylation, carbamoylation or sulfonylation at the 2-position of the isoquinoline ring, and further where a suitable splitting off group is present on the introduced substituent of the compounds obtained by the alkanoylation, substituting the splitting off group with nucleophilic agents such as thiol compounds, primary or secondary amines and amide compounds (referred to as Process 3). Where the compounds obtained by process 3 contain an acylthio group, the acylthio group may be converted into a mercapto group, if desired, by alkali hydrolysis (referred to as Process 4). Where the compounds obtained by processes 1 to 3 contain a nitro group in $R^3$ and/or $R^4$, the nitro group may be converted into a primary amino group using an appropriate reducing agent (referred to as Process 5). Where the compounds obtained by Processes 1 to 3 contain a thio group, the thio group may be converted into a sulfinyl group or a sulfonyl group using an appropriate oxidizing agent, if necessary (Process 6).

Hereafter these processes are described more specifically.

Process 1

The alkanoylation of Compound (II) at the 2-position of the isoquinoline ring can be performed by reacting a carboxylic acid reactive derivative (for example, an acid halide, an acid anhydride, an activated amide, an activated ester) corresponding to the alkanoyl group to be introduced, with (II) in an appropriate solvent (for example, chloroform, dichloromethane, dimethylformamide, tetrahydrofuran, 1,4-dioxane) at 0°C to room temperature, if necessary, in the presence of an appropriate basic for example, triethylamine, pyridine, 4-N,N-dimethylaminopyridine). The alkoxycarbonylation and sulfonylation may be conducted in a similar manner by reacting (II) with an alkoxycarbonyl halide or a sulfonyl halide corresponding to the group to be introduced. The carbamoylation may be effected either by reacting (II) with a carbamoyl halide corresponding to the group to be introduced, in the same manner as in the alkanoylation, or by reacting isocyanate or isothiocyanate with (II) at 0°C to room temperature in an appropriate solvent (for example, chloroform, dichloromethane, tetrahydrofuran, 1,4-dioxane).

The condensation of Compound (III) with Compound (IV) is carried out preferably in an appropriate solvent. Examples of the solvent used are acetone, acetonitrile, methanol, 1,4-dioxane, tetrahydrofuran, dimethylsulfoxide and dimethylformamide. Examples of the base used in the reaction include inorganic bases such as alkali metal carbonates, alkali metal hydrogencarbonate, alkali metal hydroxides, alkali metal hydrides and alkali metal alkoxides. In general, the reaction can be carried out by dissolving Compound (III) in the solvent described above, adding a suitable base to the solution, further adding Compound (IV) and then reacting the mixture at 0°C to 80°C.

Process 2

The condensation of Compound (III) with α,α′-dihaloxylenes (o-, m-, p-) is carried out in the same manner as in Process 1. The substitution of one halogen atom remained on the group introduced in the resulting compound may be performed by adding an excess amount of a primary or secondary amine to be introduced in an appropriate solvent (for example, methanol, ethanol, 1,4-dioxane, dimethylformamide or dimethylsulfoxide) and reacting them at 0°C to the boiling point of the solvent.

Process 3

The synthesis of Compound (V) via deformylation by alkali hydrolysis can be performed by adding 1 equivalent to an excess amount of alkali metal hydroxide aqueous solution to the starting compound in an appropriate solvent (methanol, ethanol or 1,4-dioxane) and reacting them at room temperature to the boiling point of the solvent. The alkanoylation, alkoxy-carbonylation, carbamoylation and sulfonylation may be performed in a manner similar to Process 1. Where the alkanoyl group has a splitting off group, the introduction of the thiol compound by substitution of this splitting off group can be performed by reacting the thiol compound with the starting compound in a suitable solvent (for example, methanol, ethanol, chloroform, tetrahydrofuran, dimethylformamide or dimethyl sulfoxide) at 0°C to the boiling point of the solvent, if necessary, in the presence of an appropriate base (for example, an organic base such as triethylamine and pyridine; an inorganic base such as an alkali metal hydroxide, an alkali metal carbonate, an alkali metal hydrogencarbonate, an alkali metal hydride and an alkali metal alkoxide).

The introduction of the primary or secondary amine can be performed as in Process 2. The introduction of the amide compound may be carried out by reacting the amide compound with the starting compound in an appropriate solvent (tetrahydrofuran, dimethylformamide or 1,4-dioxane) at 0°C to the boiling point of the solvent in the presence of an appropriate base (for example, an alkali metal hydride).

Process 4

The conversion of the acylthio group into the mercapto group by alkali hydrolysis may be conducted by adding an aqueous solution of an appropriate base (for example, an alkali metal hydroxide, an alkali metal carbonate, an alkali metal hydrogencarbonate, ammonia) in an appropriate solvent (for example, methanol, ethanol, 1,4-dioxane) to the starting compound and then reacting them at 0°C to the boiling point of the solvent.

Process 5

The conversion of the nitro group to the primary amino group through reduction may be carried out by adding tin (II) chloride to the starting compound in an appropriate solvent (for example, ethanol or ethyl acetate) and reacting them at 60°C to the boiling point of the solvent.

Process 6

The conversion of the thio group into the sulfinyl group or sulfonyl group by oxidation may be carried out by adding an appropriate oxidizing agent (for example, oxone, m-chloroperbenzoic acid, hydrogen peroxide) to the starting compound in an appropriate solvent (for example, chloroform, dichloromethane, acetic acid or acetone) and reacting them at 0°C to 60°C. An amount of the oxidizing agent used is in the range of 1 to 1.2 equivalents in the case of sulfinylation and in the range of 2 to 3 equivalents in the case of sulfonylation, based on the starting compound having the thio group.

Compounds (I) obtained by these processes contain stereoisomers based on the presence of asymmetric carbon at the 1-position of the isoquinoline ring. Where the starting Compound (II) is a racemic mixture, the racemic mixture is subjected to optical resolution using an appropriate optically active acid in a conventional manner and the respective processes described above are applied to the products, whereby the respective stereoisomers may be synthesized. As the appropriate acid used for the optical resolution, there are (+)-tartaric acid, (-)-tartaric acid, (+)-dibenzoyltartaric acid and (-)-dibenzoyltartaric acid. The starting material, 1,2,3,4-tetrahydroisoquinoline derivatives [II] may be synthesized by the known method (see PCT/JP89/00825, WO90/02119). That is, Compound [II] may be prepared from phenethylamines via Bischler-Napieralski reaction.

The starting Compound (IV) may be synthesized by converting the hydroxy group of aminoalcohols obtained by known methods [for example, see Japanese Patent Application KOKAI (Laid-Open) No. 55-53247, J. Med. Chem., 27, 1047 (1984)] into a splitting off group in a conventional manner. That is, Compound (IV) may be synthesized either by reacting with a reagent such as thionyl chloride, thionyl bromide, phosphorus pentachloride, phosphorus oxychloride, etc. in the absence of any solvent or in an appropriate solvent (for example, chloroform or dichloromethane) thereby to

convert into the halide or by reacting with a reagent such as methanesulfonyl chloride and p-toluenesulfonyl chloride to convert into the mesylate or tosylate.

Preferred compounds of the present invention are listed in the following Table.

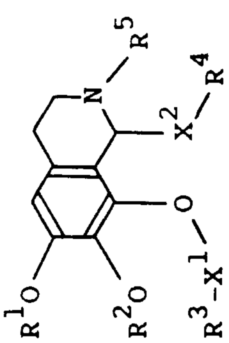

(I)

| Compound No. | $R^1$ | $R^2$ | $R^3$ | $R^4$ | $R^5$ | $X^1$ | $X^2$ |
|---|---|---|---|---|---|---|---|
| 1 | $CH_3-$ | $CH_3-$ | 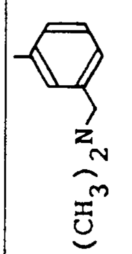 | 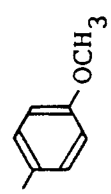 | -CHO | $-CH_2-$ | $-CH_2-$ |
| 2 | $CH_3-$ | $CH_3-$ | 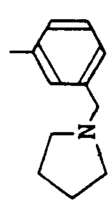 | 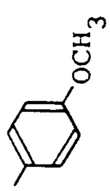 | -CHO | $-CH_2-$ | $-CH_2-$ |
| 3 | $CH_3-$ | $CH_3-$ | 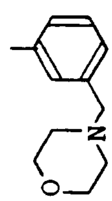 | 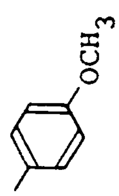 | -CHO | $-CH_2-$ | $-CH_2-$ |
| 4 | $CH_3-$ | $CH_3-$ | 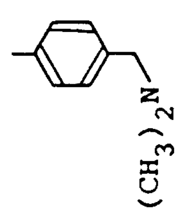 | 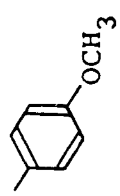 | -CHO | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 5 | $CH_3-$ | $CH_3-$ | 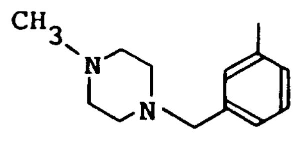 | 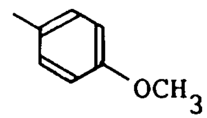 | -CHO | $-CH_2-$ | $-CH_2-$ |
| 6 | $CH_3-$ | $CH_3-$ | Ph- | 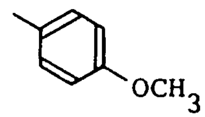 | -CHO | $-CH_2NCH_2CH_2CH_2-$<br>$CH_3$ (on N) | $-CH_2-$ |
| 7 | $CH_3-$ | $CH_3-$ | 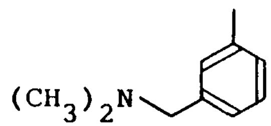 | 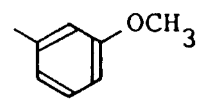 | -CHO | $-CH_2-$ | $-CH_2-$ |
| 8 | $CH_3-$ | $CH_3-$ | 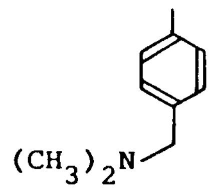 | 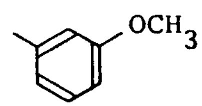 | -CHO | $-CH_2-$ | $-CH_2-$ |
| 9 | $CH_3-$ | $CH_3-$ | 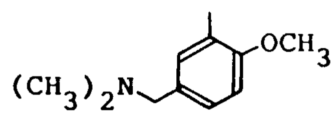 | 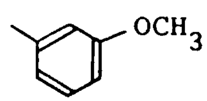 | -CHO | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 10 | $CH_3-$ | $CH_3-$ | 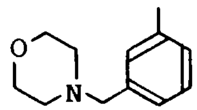 | 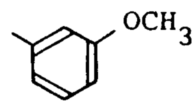 | -CHO | $-CH_2-$ | $-CH_2-$ |
| 11 | $CH_3-$ | $CH_3-$ | 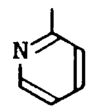 | 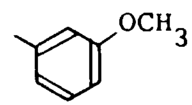 | -CHO | $-CH_2-$ | $-CH_2-$ |
| 12 | $CH_3-$ | $CH_3-$ | 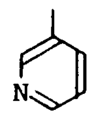 | 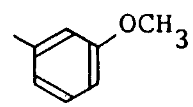 | -CHO | $-CH_2-$ | $-CH_2-$ |
| 13 | $CH_3-$ | $CH_3-$ | 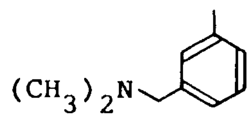 | 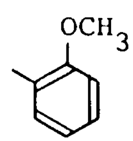 | -CHO | $-CH_2-$ | $-CH_2-$ |
| 14 | $CH_3-$ | $CH_3-$ | 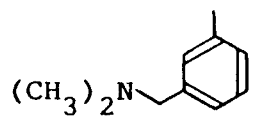 | 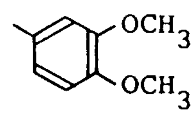 | -CHO | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 15 | $CH_3-$ | $CH_3-$ | 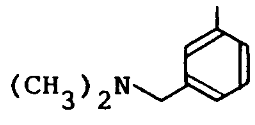 | 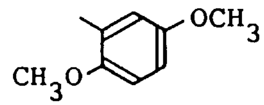 | -CHO | $-CH_2-$ | $-CH_2-$ |
| 16 | $CH_3-$ | $CH_3-$ | 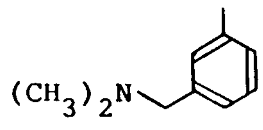 | 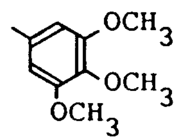 | -CHO | $-CH_2-$ | $-CH_2-$ |
| 17 | $CH_3-$ | $CH_3-$ | 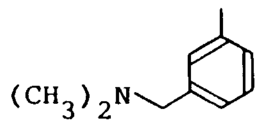 | 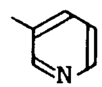 | -CHO | $-CH_2-$ | $-CH_2-$ |
| 18 | $CH_3-$ | $CH_3-$ | 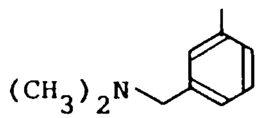 | 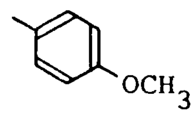 | -CHO | $-CH_2-$ | $-CH_2CH_2-$ |
| 19 | $-CH_2-$ | | 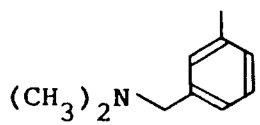 | 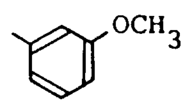 | -CHO | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 20 | $-CH_2-$ | | 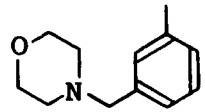 | 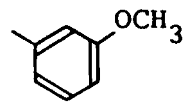 | -CHO | $-CH_2-$ | $-CH_2-$ |
| 21 | $CH_3-$ | $CH_3-$ | Ph- | 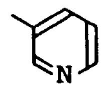 | -CHO | $-S(CH_2)_4-$ | $-CH_2-$ |
| 22 | $CH_3-$ | $CH_3-$ | 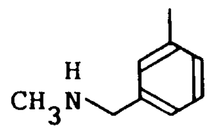 | 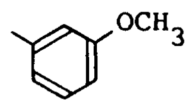 | -CHO | $-CH_2-$ | $-CH_2-$ |
| 23 | $CH_3-$ | $CH_3-$ | 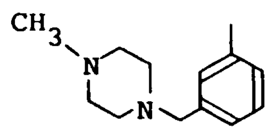 | 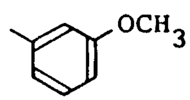 | -CHO | $-CH_2-$ | $-CH_2-$ |
| 24 | $CH_3-$ | $CH_3-$ | 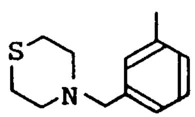 | 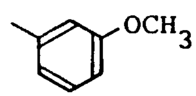 | -CHO | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 25 | $CH_3-$ | $CH_3-$ | 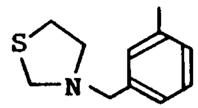 | 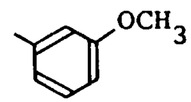 | -CHO | $-CH_2-$ | $-CH_2-$ |
| 26 | $CH_3-$ | $CH_3-$ | 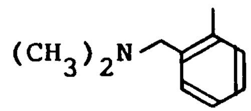 | 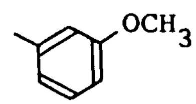 | -CHO | $-CH_2-$ | $-CH_2-$ |
| 27 | $CH_3-$ | $CH_3-$ | 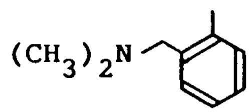 | 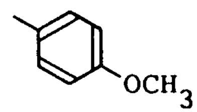 | -CHO | $-CH_2-$ | $-CH_2-$ |
| 28 | $CH_3-$ | $CH_3-$ | 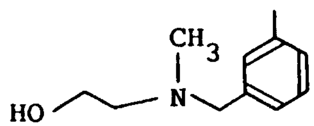 | 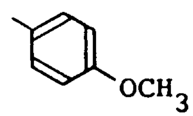 | -CHO | $-CH_2-$ | $-CH_2-$ |
| 29 | $CH_3-$ | $CH_3-$ | 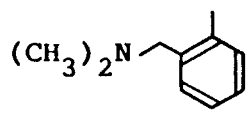 | 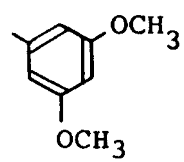 | -CHO | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 30 | $CH_3-$ | $CH_3-$ | 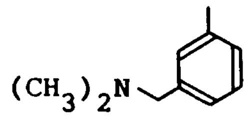 | 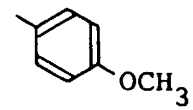 | $-COCH_2OH$ | $-CH_2-$ | $-CH_2-$ |
| 31 | $CH_3-$ | $CH_3-$ | 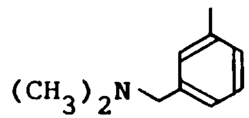 | 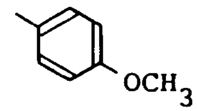 | $-CO(CH_2)_2OH$ | $-CH_2-$ | $-CH_2-$ |
| 32 | $CH_3-$ | $CH_3-$ | 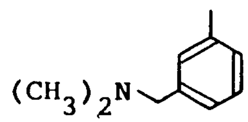 | 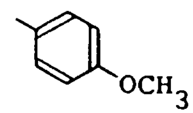 | $-COCH(OH)CH_3$ | $-CH_2-$ | $-CH_2-$ |
| 33 | $CH_3-$ | $CH_3-$ | 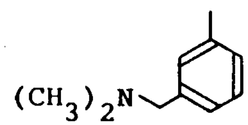 | 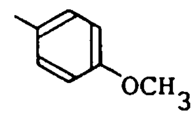 | $-COCH_2OCH_3$ | $-CH_2-$ | $-CH_2-$ |
| 34 | $CH_3-$ | $CH_3-$ | 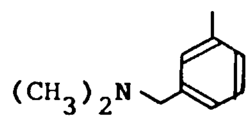 | 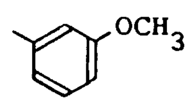 | $-COCH_2OH$ | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 35 | $CH_3-$ | $CH_3-$ | 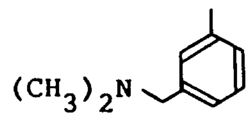 | 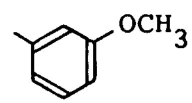 | $-CO(CH_2)_2OH$ | $-CH_2-$ | $-CH_2-$ |
| 36 | $CH_3-$ | $CH_3-$ | 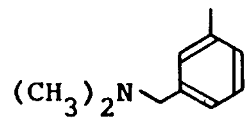 | 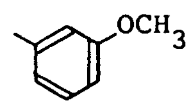 | $-COCH_2OCH_3$ | $-CH_2-$ | $-CH_2-$ |
| 37 | $CH_3-$ | $CH_3-$ | 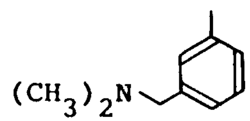 | 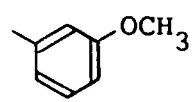 | $-COCH_2N(CH_3)COCH_3$ | $-CH_2-$ | $-CH_2-$ |
| 38 | $CH_3-$ | $CH_3-$ | 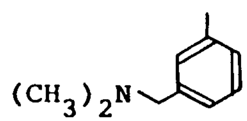 | 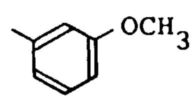 | $-SO_2CH_3$ | $-CH_2-$ | $-CH_2-$ |
| 39 | $CH_3-$ | $CH_3-$ | 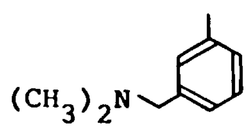 | 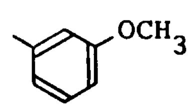 | $-COCH_3$ | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 40 | $CH_3-$ | $CH_3-$ | 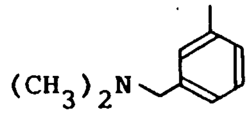 | 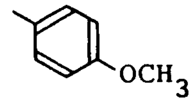 | $-CONHCH_3$ | $-CH_2-$ | $-CH_2-$ |
| 41 | $CH_3-$ | $CH_3-$ | 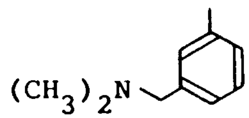 | 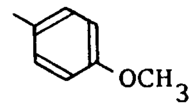 | $-CSNHCH_3$ | $-CH_2-$ | $-CH_2-$ |
| 42 | $CH_3-$ | $CH_3-$ | 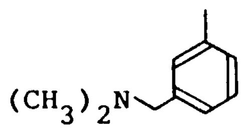 | 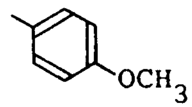 | $-CONH(CH_2)_2OH$ | $-CH_2-$ | $-CH_2-$ |
| 43 | $CH_3-$ | $CH_3-$ | 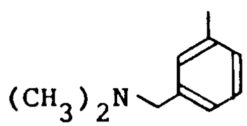 | 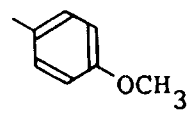 | $-CON(CH_3)_2$ | $-CH_2-$ | $-CH_2-$ |
| 44 | $CH_3-$ | $CH_3-$ | 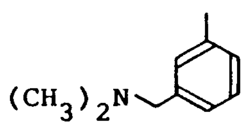 | 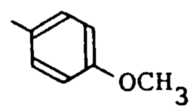 | $-CONH_2$ | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 45 | $CH_3-$ | $CH_3-$ | 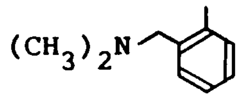 | 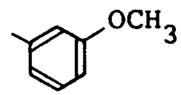 | $-CONHCH_3$ | $-CH_2-$ | $-CH_2-$ |
| 46 | $CH_3-$ | $CH_3-$ | 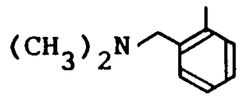 | 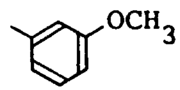 | $-COCH_2OH$ | $-CH_2-$ | $-CH_2-$ |
| 47 | $CH_3-$ | $CH_3-$ | 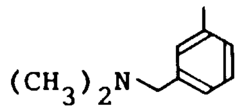 | 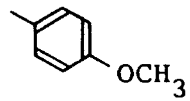 | $-COCH_2SCOCH_3$ | $-CH_2-$ | $-CH_2-$ |
| 48 | $CH_3-$ | $CH_3-$ | 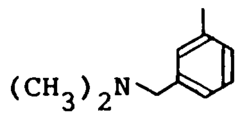 | 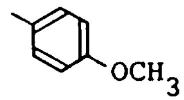 | $-COCH_2SCH_3$ | $-CH_2-$ | $-CH_2-$ |
| 49 | $CH_3-$ | $CH_3-$ | 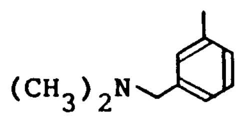 | 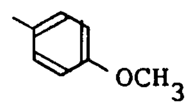 | 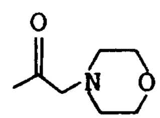 | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 50 | $CH_3-$ | $CH_3-$ | 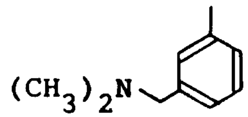 | 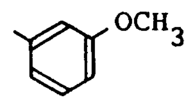 | $-COCH_2SCH_3$ | $-CH_2-$ | $-CH_2-$ |
| 51 | $CH_3-$ | $CH_3-$ | 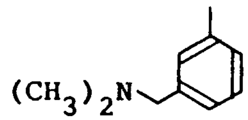 | 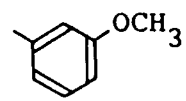 | $-COCH_2SCOCH_3$ | $-CH_2-$ | $-CH_2-$ |
| 52 | $CH_3-$ | $CH_3-$ | 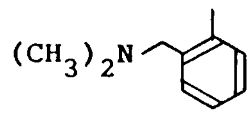 | 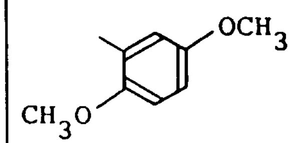 | $-COCH_2SCOCH_3$ | $-CH_2-$ | $-CH_2-$ |
| 53 | $-CH_2-$ | | 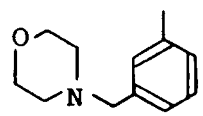 | 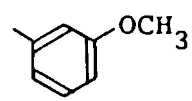 | $-COCH_2SCOCH_3$ | $-CH_2-$ | $-CH_2-$ |
| 54 | $CH_3-$ | $CH_3-$ | 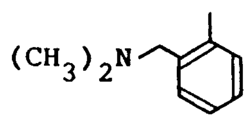 | 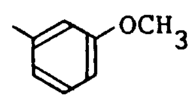 | 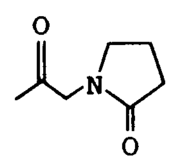 | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 55 | $CH_3$- | $CH_3$- | 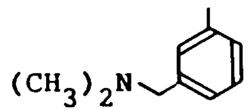 | 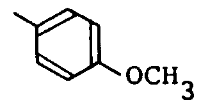 | $-CO_2CH_3$ | $-CH_2-$ | $-CH_2-$ |
| 56 | $CH_3$- | $CH_3$- | 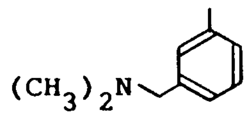 | 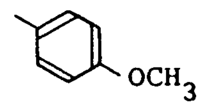 | $-SO_2CH_3$ | $-CH_2-$ | $-CH_2-$ |
| 57 | $CH_3$- | $CH_3$- | 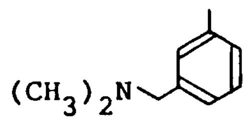 | 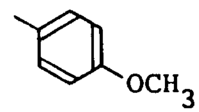 | $-COCH_3$ | $-CH_2-$ | $-CH_2-$ |
| 58 | $CH_3$- | $CH_3$- | 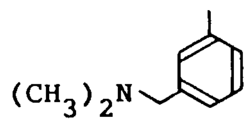 | 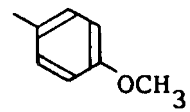 | $-COCH_2N(CH_3)_2$ | $-CH_2-$ | $-CH_2-$ |
| 59 | $CH_3$- | $CH_3$- | 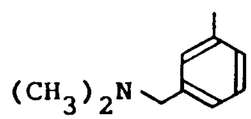 | 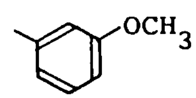 | $-CO_2CH_3$ | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 60 | $CH_3$- | $CH_3$- | 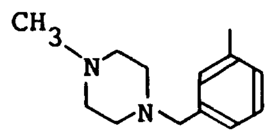 | 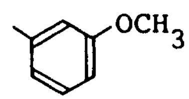 | $-CO_2CH_3$ | $-CH_2-$ | $-CH_2-$ |
| 61 | $CH_3$- | $CH_3$- | 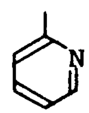 | 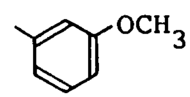 | $-CONHCH_3$ | $-CH_2-$ | $-CH_2-$ |
| 62 | $CH_3$- | $CH_3$- | 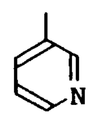 | 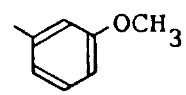 | $-CONHCH_3$ | $-CH_2-$ | $-CH_2-$ |
| 63 | $CH_3$- | $CH_3$- | 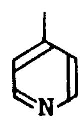 | 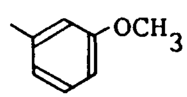 | $-CONHCH_3$ | $-CH_2-$ | $-CH_2-$ |
| 64 | $CH_3$- | $CH_3$- | 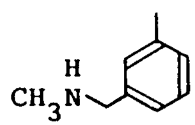 | 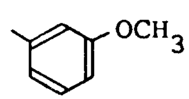 | $-CONHCH_3$ | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 65 | $CH_3-$ | $CH_3-$ | 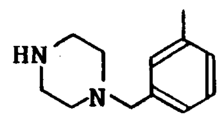 | 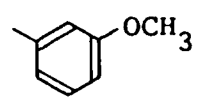 | $-CONHCH_3$ | $-CH_2-$ | $-CH_2-$ |
| 66 | $CH_3-$ | $CH_3-$ | 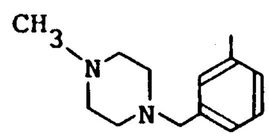 | 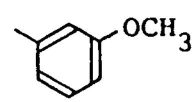 | $-CONHCH_3$ | $-CH_2-$ | $-CH_2-$ |
| 67 | $CH_3-$ | $CH_3-$ | 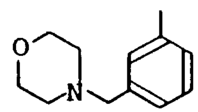 | 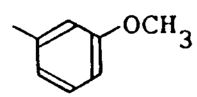 | $-CONHCH_3$ | $-CH_2-$ | $-CH_2-$ |
| 68 | $CH_3-$ | $CH_3-$ | 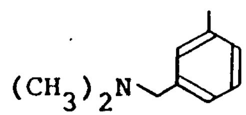 | 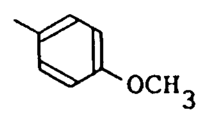 | $-COCH_2OCOCH_3$ | $-CH_2-$ | $-CH_2-$ |
| 69 | $CH_3-$ | $CH_3-$ | 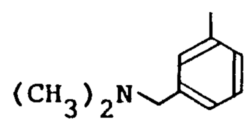 | 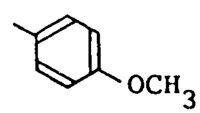 | $-COCH_2OCOCH_2OH$ | $-CH_2-$ | $-CH_2-$ |

| Compound No. | $R^1$ | $R^2$ | $R^3$ | $R^4$ | $R^5$ | $X^1$ | $X^2$ |
|---|---|---|---|---|---|---|---|
| 70 | $CH_3-$ | $CH_3-$ | 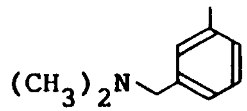 | 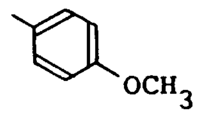 | $-COCH_2SH$ | $-CH_2-$ | $-CH_2-$ |
| 71 | $CH_3-$ | $CH_3-$ | 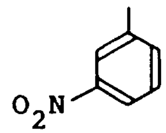 | 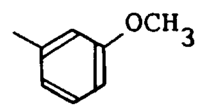 | $-CHO$ | $-CH_2-$ | $-CH_2-$ |
| 72 | $CH_3-$ | $CH_3-$ | 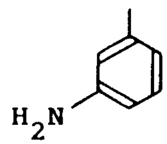 | 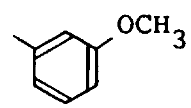 | $-CHO$ | $-CH_2-$ | $-CH_2-$ |
| 73 | $CH_3-$ | $CH_3-$ | 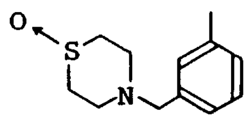 | 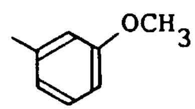 | $-CHO$ | $-CH_2-$ | $-CH_2-$ |
| 75 | $CH_3-$ | $CH_3-$ | 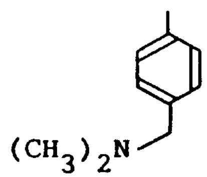 | 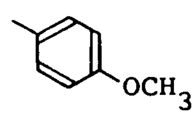 | $-COCH_2OH$ | $-CH_2-$ | $-CH_2-$ |

The pharmacological activities of the compounds according to the present invention are described below.

Effect on ventricular fibrillation and mortality induced by coronary artery occlusion and reperfusia in rats:

Under anesthesia with pentobarbital, male Spraque Dawley (SD) rats were thoracotomized. The compounds of the present invention were studied with respect to the influence on ventricular fibrillation and mortality induced by reperfusion after ligation of the left coronary artery for 5 minutes. At the same time, blood pressure and heart rate were determined through a catheter inserted into the carotid artery. Each test compound was intravenously administered 2 minutes prior to the ligation. The results are shown in Table 1. The hydrochloride of Compound 1 was intraduodenally administered 30 minutes before the ligation. The results are shown in Table 2.

Table 1

| Effect on ventricular fibrillation and mortality induced by reperfusion after coronary artery ligation in rats (intravenous administration) | | | | | | |
|---|---|---|---|---|---|---|
| Test Compound | Dose (mg/kg) | Number of animals | Incident rate of ventricular fibrillation (%) | Mortality rate (%) | Change in blood pressure (%) | Change in heart rate (%) |
| Control | | 5 | 100 | 100 | 6.3 | -6.8 |
| Cpd. 1 | 1 | 5 | 20 | 0 | 1.8 | -15.0 |
| Cpd. 15 | 1 | 5 | 20 | 0 | 1.9 | -9.9 |
| Cpd. 17 | 1 | 5 | 0 | 0 | 10.0 | -12.5 |
| Cpd. 18 | 1 | 5 | 60 | 20 | 3.7 | -13.3 |
| Cpd. 33 | 1 | 5 | 40 | 20 | 7.2 | -11.4 |
| Cpd. 40 | 1 | 5 | 40 | 0 | -0.5 | -11.5 |
| Cpd. 43 | 1 | 5 | 20 | 0 | 6.8 | -11.6 |
| Cpd. 46 | 1 | 5 | 20 | 20 | -5.2 | -21.3 |
| Cpd. 49 | 1 | 5 | 0 | 0 | 1.0 | -9.3 |
| Cpd. 50 | 1 | 5 | 60 | 40 | 5.2 | -13.8 |
| Cpd. 52 | 1 | 5 | 20 | 0 | -8.8 | -15.6 |
| Cpd. 55 | 1 | 5 | 40 | 20 | 6.4 | -11.5 |
| Cpd. 58 | 1 | 5 | 0 | 0 | -4.0 | -7.9 |
| Cpd. 59 | 1 | 5 | 0 | 0 | 1.3 | -16.5 |
| Cpd. 67 | 1 | 5 | 40 | 40 | 2.6 | -11.7 |
| Cpd. 70 | 1 | 5 | 20 | 20 | -8.5 | -18.1 |
| Lidocaine | 1 | 5 | 100 | 100 | -4.7 | -12.3 |

As shown in Table 1, the compounds of the present invention prevent the occurrence of ventricular fibrillation and mortality rate in the dose of 1.0 mg/kg and the effects were more potent than those with Lidocaine. In this case, a remarkable bradycardiac activity was noted. As demonstrated above, the compounds of the present invention are effective for the conditions induced by reperfusion after coronary artery ligation.

Table 2

| Effect on ventricular fibrillation and mortality induced by reperfusion after coronary artery ligation in rats (intraduodenal administration) | | | | |
|---|---|---|---|---|
| Test Compound | Dose (mg/kg) | Number of animals | Incident rate of ventricular fibrillation (%) | Mortality rate (%) |
| Control | | 6 | 100 | 50 |
| Cpd. 1 hydrochloride | 10 | 6 | 33.3 | 0 |
| | 50 | 6 | 0 | 0 |

As shown in Table 2, Compound 1 hydrochloride at doses of 10 and 50 mg/kg dose-dependently prevented ventricular fibrillation and mortality rate induced by coronary artery occlusion and reperfusion after intraduodenal administration in rats. This result indicates that intraduodenal administration of the compound of the present invention is effective for the conditions induced by reperfusion after coronary artery ligation.

Effect on ventricular fibrillation induced in dog by reperfusion after coronary artery ligation in dogs:

Under anesthesia with pentobarbital, both sexs beagle dogs were thoracotomized. The compounds of the present invention were studied with respect to the influence on ventricular fibrillation and mortality induced by reperfusion after ligation of the left coronary artery for 30 minutes. Test compound (Compound 1 hydrochloride) and Verapamil were intravenously administered 5 minutes prior to the ligation. The results are shown in Table 3.

Table 3

| Effect on ventricular fibrillation in dog induced by reperfusion after coronary artery ligation in dogs | | | |
|---|---|---|---|
| Test Compound | Dose (mg/kg) | Number of animals | Incident rate of ventricular fibrillation (%) |
| Control | | 10 | 90 |
| Cpd. 1 Hydrochloride | 1.0 | 10 | 30 |
| Verapamil | 0.1 | 10 | 50 |

As shown in Table 1, Compound 1 hydrochloride markedly inhibited ventricular fibrillation in the dose of 1.0 mg/kg. The effect was more potent than that of Verapamil at the dose of 0.1 mg/kg. As demonstrated above, the compound of the present invention is also effective for the conditions induced in dog by reperfusion after coronary artery ligation in dogs.

The foregoing results of the pharmacological studies reveal that the compounds of the present invention exhibit the anti-arrhythmic activity and bradycardiac activity. Therefore, the present invention can provide an effective method for the treatment of arrhythmia, myocardiac infarction and angina pectoris.

As mode for application of the anti-arrhythmic and bradycardiac agents according to the present invention, various forms can be selected depending upon purposes. There are, for example, oral preparations such as tablets, capsules, powders, granules, liquid or elixir, etc. and parenteral preparations such as sterilized liquid forms, e.g., liquid or suspension, etc.

Solid preparations may be prepared in the form of tablets, capsules, granules or powders as they are but appropriate additives may also be used for the preparations. Examples of such additives include sugars such as lactose, glucose, etc.; starch of corn, wheat, rice, etc.; fatty acids such as stearic acid, etc.; inorganic salts such as magnesium metasilicate aluminate, anhydrous calcium phosphate, etc.; synthetic high molecular substances such as polyvinylpyrrolidone, polyalkylene glycol, etc.; fatty acid salts such as calcium stearic acid, magnesium stearate, etc.; alcohols such as stearyl alcohol or benzyl alcohol, etc.; synthetic cellulose derivatives such as methyl cellulose, carboxy methylcellulose, ethyl cellulose, hydroxypropylmethyl cellulose, etc.; and other additives conventionally used, such as water, gelatin, talc, vegetable oils, gum arabic, etc.

In general, these capsule, tablet, granule and powder preparations contain the effective ingredient in an amount of 0.1 to 100 wt%, preferably 1 to 100 wt%.

The liquid preparation is prepared in the form of a suspension, syrup or injection, using appropriate additives generally used in liquid preparations, using water, alcohols or vegetable oils such as soybean oil, peanut oil or sesame oil, etc.

As suitable solvents in the case of parenteral administration such as intramuscular injection, intravenous injection or subcutaneous injection, there are, for example, distilled water for injection, Lidocaine hydrochloride solution (for intramuscular injection), physiological saline, glucose aqueous solution, ethanol, liquid for intravenous injection (for example, aqueous solution of citric acid and sodium citrate, etc.) or electrolyte solution (for drip injection and intravenous injection), etc. or a mixture thereof.

These injections may be previously dissolved or may also be in such a form that they are provided in the form of powders by adding appropriate additives thereto and are dissolved upon use. These injections contain the effective ingredient generally in the range of 0.005 to 10 wt%, preferably in the range of 0.05 to 5 wt%.

The liquid preparation for oral administration such as a suspension or syrup contains the effective ingredient in the range of 0.1 to 50 wt%.

The dose of the anti-arrhythmic, anti-myocardiac infarction or anti-angina pectoris agent of the present invention may vary depending upon age, health state, body weight or conditions of the patient but its daily dose is in the range of 0.01 to 1 mg/kg for parenteral administration and in the range of 0.1 to 10 mg/kg for oral administration, for adult.

Hereafter the present invention is described in more detail by referring to the examples but is not deemed to limited thereto.

REFERENTIAL EXAMPLE 1

Preparation of 2-formyl-6,7-dimethoxy-1-(4-methoxybenzyl)-1,2,3,4-tetrahydroisoquinolin-8-ol (intermediate 1)

To a suspension of 6,7-dimethoxy-1-(4-methoxybenzyl)-1,2,3,4-tetrahydroisoquinolin-8-ol (2.26 g, 6.86 mmole) in chloroform (60 ml) was added formic trimethylacetic anhydride (1.16 g, 8.91 mmole) at 0°C. The mixture was stirred for 30 minutes and concentrated under reduced pressure. The residue was recrystallized from ethyl acetate-hexane to give intermediate 1 (2.23 g) as colorless crystals.

m.p.: 171-173°C
FAB-MS(m/z, $(C_{20}H_{23}NO_5+H)^+$): 358

Intermediates 2~10 (Referential Examples 2~10) were prepared using the corresponding 1,2,3,4-tetrahydro-isoquinoline derivatives, according to the reaction in Referential Example 1.

REFERENTIAL EXAMPLE 2

2-Formyl-6,7-dimethoxy-1-(3-methoxybenzyl)-1,2,3,4-tetrahydroisoquinolin-8-ol (intermediate 2)

m.p.: 163-167°C
FAB-MS(m/z,$(C_{20}H_{23}NO_5+H)^+$): 358

REFERENTIAL EXAMPLE 3

2-Formyl-6,7-dimethoxy-1-(2-methoxybenzyl)-1,2,3,4-tetrahydroisoquinolin-8-ol (intermediate 3)

m.p.: 156-157°C
FAB-MS(m/z, $(C_{20}H_{23}NO_5+H)^+$): 358

REFERENTIAL EXAMPLE 4

2-Formyl-6,7-dimethoxy-1-(3,4-dimethoxybenzyl)-1,2,3,4-tetrahydroisoquinolin-8-ol (intermediate 4)

m.p.: 155-157°C
FAB-MS(m/z, $(C_{21}H_{25}NO_6+H)^+$): 388

REFERENTIAL EXAMPLE 5

2-Formyl-6,7-dimethoxy-1-(2,5-dimethoxybenzyl)-1,2,3,4-tetrahydroisoquinolin-8-ol (intermediate 5)

m.p.: 143.0-144.5°C
FAB-MS(m/z, $(C_{21}H_{25}NO_6+H)^+$): 388

REFERENTIAL EXAMPLE 6

2-Formyl-6,7-dimethoxy-1-(3,4,5-trimethoxybenzyl)-1,2,3, 4-tetrahydroisoquinolin-8-ol (intermediate 6)

m.p.: 190-195°C
FAB-MS(m/z, $(C_{22}H_{27}NO_7+H)^+$): 418

REFERENTIAL EXAMPLE 7

2-Formyl-6,7-dimethoxy-1-(3-pyridylmethyl)-1,2,3,4-tetrahydroisoquinolin-8-ol (intermediate 7)

m.p.: 199-200°C
FAB-MS(m/z, $(C_{18}H_{20}N_2O_4+H)^+$): 329

REFERENTIAL EXAMPLE 8

2-Formyl-6,7-dimethoxy-1-(4-methoxyphenethyl)-1,2,3,4-tetrahydroisoquinolin-8-ol (intermediate 8)

m.p.: 151.0-152.5°C
FAB-MS(m/z, $(C_{21}H_{25}NO_5+H)^+$): 372

REFERENTIAL EXAMPLE 9

2-Formyl-1-(3-methoxybenzyl)-6,7-methylenedioxy-1,2,3,4-tetrahydroisoquinolin-8-ol (intermediate 9)

m.p.: 205-207°C
FAB-MS(m/z, $(C_{19}H_{19}NO_5+H)^+$): 342

REFERENTIAL EXAMPLE 10

2-Formyl-6,7-dimethoxy-1-(3,5-dimethoxybenzyl)-1,2,3,4-tetrahydroisoquinolin-8-ol (intermediate 10)

m.p.: 176.5-178.0°C
FAB-MS(m/z, $(C_{21}H_{25}NO_6+H)^+$): 388

REFERENTIAL EXAMPLE 11

(1) Preparation of (-)-6,7-dimethoxy-1-(4-methoxybenzyl)-1,2,3,4-tetrahydroisoquinolin-8-ol

(-)-6,7-Dimethoxy-1-(4-methoxybenzyl)-1,2,3,4-tetrahydroisoquinolin-8-ol (+)-dibenzoyl-D-tartrate was obtained from (±)-6,7-dimethoxy-1-(4-methoxybenzyl)-1, 2,3,4-tetrahydroisoquinolin-8-ol and (+)-dibenzoyl-D-tartaric acid by a conventional method. The salt was purified by repeated recrystallizations from chloroform-acetone-methanol (10:6:1).

m.p.: 173.0-174.0°C (dec)
Optical Rotation: $[\alpha]_D^{25}$ =+58.3° (C=0.336, methanol)

The salt was treated with a saturated aqueous sodium hydrogencarbonate solution to give the captioned compound as colorless crystals.

Optical Rotation: $[\alpha]_D^{25}$ =-15.8° (C=0.419, chloroform)

(2) Preparation of (+)-6,7-dimethoxy-1-(4-methoxybenzyl)-1,2,3,4-tetrahydroisoquinolin-8-ol

The combined mother liquors of recrystallization in the above (1) were concentrated and the residue was treated with a saturated aqueous sodium hydrogen-carbonate solution to give crude (+)-6,7-dimethoxy-1-(4-methoxybenzyl)-1,2,3,4-tetrahydroisoquinolin-8-ol. The compound was treated with (-)-dibenzoyl-L-tartaric acid to give (+)-6,7-dimethoxy-1-(4-methoxybenzyl)-1,2,3,4-tetrahydroisoquinolin-8-ol (-)-dibenzoyl-L-tartrate. The salt was purified by repeated recrystallizations from chloroform-acetone-methanol (10:6:1).

m.p.: 172.0-173.0°C (dec)

Optical Rotation: $[\alpha]_D^{25}$ =-65.6° (C=0.308, methanol)

The salt was treated with a saturated aqueous sodium hydrogencarbonate solution to give the captioned compound as colorless crystals.

Optical Rotation: $[\alpha]_D^{25}$ =+16.6° (C=0.445, chloroform)

EXAMPLE 1

(1) Preparation of Compound 1

Intermediate 1 obtained in Referential Example 1 (360 mg, 1.01 mmole) was dissolved in dimethyl sulfoxide (6ml), and potassium hydroxide (245 mg, 3.71 mmole, 85%) was added, and the reaction mixture was stirred under nitrogen at room temperature for 5 minutes. To the mixture was added dropwise a solution of 3-chloromethyl-N,N-dimethylbenzylamine hydrochloride (267 mg, 1.21 mmole) in dimethyl sulfoxide (6ml) over a period of 1 hour, and the mixture was stirred at room temperature for 4 hours. The reaction mixture was diluted with ethyl acetate and washed successively with water and brine. The organic layer was dried over $MgsO_4$ and concentrated under reduced pressure. The residue was purified by MPLC (Merck, Lichroprep Si 60/chloroform:methanol=40:1~10:1) to give the captioned compound (420mg) as a colorless oil.

IR(neat, $cm^{-1}$): 2944,1677,1611,1515,1431,1344,1248, 1179,1122,1086,1032,753

FAB-MS(m/z, $(C_{30}H_{36}N_2O_5+H)^+$): 505

$^1$H-NMR(300MHz, $CDCl_3$, δ ppm):

2.21+2.27(6H,s×2),2.65(1H,dd,J=10.7Hz,13.7Hz), 2.70-2.91(2H,m),3.07(1H,dd,J=2.5Hz,13.7Hz),3.10-3.29+4.41(2H,m+ddd,J=2.1Hz,6.7Hz,13.2Hz), 3.39+3.44+3.48-3.52(2H,dx2+m,J=13.5Hz, J=13.5Hz),3.74+3.75(3H,s×2), 3.86+3.88(3H,s×2), 3.93(3H,s),4.30+5.73(1H,dd×2,J=2.5Hz,10.7Hz, J=3.4Hz,9.0Hz),5.11(1H,d,J=10.9Hz), 5.28+5.35(1H, d×2,J=10.9Hz,J=10.9Hz),6.39+6.46(1H,s×2),6.64-6.87(4H,m),7.19(1H,s),7.30-7.39+7.46-7.52(3H,m×2),7.41+7.97(1H,s×2)

(2) Preparation of Compound 1 hydrochloride

Compound 1 was dissolved in methanol containing hydrogen chloride. The mixture was concentrated under reduced pressure, and the residue was recrystallized from a mixture of methanol and diethytl ether to give the captioned compound as a colorless powder.

m.p.: 161-167°C

| High Resolution FAB-MS(m/z, $(C_{30}H_{36}N_2O_5+H)^+$): | |
|---|---|
| Calcd.: | 505.2702 |
| Found : | 505.2719 |

Compounds 2~6 (Examples 2~6) were prepared by the reaction of intermediate 1 (Referential Example 1) with the corresponding chlorides, N-(3-chloromethylbenzyl)pyrrolidine hydrochloride, N-(3-chloromethylbenzyl)morpholine hydrochloride, 4-chloromethyl-N,N-di-methylbenzylamine hydrochloride, N-(3-chloromethylbenzyl)-N′-methylpiperazine dihydrochloride, N-(3-chloropropyl)-N-methylbenzylamine hydrochloride, in the same manner as described in Example 1-(1).

EXAMPLE 2

Compound 2

appearance: colorless oil

IR(neat, $cm^{-1}$): 2938,1674,1611,1515,1431,1344,1245, 1122,1083,1032,753

| High Resolution FAB-MS(m/z, $(C_{32}H_{38}N_2O_5+H)^+$): | |
|---|---|
| Calcd.: | 531.2859 |
| Found : | 531.2889 |

$^1$H-NMR(300MHz, $CDCl_3$, δppm):

1.57(2H,brs),1.75(2H,brs),2.46(4H,brs),2.64(1H, dd,J=10.6Hz,13.8Hz),2.66-2.91(2H,m),3.07(1H,dd, J=2.7Hz, 13.8Hz),3.13(1H,ddd,J=5.4Hz,11.2Hz, 13.3Hz),3.46-3.72(2H,m),3.75(3H,s),3.88(3H,s), 3.93(3H,s),4.29+5.69-5.77 (1H,dd+m,J=2.7Hz, 10.6Hz),4.40(1H,ddd,J=2.5Hz,6.4Hz,13.3Hz),5.11 (1H,d,J=11.3Hz),5.24-5.31+5.34(1H,m+d, J=11.3Hz), 6.39+6.45(1H,s×2),6.64-6.76(4H,m),7.17(1H,s), 7.34(3H,m),7.41+7.97(1H,s×2)

EXAMPLE 3

Compound 3

appearance: colorless oil

FAB-MS(m/z, $(C_{32}H_{38}N_2O_6+H)^+$): 547

IR(neat, $cm^{-1}$): 2938,1677,1608,1518,1458,1431,1269, 1179,1119,1083,1032,1008

$^1$H-NMR(300MHz, $CDCl_3$, δppm):

2.38+2.41-2.47(4H,t+m,J=4.7Hz),2.64(1H,dd, J=10.7Hz,13.5Hz),2.72-2.90(2H,m),3.06(1H,dd, J=2.8Hz, 13.5Hz),3.10-3.30+4.40(2H,m+ddd,J=2.5Hz, 4.0Hz,13.0Hz),3.44+3.48(2H,d×2,J=13.5Hz, J=13.5Hz),3.64+3.65-4.00(4H,t+m,J=4.7Hz),3.74+ 3.75(3H,s×2),3.86+3.88(3H,s×2),3.93(3H,s), 4.28+5.72(1H,dd×2,J=2.5Hz,10.8Hz, J=4.2Hz, 10.8Hz),5.13(1H,d,J=11.3Hz),5.26+5.33(1H,d×2, J=11.3Hz,J=11.3Hz),6.39+6.46(1H,s×2),6.67-6.85 (4H,m), 7.17(1H,s),7.33-7.37(3H,m),7.38+7.97(1H,s ×2)

EXAMPLE 4

Compound 4

appearance: pale yellow oil

| High Resolution FAB-MS(m/z, $(C_{30}H_{36}N_2O_5+H)^+$): | |
|---|---|
| Calcd.: | 505.2702 |
| Found : | 505.2719 |

$^1$H-NMR(300MHz, $CDCl_3$, δppm):

2.25+2.28(6H,s×2),2.66(1H,dd,J=10.7Hz,13.9Hz), 2.71-2.92(2H,m),3.08(1H,dd,J=2.7Hz,13.9Hz),3.13-3.30 (1H,m),3.46+3.49(2H,s ×2),3.74+3.75(3H,s× 2),3.86+3.88(3H,s×2),3.93(3H,s),4.35+5.70-5.77 (1H,dd+m,J=2.7Hz, 10.7Hz),4.36-4.45(1H,m),5.11+ 5.12(1H,d×2,J=11.1Hz,J=11.1Hz),5.24+5.33(1H,d× 2,J=11.1Hz,J=11.1Hz),6.39+6.46 (1H,s×2),6.67-6.90(4H,m),7.22+7.97(1H,s ×2),7.34(2H,d, J=8.2Hz),7.42(2H,d,J=8.2Hz)

EXAMPLE 5

Compound 5

appearance: colorless oil

IR(neat, $cm^{-1}$): 2938,2800,1677,1518,1497,1458,1428, 1248,1122,1083,1032,825,753

| High Resolution FAB-MS(m/z, ($C_{33}H_{41}N_3O_5$+H)$^+$): | |
|---|---|
| Calcd.: | 560.3124 |
| Found : | 560.3134 |

$^1$H-NMR(300MHz, $CDCl_3$, δppm):

2.22-2.56(8H,m),2.27(3H,s),2.64(1H,dd,J=10.6Hz, 14.0Hz),2.72(1H,ddd,J=2.2Hz,5.3Hz,16.8Hz),2.85 (1H,ddd, J=6.3Hz,10.9Hz,16.8Hz),3.06(1H,dd, J=2.6Hz,14.0Hz),3.14(1H,ddd,J=5.3Hz,10.9Hz, 12.8Hz),3.21-3.31+4.40(1H, m+ddd,J=2.2Hz,6.3Hz, 12.8Hz),3.40-3.56(2H,m),3.75(3H,s),3.88(3H,s), 3.93(3H,s),4.29+5.69-5.75(1H,dd+m, J=2.6Hz, 10.6Hz),5.12(1H,d,J=11.1Hz),5.22-5.28+5.33(1H, m+d,J=11.1Hz),6.39+6.46(1H,sx2),6.64-6.86(4H, m),7.18 (1H,s),7.24-7.51(3H,m),7.38+7.97(1H,sx2)

EXAMPLE 6

Compound <u>6</u>

appearance: colorless oil

IR(neat, cm$^{-1}$): 2944,2840,2796,1676,1606,1586,1516, 1498,1458,1432,1248,1122,1034,738

FAB-MS(m/z, ($C_{31}H_{38}N_2O_5$+H)$^+$): 519

$^1$H-NMR(300MHz, $CDCl_3$, δppm):

2.06-2.15(2H,m),2.24+2.25(3H,sx2),2.65(2H,brt, J=7.3Hz),2.76(1H,dd,J=10.5Hz,13.9Hz),2.70-2.92 (2H,m), 3.14(1H,dd,J=2.8Hz,13.9Hz),3.17-3.25(1H, m),3.54(2H,s),3.75+3.77(3H,sx2),3.84(3H,s),3.85 (3H,s),4.14-4.21(1H,m), 4.34-4.48(2H,m),4.62+5.73 (1H,ddx2,J=2.8Hz,10.5Hz,J=4.2Hz,9.8Hz),6.35+ 6.42(1H,sx2),6.75-6.85(2H,m),7.00-7.07(2H,m), 7.22-7.33(5H,m),7.51+8.00(1H,sx2)

Compounds <u>7</u>~<u>12</u> (Examples 7~12) were prepared by the reaction of intermediate <u>2</u> (Referential Example 2) with the corresponding chlorides, 3-chloromethyl-N,N-dimethylbenzylamine hydrochloride, 4-chloromethyl-N,N-dimethylbenzylamine hydrochloride, 3-chloromethyl-4-methoxy-N,N-dimethylbenzylamine hydrochloride, N-(3-chloromethylbenzyl)morpholine hydrochloride, 2-chloromethylpyridine hydrochloride, 3-chloromethylpyridine hydrochloride in the same manner as described in Example 1-(1).

EXAMPLE 7

Compound <u>7</u>

appearance: pale yellow oil

IR(neat, cm$^{-1}$): 3466,2776,1605,1497,1437,1344,1236, 1122,1032,800,703

| High Resolution FAB-MS(m/z, ($C_{30}H_{36}N_2O_5$+H)$^+$): | |
|---|---|
| Calcd.: | 505.2702 |
| Found : | 505.2730 |

$^1$H-NMR(300MHz, $CDCl_3$, δppm):

2.22+2.29(6H,sx2),2.70(1H,dd,J=10.7Hz,13.8Hz), 2.68-2.91(2H,m),3.12(1H,dd,J=2.8Hz,13.8Hz),3.11-3.18 (1H,m),3.42(1H,d,J=13.1Hz),3.48(1H,d, J=13.1Hz),3.67+3.70(3H,sx2),3.86+3.88(3H,sx2), 3.92(3H,s),4.35-4.42(2H, m),5.11+5.14(1H,dx2, J=10.1Hz,J=11.2Hz),5.28+5.33(1H,dx2,J=10.1Hz, J=11.2Hz),6.40+6.46(1H,sx2),6.44+6.46 (1H,sx2), 6.69-6.72(1H,m),7.07-7.10(1H,m),7.23(1H,s),7.29-7.47(4H,m),7.39+7.92(1H,s x2)

EXAMPLE 8

Compound 8

appearance: colorless oil
IR(neat, $cm^{-1}$): 2944,2776,1677,1605,1584,1497,1458, 1437,1122,1083,1035,966

| High Resolution FAB-MS(m/z, ($C_{30}H_{36}N_2O_5$+H)$^+$): | |
|---|---|
| Calcd.: | 505.2702 |
| Found : | 505.2713 |

$^1$H-NMR(300MHz, $CDCl_3$, δppm):
2.24+2.28(6H,s×2),2.71(1H,dd,J=10.7Hz,13.8Hz), 2.70-2.89(2H,m),3.13(1H,dd,J=2.5Hz,13.8Hz),3.12-3.20 (1H,m),3.46(2H,brs),3.68+3.71(3H,s ×2), 3.86+3.88(3H,s×2),3.92(3H,s),4.35-4.43(1H,m), 4.43(1H,dd,J=2.5Hz, 10.7Hz),5.15(1H,d,J=11.1Hz), 5.31(1H,d,J=11.1Hz),6.40+6.44(1H,s×2),6.44-6.59 (2H,m),6.67-6.72(1H,m),7.07-7.13(1H,m),7.29-7.42 (4H,m),7.42+7.98(1H,s×2)

EXAMPLE 9

Compound 9

appearance: colorless oil
IR(neat, $cm^{-1}$): 2944,2776,1674,1605,1584,1500,1461, 1437,1260,1122,1083,1032,756

| High Resolution FAB-MS(m/z, ($C_{31}H_{38}N_2O_6$+H)$^+$): | |
|---|---|
| Calcd.: | 535.2808 |
| Found : | 535.2828 |

$^1$H-NMR(300MHz, $CDCl_3$, δppm):
2.20+2.30(6H,s×2),2.67(1H,dd,J=10.7Hz,13.6Hz), 2.70-2.89(2H,m),3.13(1H,dd,J=2.5Hz,13.6Hz),3.10-3.19 (1H,m),3.40(2H,brs),3.67+3.71(3H,s ×2),3.74 (3H,s),3.85+3.87(3H,s×2),3.91+3.95(3H,s×2), 4.36-4.43(1H,m), 4.54(1H,dd,J=2.5Hz,10.7Hz),5.14 (1H,d,J=10.9Hz),5.42(1H,d,J=10.9Hz),6.37+6.43 (1H,s×2),6.43-6.54(2H,m),6.68-6.71(1H,m),8.67 (1H,d,J=8.1Hz),7.05-7.13(1H,m),7.27-7.29(2H,m), 7.43+7.98(1H,s×2)

EXAMPLE 10

Compound 10

appearance: colorless oil
IR(neat, $cm^{-1}$): 2938,2854,1677,1605,1584,1497,1458, 1434,1269,1119,864,753

| High Resolution FAB-MS(m/z, ($C_{32}H_{38}N_2O_6$+H)$^+$): | |
|---|---|
| Calcd.: | 547.2808 |
| Found : | 547.2781 |

$^1$H-NMR(300MHz, $CDCl_3$, δppm):
2.37+2.43(4H,brt×2,J=4.6Hz,J=4.6Hz),2.69(1H,dd, J=10.9Hz,13.6Hz),2.71-3.00(2H,m),3.11(1H,dd, J=2.4Hz,13.6Hz),3.10-3.19(1H,m),3.43(1H,d, J=3.2Hz),3.49(1H,d,J=3.2Hz),3.64-3.69(4H,m),3.71 (3H,s),3.86+3.88(3H, sx2),3.93(3H,s),4.32-4.42 (2H,m),5.16(1H,d,J=11.3Hz),5.32(1H,d,J=11.3Hz), 6.40-6.55(3H,m),6.69-6.72(1H,m),7.07-7.13(1H,m), 7.20(1H,s),7.31-7.38(3H,m),7.38+7.98(1H,sx2)

EXAMPLE 11

Compound 11

appearance: colorless oil
IR(neat, $cm^{-1}$): 2944,1674,1605,1497,1458,1437,1344, 1269,1122,1086,1038,762

| High Resolution FAB-MS(m/z, $(C_{26}H_{28}N_2O_5+H)^+$): | |
|---|---|
| Calcd.: | 449.2076 |
| Found : | 449.2091 |

$^1$H-NMR(300MHz, $CDCl_3$, δppm):
2.76(1H,dd,J=10.9Hz,13.8Hz),2.73-2.95(2H,m), 3.14-3.24(2H,m),3.68+3.71(3H,sx2),3.86+3.87(3H, sx2),3.91 (3H,s),4.41(1H,ddd,J=2.7Hz,6.6Hz, 13.2Hz),4.71(1H,dd,J=2.8Hz,10.9Hz),5.27+5.29(1H, dx2,J=12.1Hz,J=12.7Hz), 5.40+5.45(1H,dx2, J=12.7Hz,J=12.1Hz),6.42+6.47(1H,sx2),6.51-6.59 (2H,m),6.69-6.73(1H,m),7.08-7.14(1H,m),7.26-7.30 (1H,m),7.41+7.99(1H,sx2),7.51-7.54(1H,m),7.72-7.77(1H,m),8.63-8.65(1H,m)

EXAMPLE 12

Compound 12

appearance: colorless oil
IR(neat, $cm^{-1}$): 2944,1674,1605,1584,1497,1434,1344, 1267,1233,1122,1086,1032

| High Resolution FAB-MS(m/z, $(C_{26}H_{28}N_2O_5+H)^+$): | |
|---|---|
| Calcd.: | 449.2076 |
| Found : | 449.2077 |

$^1$H-NMR(300MHz, $CDCl_3$, δppm):
2.59-2.91(2H,m),2.76(1H,dd,J=10.5Hz,13.9Hz),3.10 (1H,dd,J=2.9Hz,13.9Hz),3.13-3.29(1H,m),3.69+3.71 (3H, sx2),3.86+3.90(3H,sx2),3.86+3.88(3H,sx2), 4.37-4.45(1H,m),4.47+5.76(1H,ddx2,J=2.9Hz, 10.5Hz,J=4.3Hz, 9.2Hz),5.13+5.20(1H,dx2, J=11.4Hz,J=11.6Hz),5.25+5.34(1H,dx2,J=11.4Hz, J=11.6Hz),6.43+6.49(1H,sx2),6.51-6.58(2H,m), 6.69-6.74(1H,m),7.09+7.14(1H,x2,J=7.9Hz, J=7.8Hz),7.32-7.38(1H,m),7.37+8.00(1H,sx2), 7.79+7.90-7.94(1H,td+m,J=2.1Hz,7.8Hz),8.61-8.64 (1H,m),8.74-8.75(1H,m)

Compounds 13~21 (Examples 13~21) were prepared by the reaction of corresponding intermediates 3~9 (Referential Examples 3~9) with the corresponding chlorides, 3-chloromethyl-N,N-dimethylbenzylamine hydrochloride, N-(3-chloromethylbenzyl)morpholine hydrochloride, (4-chlorobutylthio)benzene, in the same manner as described in Example 1-(1).

EXAMPLE 13

Compound 13

appearance: pale brown oil
IR(neat, $cm^{-1}$): 2944,1674,1605,1500,1464,1434,1344, 1245,1122,1086,1032,753

| High Resolution FAB-MS(m/z, ($C_{30}H_{36}N_2O_5$+H)$^+$): | |
|---|---|
| Calcd.: | 505.2705 |
| Found : | 505.2704 |

$^1$H-NMR(300MHz, $CDCl_3$, δppm):

2.21+2.27(6H,s×2),2.77-2.83(2H,m),3.23(2H,d, J=6.3Hz),3.25-3.38(1H,m),3.41(1H,d,J=13.1Hz), 3.46(1H,d, J=13.1Hz),3.64+3.67(3H,s×2),3.86+3.88 (3H,s×2),3.86+3.90(3H,s×2),4.23-4.30(1H,m), 4.53(1H,t,J=7.2Hz),5.15(1H, d,J=11.3Hz),5.30(1H, d,J=11.3Hz),6.41+6.47(1H,s×2),6.72-6.79(3H,m), 7.12-7.18(1H,m),7.29-7.40(5H,m)

EXAMPLE 14

Compound 14

appearance: colorless oil

IR(neat, $cm^{-1}$): 3466,2944,1668,1518,1500,1458,1431, 1269,1239,1122,1029

| High Resolution FAB-MS(m/z, ($C_{31}H_{38}N_2O_6$+H)$^+$): | |
|---|---|
| Calcd.: | 535.2808 |
| Found : | 535.2838 |

$^1$H-NMR(300MHz, $CDCl_3$, δppm):

2.21+2.29(6H,s×2),2.70(1H,dd,J=10.4Hz,14.0Hz), 2.68-2.94(2H,m),3.07(1H,dd,J=2.8Hz,14.0Hz),3.11-3.19 (1H,m),3.41(1H,d,J=13.5Hz),3.45(1H,d, J=13.5Hz),3.65+3.67(3H,s×2),3.82+3.83(3H,s×2), 3.85+3.88(3H,s×2),3.93 (3H,s),4.36-4.43(2H,m), 5.16(1H,d,J=11.5Hz),5.33(1H,d,J=11.5Hz),6.46-6.52(3H,m),6.65-6.70(1H,m),7.28-7.50(4H, m), 7.37+8.01(1H,s×2)

EXAMPLE 15

Compound 15

appearance: colorless oil

IR(neat, $cm^{-1}$): 2944,2836,1671,1608,1506,1432,1227, 1122,1083,1032,790,745,695

| High Resolution FAB-MS(m/z, ($C_{31}H_{38}N_2O_6$+H)$^+$): | |
|---|---|
| Calcd.: | 535.2808 |
| Found : | 535.2825 |

$^1$H-NMR(300MHz, $CDCl_3$, δppm):

2.01+2.28(6H,s×2),2.70-2.91(2H,m),3.01(2H,d, J=7.1Hz),3.28(1H,ddd,J=6.3Hz,10.0Hz,13.4Hz),3.45 (1H,d, J=13.0Hz),3.47(1H,d,J=13.0Hz),3.61+3.63 (3H,s×2),3.63+3.66(3H,s×2),3.85+3.88(3H,s×2), 3.88+3.90(3H,s×2), 4.25(1H,ddd,J=3.5Hz,6.3Hz, 13.4Hz),4.54+5.89(1H,t+dd,J=7.1Hz,J=3.8Hz, 10.0Hz),5.17+5.18(1H,d×2,J=11.2Hz, J=11.2Hz), 5.27+5.28(1H,d×2,J=11.2Hz,J=11.2Hz),6.41+6.46 (1H,s×2),6.47(1H,s),6.60-6.75(2H,m),7.24-7.55 (4H,m),7.38+7.92(1H,s×2)

EXAMPLE 16

Compound 16

appearance: yellow oil

| High Resolution FAB-MS(m/z, ($C_{32}H_{40}N_2O_7$+H)$^+$): | |
|---|---|
| Calcd.: | 565.2914 |
| Found : | 565.2920 |

$^1$H-NMR(300MHz, $CDCl_3$, δppm):

2.21+2.34(6H,s×2),2.73(1H,dd,J=10.5Hz,13.9Hz), 2.79-3.01(2H,m),3.06(1H,dd,J=2.6Hz,13.9Hz),3.10-3.27 (1H,m),3.43(2H,s),3.68(6H,s),3.78+3.79(3H,s ×2),3.87+3.88(3H,s×2),3.92(3H,s),4.37-4.48(1H, m),4.41-4.48+5.75-5.82(1H,m×2),5.11+5.18(1H,dx 2,J=11.5Hz,J=11.5Hz),5.27+5.32(1H,d×2,J=11.5Hz, J=11.5Hz),6.13+6.17(2H,s×2), 6.40+6.47(1H,s×2), 7.26-7.55(4H,m),7.39+8.06(1H,s×2)

EXAMPLE 17

Compound 17

appearance: pale yellow oil
IR(neat, cm$^{-1}$): 2944,1674,1497,1458,1434,1344,1122, 1086,1029

| High Resolution FAB-MS(m/z, ($C_{28}H_{33}N_3O_4$+H)$^+$): | |
|---|---|
| Calcd.: | 476.2549 |
| Found : | 476.2520 |

$^1$H-NMR(300MHz, $CDCl_3$, δppm):

2.18+2.24(6H,s×2),2.60-2.95(2H,m),2.73(1H,dd, J=10.7Hz,13.9Hz),3.14(1H,dd,J=2.6Hz,13.9Hz), 3.15-3.40 (1H,m),3.40+3.47(2H,s×2),3.86+3.88(3H, s×2),3.90+3.94(3H,s×2),4.31+5.71(1H,dd×2, J=2.6Hz,10.7Hz,J=2.8Hz, 9.0Hz),4.38-4.50(1H,m), 5.09+5.13(1H,d×2,J=10.5Hz,J=10.5Hz),5.31+5.36 (1H,d×2,J=10.5Hz,J=10.5Hz),6.41+6.46 (1H,s×2), 7.00-7.61(8H,m),7.60+7.95(1H,s×2)

EXAMPLE 18

Compound 18

appearance: colorless oil
IR(neat, cm$^{-1}$): 2944,2775,1671,1611,1515,1434,1344, 1245,1122,1086,1032,827

| High Resolution FAB-MS(m/z, ($C_{31}H_{38}N_2O_5$+H)$^+$): | |
|---|---|
| Calcd.: | 519.2859 |
| Found : | 519.2830 |

$^1$H-NMR(300MHz, $CDCl_3$, δppm):

1.78-1.94+2.15-2.45(2H,m×2),2.21+2.30(6H,s×2), 2.48-3.11(5H,m),3.43+3.57(2H,s×2),3.75+3.78(3H, s×2), 3.82+3.84(3H,s×2),3.83+3.87(3H,s×2), 4.22+5.59(1H,dd×2,J=3.0Hz,10.9Hz,J=3.0Hz, 10.7Hz),4.40(1H,ddd,J=2.5Hz,6.8Hz,12.8Hz),5.04-5.21(2H,m),6.38+6.40(1H,s ×2),6.74+6.77(2H,d×2, J=8.3Hz,J=8.8Hz),6.97+7.00(2H,d×2, J=8.3Hz, J=8.8Hz),7.11-7.45(4H,m),7.73+8.22(1H,s×2)

EXAMPLE 19

Compound 19

appearance: colorless oil
IR(neat, $cm^{-1}$): 2944,2780,1674,1479,1440,1389,1263, 1155,1095,1038,695

| High Resolution FAB-MS(m/z, $(C_{29}H_{32}N_2O_5+H)^+$): | |
|---|---|
| Calcd.: | 489.2389 |
| Found : | 489.2408 |

$^1$H-NMR(300MHz, $CDCl_3$, δppm):
2.26+2.32(6H,s×2),2.54-2.92(3H,m),3.06-3.34(2H, m),3.54+3.64(2H,s×2),3.68+3.69(3H,s×2),3.43-3.52+4.39 (1H,m+ddd,J=2.4Hz,6.3Hz,13.2Hz), 4.58+5.81(1H,dd×2,J=10.4Hz,12.4Hz,J=3.8Hz, 9.4Hz),5.32+5.33(1H,d×2, J=11.4Hz,J=11.2Hz), 5.39+5.42(1H,d×2,J=11.4Hz,J=11.2Hz),5.92+5.96 (1H,d+s,J=3.0Hz),5.95+5.96(1H,d+s, J=3.0Hz), 6.30+6.37(1H,s×2),6.46-6.76(3H,m),7.03-7.50(5H, m),7.40+7.97(1H,s×2)

EXAMPLE 20

Compound 20

appearance: colorless oil
IR(neat, $cm^{-1}$): 2944,2812,1677,1626,1482,1440,1263, 1155,1116,1038,864,753

| High Resolution FAB-MS(m/z, $(C_{31}H_{34}N_2O_6+H)^+$): | |
|---|---|
| Calcd.: | 531.2495 |
| Found : | 531.2500 |

$^1$H-NMR(300MHz, $CDCl_3$, δppm):
2.38(4H,t,J=4.6Hz),2.51-2.90(3H,m),3.08-3.35(2H, m),3.48(2H,s),3.64(4H,t,J=4.6Hz),3.65+3.68(3H,s ×2),4.39 (1H,ddd,J=2.8Hz,6.6Hz,13.1Hz),4.56+5.79 (1H,dd×2,J=2.6Hz,10.5Hz,J=4.0Hz,9.4Hz),5.30+ 5.33(1H,d×2,J=11.4Hz, J=11.3Hz),5.39+5.42(1H,d× 2,J=11.4Hz,J=11.3Hz),5.93+5.97(1H,d+s,J=6.3Hz), 5.94+5.97(1H,d+s,J=6.3Hz), 6.30+6.37(1H,s×2), 6.46-6.76(3H,m),7.10(1H,t,J=8.0Hz),7.39-7.48(4H, m),7.43+7.96(1H,s×2)

EXAMPLE 21

Compound 21

appearance: pale yellow oil
IR(neat, $cm^{-1}$): 1605,1404,1311,1272,1236,1191,1026, 717,693

| High Resolution FAB-MS(m/z, $(C_{28}H_{32}N_2O_4S+H)^+$): | |
|---|---|
| Calcd.: | 493.2161 |
| Found : | 493.2151 |

$^1$H-NMR(300MHz, $CDCl_3$, δppm):

1.80-2.10(4H,m),2.55-2.65+2.69-2.80(1H,m×2), 2.87(1H,dd,J=10.5Hz,14.2Hz),2.80-3.02(1H,m), 3.00-3.10(2H,m),3.22+3.24(1H,dd×2,J=4.2Hz, 14.2Hz,J=4.2Hz,14.2Hz),3.40-3.62(1H,m),3.81+3.82 (3H,s×2),3.84+3.85(3H,s×2),4.00-4.16(1H,m), 4.20-4.40(1H,m),4.46(1H,ddd,J=2.4Hz,6.9Hz, 13.2Hz),4.67+5.71(1H,dd×2,J=4.2Hz,10.5Hz, J=4.2Hz,10.5Hz),6.38+6.43(1H,s×2),7.12-7.56(7H, m),7.60+7.99(1H,s×2),8.26+8.45(1H,d,J=1.8Hz, J=1.8Hz),8.44+8.51(1H,dd,J=1.8Hz,4.9Hz,J=1.8Hz, 4.9Hz)

EXAMPLE 22

(1) Preparation of 8-(3-chloromethylbenzyloxy)-2-formyl-6,7-dimethoxy-1-(3-methoxybenzyl)-1,2,3,4-tetrahydroisoquinoline

To a suspension of sodium hydride (80 mg, 1.96 mmole, 60%) in dimethyl sulfoxide (1 ml) was added a solution of intermediate 2 obtained in Referential Example 2 (350 mg, 0.98 mmole) at room temperature under nitrogen, and the reaction mixture was stirred for 10 minutes. The resulting mixture was added dropwise to a solution of α,α′ -dichloro-m-xylene (172 mg, 0.98 mmole) in dimethyl sulfoxide (1 ml) at room temperature, and stirred for 2 hours. The reaction mixture was chilled in ice, diluted with water, and extracted with ethyl acetate. The combined organic layers were dried over $MgsO_4$, and concentrated under reduced pressure. The residue was purified by column chromatography (silica gel/ethyl acetate) to give the desired compound (387 mg) as a pale yellow oil.

FAB-MS(m/z, $(C_{28}H_{30}NO_5Cl+H)^+$): 496

(2) Preparation of Compound 22

The compound obtained in (1) (64.0 mg, 0.129 mmole) was treated with a 40% solution of methylamine in methanol (2 ml), and stirred at room temperature for 4 hours. The mixture was evaporated under reduced pressure, and the residue was purified by preparative thin-layer chromatography (Merck, silica gel $60F_{254}$/chloroform:methanol=10:1) to give the captioned compound (51.2 mg) as a pale yellow oil.

IR(neat, $cm^{-1}$): 2938,2794,1674,1605,1584,1497,1458, 1437,1377,1344,1269,1236,1191,1151, 1122, 1083,1032,1005,963,753,699

| High Resolution FAB-MS(m/z, $(C_{29}H_{34}N_2O_5+H)^+$): | |
|---|---|
| Calcd.: | 491.2546 |
| Found : | 491.2540 |

$^1$H-NMR(300MHz, $CDCl_3$, δppm):

2.42+2.53(3H,s×2),2.63-2.91(3H,m),3.07-3.20(2H, m),3.53-3.60+4.34-4.42(1H,m×2),3.70+3.73(3H,s× 2),3.88 (2H,s),3.81+3.87(3H,s×2),3.92+3.96(3H,s ×2),4.34-4.42+5.37-5.42(1H,m×2),5.05+5.14(1H,d ×2,J=11.3Hz,J=11.3Hz), 5.33+5.42(1H,d×2, J=11.3Hz,J=11.3Hz),6.35-6.50(3H,m),6.68-6.74(1H, m),7.08-7.17(1H,m),7.30-7.45(4H,m), 7.47+7.78(1H, s×2)

Compounds 23~25 (Examples 23~25) were prepared by the reaction of the compound obtained in Example 22-(1) with the corresponding amines, N-methylpiperazine, thiomorpholine, 1,3-thiazolidine, in the same manner as described in Example 22-(2). Compounds 26~29 were prepared using intermediate 2 obtained in Referential Example 2, α,α′ -dichloro-o-xylene and dimethylamine (Example 26); intermediate 1 obtained in Referential Example 1, α,α′ -dichloro-o-xylene and dimethylamine (Example 27); intermediate 1, α,α′ -dichloro-m-xylene and N-methylethanolamine (Example 28); intermediate 10 obtained in Referential Example 10, α,α′ -dichloro-o-xylene and dimethylamine (Example 29), in the same manner as described in Example 22.

EXAMPLE 23

Compound 23

appearance: pale yellow oil
IR(neat, $cm^{-1}$): 2938,2800,1677,1605,1584,1497,1461, 1437,1374,1344,1269,1236,1188,1167,1122,1086, 1035,1014,978,963,825, 753,699

| High Resolution FAB-MS(m/z, $(C_{33}H_{41}N_3O_5+H)^+$): | |
|---|---|
| Calcd.: | 560.3124 |
| Found : | 560.3143 |

$^1$H-NMR(300MHz, $CDCl_3$, δppm):
2.29+2.31(3H,sx2),2.32-2.56(8H,m),2.69(1H,dd, J=11.0Hz,14.0Hz),2.74-2.91(2H,m),3.11(1H,dd, J=3.0Hz, 14.0Hz),3.10-3.20(1H,m),3.47+3.54(2H,sx 2),3.70+3.73(3H,sx2),3.86+3.88(3H,sx2),3.93 (3H,s),4.30-4.42(1H,m), 4.36+5.76-5.82(1H,dd+m, J=3.0Hz,11.0Hz),5.12+5.14(1H,dx2,J=11.2Hz, J=11.2Hz),5.26+5.32(1H,d × 2,J=11.2Hz, J=11.2Hz), 6.40-6.58(3H,m),6.68-6.73(1H,m),7.09+7.10(1H,tx 2,J=8.0Hz,J=8.0Hz),7.20-7.50(4H,m),7.37+7.98(1H, sx2)

EXAMPLE 24

Compound 24

appearance: pale brown oil
IR(neat, $cm^{-1}$): 2938,1677,1605,1584,1497,1458,1434, 1371,1344,1266,1236,1191,1155,1122, 1083,1035, 1005,963,798,753,699

| High Resolution FAB-MS(m/z, $(C_{32}H_{38}N_2O_5S+H)^+$): | |
|---|---|
| Calcd.: | 563.2579 |
| Found : | 563.2597 |

$^1$H-NMR(300MHz, $CDCl_3$, δppm):
2.55-2.91(11H,m),2.69(1H,dd,J=11.0Hz,13.8Hz), 3.11(1H,dd,J=3.0Hz,13.8Hz),3.47+3.54(2H,sx2), 3.67+3.70 (3H,sx2),3.86+3.88(3H,sx2),3.93(3H, s),4.34+5.75-5.81(1H,dd+m,J=3.0Hz,11.0Hz),4.35-4.42(1H,m),5.12+5.16(1H,d x2,J=11.2Hz,J=11.2Hz), 5.26+5.32(1H,dx2,J=11.2Hz,J=11.2Hz),6.39-6.57 (3H,m),6.65-6.73(1H,m), 7.06+7.10 (1H,tx2, J=7.7Hz,J=7.7Hz),7.19-7.48(4H,m),7.35+7.98(1H,sx2)

EXAMPLE 25

Compound 25

appearance: pale yellow oil
IR(neat, $cm^{-1}$): 2944,1674,1605,1563,1497,1434,1374, 1344,1266,1233,1155,1122,1083,1044

| High Resolution FAB-MS(m/z, $(C_{31}H_{36}N_2O_5S+H)^+$): | |
|---|---|
| Calcd.: | 549.2423 |
| Found : | 549.2443 |

$^1$H-NMR(300MHz, $CDCl_3$, δppm):

2.70(1H,dd,J=10.7Hz,13.6Hz),2.74-3.20(7H,m),3.11 (1H,dd,J=2.0Hz,13.6Hz),3.52+3.58(2H,s×2),3.67+ 3.71 (3H,s×2),3.86+3.88(3H,s×2),3.93(3H,s), 3.96+4.04(2H,s×2),4.33-4.43+5.74-5.80(2H,m×2), 5.13+5.16(1H,d×2, J=11.2Hz,J=11.2Hz),5.27+5.33 (1H,d×2,J=11.2Hz,J=11.2Hz),6.39-6.56(3H,m), 6.66-6.74(1H,m),7.09+7.10 (1H,t×2, J=8.0Hz, J=8.0Hz),7.20-7.23(1H,m),7.33-7.42(3H,m),7.44+7.97(1H,s×2)

EXAMPLE 26

Compound 26

appearance: colorless powder
m.p.: 126~128°C
IR(neat, $cm^{-1}$): 1677,1605,1458,1434,1122,1086,1032

| High Resolution FAB-MS(m/z, ($C_{30}H_{36}N_2O_5$+H)$^+$): | |
|---|---|
| Calcd.: | 505.2702 |
| Found : | 505.2716 |

$^1$H-NMR(300MHz, $CDCl_3$, δppm):

2.18+2.21(6H,s×2),2.72(1H,dd,J=11.0Hz,13.6Hz), 2.80-2.95(1H,m),3.11(1H,dd,J=2.8Hz,13.6Hz),3.10-3.24 (1H,m),3.38(1H,d,J=12.8Hz),3.57(1H,d, J=12.8Hz),3.40-3.60(1H,m),3.65+3.66(3H,s×2), 3.87+3.89(3H,s×2),3.87+ 3.92(3H,s×2),4.34-4.46+5.79(2H,m+dd,J=3.0Hz,6.7Hz),5.22+5.24(1H,d ×2, J=11.8Hz,J=11.8Hz),5.47+5.57(1H,d×2, J=11.8Hz, J=11.8Hz),6.37(1H,d,J=7.5Hz),6.40(1H,d,J=2.4Hz), 6.47(1H,s),6.69(1H,dd,J=2.4Hz,7.5Hz),7.08(1H,t, J=7.5Hz),7.30-7.41(3H,m),7.50-7.58(1H,m),7.36+7.98(1H,s×2)

EXAMPLE 27

Compound 27

appearance: colorless oil
IR(neat, $cm^{-1}$): 2938,2860,2770,1674,1611,1515,1458, 1434,1248,1179,1122,1032,843,756

| High Resolution FAB-MS(m/z, ($C_{30}H_{36}N_2O_5$+H)$^+$): | |
|---|---|
| Calcd.: | 505.2702 |
| Found : | 505.2725 |

$^1$H-NMR(300MHz, $CDCl_3$, δppm):

2.20+2.25(6H,s×2),2.66(1H,dd,J=10.7Hz,13.9Hz), 2.70-2.84(2H,m),3.05(1H,dd,J=2.9Hz,13.9Hz),3.16 (1H,ddd, J=5.4Hz,10.9Hz,13.2Hz),3.42(1H,d, J=13.1Hz),3.62(1H,d,J=13.1Hz),3.74+3.75(3H,s× 2),3.87+3.89(3H,s×2),3.93(3H, s),4.33+5.70(1H,dd ×2,J=2.9Hz,10.7Hz,J=2.9Hz,10.7Hz),4.40(1H,ddd, J=2.4Hz,6.3Hz,13.2Hz),5.21(1H,d,J=11.7Hz), 5.46+5.59(1H,d×2,J=11.5Hz,J=11.7Hz),6.41+6.47 (1H,s×2),6.61-6.83(4H,m),7.27-7.72(4H,m), 7.22+7.98(1H,s×2)

EXAMPLE 28

Compound 28

appearance: pale yellow oil
IR(neat, $cm^{-1}$): 1668,1516,1498,1456,1432,1248,1122, 1084,1032

| High Resolution FAB-MS(m/z, ($C_{31}H_{38}N_2O_6$+H)$^+$): | |
|---|---|
| Calcd.: | 535.2808 |
| Found : | 535.2829 |

$^1$H-NMR(300MHz, $CDCl_3$, δppm):
2.17+2.28(3H,s×2),2.56(2H,t,J=5.3Hz),2.65(1H, dd,J=10.8Hz,13.9Hz),2.50-2.92(2H,m),3.05(1H,dd, J=2.6Hz, 13.9Hz),3.05-3.28(1H,m),3.55+3.56(2H,s× 2),3.60(2H,t,J=5.3Hz),3.74+3.76(3H,s×2),3.86+ 3.88(3H,s×2),3.90+3.94 (3H,s×2),4.26+5.70(1H,dd ×2,J=2.6Hz,10.8Hz,J=4.3Hz,13.5Hz),4.39(1H,ddd, J=2.3Hz,6.4Hz,13.1Hz),5.08+5.13(1H, d×2, J=10.7Hz,J=11.1Hz),5.30+5.33(1H,d×2,J=10.7Hz, 11.1Hz),6.40+6.46(1H,s×2),6.65-6.83(4H,m),7.16 (1H,s), 7.29-7.41(3H,m),7.38+7.96(1H,s×2)

EXAMPLE 29

Compound 29

appearance: colorless oil
IR(neat, cm$^{-1}$): 2944,2776,1677,1602,1467,1434,1344, 1206,1155,1122,840,753

| High Resolution FAB-MS(m/z, ($C_{31}H_{38}N_2O_6$+H)$^+$): | |
|---|---|
| Calcd.: | 535.2808 |
| Found : | 535.2833 |

$^1$H-NMR(300MHz, $CDCl_3$, δppm):
2.17+2.22(6H,s×2),2.70(1H,dd,J=10.8Hz,13.6Hz), 2.72-2.92(2H,m),3.09(1H,dd,J=2.8Hz,13.6Hz),3.13-3.21 (1H,m),3.38(1H,d,J=12.8Hz),3.54(1H,d, J=12.8Hz),3.63(6H,s),3.88(3H,s),3.91(3H,s),4.33-4.41(1H,m),4.45(1H,dd, J=2.8Hz,10.8Hz),5.22+5.27 (1H,d×2,J=11.7Hz,J=12.2Hz),6.04+6.13(2H,d×2, J=2.2Hz,J=2.1Hz),6.24-6.26(1H,m), 6.42+6.47(1H,s ×2),7.28-7.34(4H,m),7.35+8.00(1H,s×2),7.53-7.56(1H,m)

EXAMPLE 30

(1) Preparation of 6,7-dimethoxy-8-(3-dimethylaminomethylbenzyloxy)-1-(4-methoxybenzyl)-1,2,3,4-tetrahydroisoquinoline

To a solution of compound 1 obtained in Example 1 (268 mg, 0.531 mmole) in ethanol (4.6 ml) was added a solution of sodium hydroxide (500 mg, 12.5 mmole) in water (0.6 ml), and the reaction mixture was refluxed under an argon atmosphere for 22 hours. The reaction mixture was diluted with water, and extracted with ethyl acetate. The combined organic layers were washed with brine, dried over $MgsO_4$, and concentrated under reduced pressure to give the desired compound (213 mg) as a yellow oil.
FAB-MS(m/z, ($C_{29}H_{36}N_2O_4$+H)$^+$): 477

(2) Preparation of Compound 30

To a solution of the compound obtained in (1) (250 mg, 0.525 mmole) in dichloromethane (7 ml) were sequentially added hydroxyacetic acid (44 mg, 0.578 mmole), HOBt (78 mg, 0.577 mmole), and at 0°C EDCI (111 mg, 0.579 mmole), then the mixture was stirred under nitrogen at 0°C for 3.5 hours. To the mixture were added hydroxyacetic acid (34 mg, 0.447 mmole) and EDCI (82 mg, 0.428 mmole), and the reaction mixture was stirred at 0°C for 1.5 hours. The resulting mixture was roughly purified by dry column flash chromatography (silica gel/chloroform:methanol=10:1), and purified by preparative thin-layer chromatography (merck, silica gel 60F254/chloroform:methanol=10:1) to give the captioned compound (182 mg) as a colorless oil.
IR(neat, cm$^{-1}$): 2944,1647,1611,1515,1500,1458,1344, 1248,1122,1083,1032

| High Resolution FAB-MS(m/z, ($C_{31}H_{38}N_2O_6$+H)$^+$): | |
|---|---|
| Calcd.: | 535.2808 |
| Found : | 535.2820 |

$^1$H-NMR(300MHz, $CDCl_3$, δppm):
2.31+2.46(6H,s×2),2.70(1H,d,J=15.0Hz),2.65-3.51 (5H,m),3.43(1H,d,J=15.0Hz),3.58+3.80(2H,s×2), 3.75(3H, s),3.86+3.88(3H,s×2),3.88+3.93(3H,s× 2),4.40+5.86(1H,dd×2,J=1.8Hz,10.7Hz,J=5.4Hz, 8.3Hz),4.65-4.75(1H,m), 5.05+5.08(2H,d×2, J=11.1Hz,J=11.1Hz),5.24+5.41(1H,d×2,J=11.1Hz, J=11.1Hz),6.40+6.45(1H,s×2),6.63-6.78(4H, m), 7.40-7.55(4H,m)

Compounds 31~33 (Examples 31~33) were prepared by the condensation of the compound obtained in Example 30-(1) with the corresponding carboxylic acids, 3-hydroxypropionic acid, DL-lactic acid, methoxyacetic acid using tetrahydrofuran, dimethyformamide, dichloromethane as solvents, in the same manner as described in Example 30-(2).

EXAMPLE 31

Compound 31

appearance: pale yellow oil
IR(neat, $cm^{-1}$): 1743,1638,1515,1464,1437,1344,1248, 1179,1122,1092,1032

| High Resolution FAB-MS(m/z, ($C_{32}H_{40}N_2O_6$+H)$^+$): | |
|---|---|
| Calcd.: | 549.2965 |
| Found : | 549.2985 |

$^1$H-NMR(300MHz, $CDCl_3$, δppm):
2.21+2.22+2.23+2.26(6H,s×4),2.20-3.60(11H,m), 3.74+3.75(3H,s×2),3.86+3.88(3H,s×2),3.92+3.94 (3H,s×2), 4.20-4.40(1H,m),4.62-4.82+5.93-6.05 (1H,m×2),5.07+5.14(1H,d×2,J=11.2Hz,J=11.2Hz), 5.38+5.41(1H,d×2, =11.2Hz,J=11.2Hz),6.41+6.46 (1H,s×2),6.65-6.85(4H,m),7.30-7.50(4H,m)

EXAMPLE 32

Compound 32

appearance: pale yellow oil
IR(neat, $cm^{-1}$): 1644,1515,1497,1458,1248,1122,1089, 1032

| High Resolution FAB-MS(m/z, ($C_{32}H_{40}N_2O_6$+H)$^+$): | |
|---|---|
| Calcd.: | 549.2965 |
| Found : | 549.2985 |

$^1$H-NMR(300MHz, $CDCl_3$, δppm):
0.43+0.96+1.01+1.26(3H,d×4,J=6.6Hz,J=6.6Hz, J=6.6Hz,J=6.6Hz),2.24+2.28+2.33(6H,s×3),2.50-3.10(4H,m), 3.13-3.70(4H,m),3.74+3.86(3H,s ×2), 3.86+3.87(3H,s×2),3.88+3.89+3.92(3H,s×3),4.96+ 5.03+5.08+5.12(1H,d×4,J=11.0Hz,J=11.0Hz, J=11.0Hz,J=11.0Hz),5.20+5.30+5.42+5.46(1H,d×4, J=11.0Hz,J=11.0Hz,J=11.0Hz,J=11.0Hz), 4.75+4.88+ 5.88+5.99(1H,dd×4,J=10.9Hz,3.0Hz,J=9.1Hz,2.4Hz, J=7.9Hz,5.5Hz,J=9.7Hz,4.2Hz),4.28-4.45+4.61-4.72 (1H,m×2),6.41+6.43+6.47(1H,s×3),6.63-6.87(4H, m),7.30-7.60(4H,m)

EXAMPLE 33

Compound 33

appearance: yellow oil

IR(neat, $cm^{-1}$): 2938,2824,2776,1659,1611,1584,1518, 1500,1458,1368,1344,1305,1248,1179, 1122, 1089,1032

| High Resolution FAB-MS(m/z, ($C_{32}H_{40}N_2O_6$+H)$^+$): | |
|---|---|
| Calcd.: | 549.2965 |
| Found : | 549.2986 |

$^1$H-NMR(300MHz, $CDCl_3$, δppm):

2.26+2.31(6H,s×2),2.50-3.10(4H,m),2.80(1H,d, J=14.2Hz),3.02(3H,s),3.14-3.27(1H,m),3.35(1H,d, J=14.2Hz), 3.48(2H,s),3.73+3.74(3H,s×2),3.86+ 3.88(3H,s×2),3.87+3.93(3H,s×2),4.70-4.82(1H, m),4.70-4.82+5.95-6.02(1H, m×2),5.03+5.05(1H,dx 2,J=10.7Hz,J=10.7Hz),5.22+5.39(1H,d×2,J=10.7Hz, J=10.7Hz),6.41+6.46(1H,s×2),6.65-6.88(4H,m), 7.30-7.52(4H,m)

EXAMPLE 34

(1) Preparation of 6,7-dimethoxy-8-(3-dimethylamino-methylbenzyloxy)-1-(3-methoxybenzyl)-1,2,3,4-tetrahydroisoquinoline

The desired compound was prepared as a yellow oil (yield: 622 mg) using compound 7 obtained in Example 7 (772 mg, 1.53 mmole), in the same manner as described in Example 30-(1).

FAB-MS(m/z,($C_{29}H_{36}N_2O_4$+H)$^+$): 477

(2) Preparation of compound 34

The captioned compound was prepared as a colorless oil (yield: 42.2 mg) using the compound obtained in (1) (62.1 mg, 0.130 mmole), in the same manner as described in Example 30-(2).

IR(neat, $cm^{-1}$): 2944,1647,1605,1584,1497,1458,1344, 1266,1122,1083,1032

| High Resolution FAB-MS(m/z, ($C_{31}H_{38}N_2O_6$+H)$^+$): | |
|---|---|
| Calcd.: | 535.2808 |
| Found : | 535.2831 |

$^1$H-NMR(300MHz, $CDCl_3$, δppm):

2.23+2.27(6H,s×2),2.68-2.80(1H,m),2.80-3.00(1H, m),2.75(1H,d,J=14.5Hz),2.78(1H,dd,J=10.5Hz, 13.4Hz), 3.09(1H,dd,J=2.6Hz,13.4Hz),3.20-3.40(1H, m),3.40(1H,d,J=14.5Hz),3.46+4.07(2H,s×2),3.65+ 3.69(3H,s×2), 3.86+3.88(3H,s×2),3.86+3.93(3H,sx 2),4.47+5.95(1H,dd×2,J=2.6Hz,10.5Hz,J=4.6Hz, 8.1Hz),4.65-4.76(1H,m), 5.06+5.09(1H,d×2, J=11.2Hz,J=10.9Hz),5.19+5.39(1H,d×2,J=11.2Hz, J=10.9Hz),6.40-6.52(3H,m),6.70+6.71 (1H,dd×2, J=1.9Hz,8.1Hz,J=1.9Hz,8.1Hz),7.05+7.07(1H,t×2, J=7.8Hz,J=7.8Hz),7.30-7.50(4H,m)

Compounds 35~37 (Examples 35~37) were prepared by the condensation of the compound obtained in Example 34-(1) with the corresponding carboxylic acids, 3-hydroxypropionic acid, methoxyacetic acid, N-acetyl-sarcosine, using dimethyformamide, dichloromethane, dimethyformamide as solvents, in the same manner as described in Example 30-(2).

EXAMPLE 35

Compound 35

appearance: pale yellow oil
IR(neat, $cm^{-1}$): 1638,1605,1497,1458,1437,1266,1122, 1092,1032

| High Resolution FAB-MS(m/z, ($C_{32}H_{40}N_2O_6$+H)$^+$): | |
|---|---|
| Calcd.: | 549.2965 |
| Found : | 549.2976 |

$^1$H-NMR(300MHz, $CDCl_3$, δppm):
2.22+2.30(6H,s×2),2.60-2.94(3H,m),3.06-3.26(2H, m),3.30-3.95(4H,m),3.43(2H,s),3.66+3.70(3H,s× 2),3.85+3.88(3H,s×2),3.86+3.93(3H,s×2),4.70-4.82+6.05(2H,m+dd,J=3.4Hz,8.0Hz),5.08+5.11(1H, d×2,J=11.2Hz,J=11.2Hz), 5.38+5.39(1H,d×2, J=11.2Hz,J=11.2Hz),6.40-6.55(3H,m),6.69+6.71(1H, dd×2,J=2.0Hz,7.7Hz,J=2.0Hz,7.7Hz), 7.05+7.07(1H, t×2,J=7.7Hz,J=7.7Hz),7.25-7.50(4H,m)

EXAMPLE 36

Compound 36

appearance: colorless oil
IR(neat, $cm^{-1}$): 2944,1656,1605,1497,1458,1440,1266, 1122,1089

| High Resolution FAB-MS(m/z, ($C_{32}H_{40}N_2O_6$+H)$^+$): | |
|---|---|
| Calcd.: | 549.2965 |
| Found : | 549.2979 |

$^1$H-NMR(300MHz, $CDCl_3$, δppm):
2.25+2.29(6H,s×2),2.50-2.65+2.65-2.77(1H,m×2), 2.80(1H,dd,J=10.6Hz,13.6Hz),2.85(1H,d,J=14.3Hz), 2.77-2.96(1H,m),3.01+3.20(3H,s×2),3.10(1H,dd, J=2.6Hz,13.6Hz),3.15-3.30(1H,m),3.36(1H,d, J=14.3Hz),3.47+3.49 (2H,s×2),3.66+3.68(3H,s×2), 3.85+3.88(3H,s×2),3.86+3.93(3H,s×2),4.70-4.82 (1H,m),4.80+6.05(1H,dd×2, =2.6Hz,10.6Hz, J=3.5Hz,8.9Hz),5.05+5.08(1H,d×2,J=11.0Hz, J=11.0Hz),5.20+5.38(1H,d×2,J=11.0Hz,J=11.0Hz), 6.41+6.46(1H,s×2),6.45-6.58(2H,m),6.66+6.71(1H, dd×2,J=1.8Hz,7.8Hz,J=1.8Hz,7.8Hz),7.05+7.08(1H, t×2,J=7.8Hz, J=7.8Hz),7.31-7.55(4H,m)

EXAMPLE 37

Compound 37

appearance: colorless oil
IR(neat, $cm^{-1}$): 1662,1605,1497,1458,1437,1269,1122, 1089,1032

| High Resolution FAB-MS(m/z, ($C_{34}H_{43}N_3O_6$+H)$^+$): | |
|---|---|
| Calcd.: | 590.3230 |
| Found : | 590.3190 |

$^1$H-NMR(300MHz, $CDCl_3$, δppm):

1.47+2.02+2.09(3H,s×3),2.09(1H,d,J=16.1Hz), 2.20+2.25+2.29(6H,s×3),2.47+2.48+2.79(3H,s×3), 2.60-2.97(2H,m),3.05-3.30(2H,m),3.38-3.75(3H,m), 3.66+3.70+3.73(3H,s×3),3.84+3.88+3.89(3H,s×3), 3.85+3.92+3.94(3H, s×3),4.28+4.37(1H,d×2, J=16.1Hz,J=16.1Hz),4.50-4.60.+4.70-4.82+6.03(2H, m×2+dd,J=3.5Hz,7.6Hz),5.06+5.14+5.26(1H,d×3, J=11.1Hz,J=10.8Hz,J=11.4Hz),5.15+5.38+5.40(1H,d ×3,J=11.1Hz,J=10.8Hz,J=11.4Hz),6.40-6.60(3H, m), 6.63-6.75(1H,m),7.05+7.06+7.12(1H,t×3,J=8.0Hz, J=8.0Hz,J=8.0Hz),7.27-7.52(4H,m)

EXAMPLE 38

Preparation of Compound 38

The compound obtained in Example 34-(1) (57.8 mg, 0.121 mmole) was dissolved in dichloromethane (1 ml), and triethylamine (26 µl, 0.187 mmole) and methane-sulfonyl chloride (15 µl, 0.194 mmole) were added under an argon atmosphere at 0°C, and the mixture was stirred for 1 hour. The reaction mixture was diluted with ethyl acetate, washed successively with a saturated aqueous sodium hydrogencarbonate solution, brine, dried over $MgSO_4$ and concentrated under reduced pressure. The residue was purified by preparative thin-layer chromatography (Merck, silica gel $60F_{254}$/chloroform: methanol=15:1) to give the captioned compound (50.6 mg) as a colorless oil.

IR(neat, $cm^{-1}$): 1602,1584,1497,1461,1320,1263,1146, 1122,1068,1029

| High Resolution FAB-MS(m/z, $(C_{30}H_{38}N_2O_6S+H)^+$): | |
|---|---|
| Calcd.: | 555.2529 |
| Found : | 555.2533 |

$^1$H-NMR(300MHz, $CDCl_3$, δppm):

2.05(3H,s),2.26(6H,s),2.61(1H,ddd,J=1.6Hz,4.2Hz, 16.8Hz),2.71(1H,dd,J=10.7Hz,13.9Hz),3.00(1H,ddd, J=6.4Hz,11.7Hz,16.8Hz),3.19(1H,dd,J=3.7Hz, 13.9Hz),3.38-3.48(1H,m),3.49(2H,s),3.68(3H,s), 3.80-4.00(1H,m), 3.86(3H,s),3.90(3H,s),5.08(1H,d, J=11.0Hz),5.13(1H,dd,J=3.7Hz,10.7Hz),5.31(1H,d, J=11.0Hz),6.43(1H,s),6.52(1H, d,J=7.8Hz),6.55(1H, d,J=2.0Hz),6.70(1H,dd,J=2.0Hz,7.8Hz),7.08(1H,t, J=7.8Hz),7.32-7.44(2H,m),7.46-7.51(2H,m)

EXAMPLE 39

Preparation of Compound 39

The compound obtained in Example 34-(1) (57.5 mg, 0.121 mmole) was dissolved in chloroform (1 ml), and acetic anhydride (22 µl, 0.233 mmole) was added under an argon atmosphere at 0°C, and the mixture was stirred overnight at room temperature. The reaction mixture was diluted with ethyl acetate, washed successively with a saturated aqueous sodium hydrogencarbonate solution, brine, dried over $MgSO_4$. The solvent was evaporated, and the residue was purified by preparative thin-layer chromatography (Merck, silica gel $60F_{254}$/chloroform: methanol=10:1) to give the captioned compound (57.4 mg) as a colorless oil.

IR(neat, $cm^{-1}$): 1647,1605,1497,1458,1428,1266,1122, 1101,1029

| High Resolution FAB-MS(m/z, $(C_{31}H_{38}N_2O_5+H)^+$): | |
|---|---|
| Calcd.: | 519.2859 |
| Found : | 519.2835 |

$^1$H-NMR(300MHz, $CDCl_3$, δppm):

1.22+2.02(3H,s×2),2.22+2.31(6H,s×2),2.40-2.60+ 2.60-2.74(1H,m×2),2.79(1H,dd,J=10.5Hz,13.4Hz), 2.75-2.95(1H,m),3.11(1H,dd,J=2.6Hz,13.4Hz),3.07-3.26(1H,m),3.43(2H,s),3.65+3.69(3H,s ×2), 3.85+3.88(3H,s×2),3.85+3.93(3H,s×2),4.70-4.80 (1H,m),4.80+6.07(1H,dd×2,J=2.6Hz,10.5Hz, J=4.6Hz,8.7Hz),5.02+5.12(1H,d×2,J=10.9Hz, J=10.9Hz),5.17+5.38(1H,d×2,J=10.9Hz,J=10.9Hz), 6.41+6.46(1H,s×2),6.45-6.55(2H,m),6.68+6.71(1H, dd×2, J=2.1Hz,7.8Hz,J=2.1Hz,7.8Hz),7.04+7.07(1H, t×2,J=7.8Hz,J=7.8Hz),7.28-7.50(4H,m)

EXAMPLE 40

Preparation of Compound 40

The compound obtained in Example 30-(1) (54.7 mg, 0.115 mmole) was dissolved in tetrahydrofuran (1.5 ml), and methyl isocyanate (12 µl, 0.203 mmole) was added under an argon atmosphere at 0°C. The mixture was stirred at 0°C for 1 hour and at room temperature for 2 hours. The reaction mixture was concentrated under reduced pressure, and the residue was purified by preparative thin-layer chromatography (Merck, silica gel $60F_{254}$/chloroform:methanol=10:1) to give the captioned compound (59.2 mg) as pale yellow amorphous.

IR(KBr, $cm^{-1}$): 2944,1632,1518,1497,1467,1425,1344, 1248,1122,1086,1032

| High Resolution FAB-MS(m/z, $(C_{31}H_{39}N_3O_5+H)^+$): | |
|---|---|
| Calcd.: | 534.2968 |
| Found : | 534.2981 |

$^1$H-NMR(300MHz, $CDCl_3$, δppm):

2.23(6H,s),2.31(3H,d,J=4.6Hz),2.58(1H,td, J=3.7Hz,14.9Hz),2.70(1H,dd,J=10.6Hz,13.4Hz), 2.78-2.92(1H,m), 3.04-3.13(1H,m),3.07(1H,dd, J=3.0Hz,13.4Hz),3.13-3.30(1H,m),3.41(1H,d, J=12.8Hz),3.47(1H,dJ=12.8Hz),3.75(3H, s),3.87(3H, s),3.91(3H,s),4.29-4.40(1H,m),4.48-4.60(1H,m), 5.05(1H,d,J=11.3Hz),5.34(1H,d,J=11.3Hz),6.44(1H, s),6.69(2H,d,J=8.8Hz),6.78(2H,d,J=8.8Hz),7.30-7.45(4H,m)

EXAMPLE 41

Preparation of Compound 41

The captioned compound was prepared as pale yellow crystals (yield: 57.8mg) using the compound obtained in Example 30-(1) (52.0 mg, 0.109 mmole) and methyl isothiocyanate (8.7 mg, 0.12 mmole), in the same manner described in Example 40.

IR(KBr, $cm^{-1}$): 3406,2938,2830,2776,1611,1584,1518, 1497,1467,1374,1344,1305,1245,1179, 1122, 1086,1032,960,843,819,792

| High Resolution FAB-MS(m/z, $(C_{31}H_{39}N_3O_4S+H)^+$): | |
|---|---|
| Calcd.: | 550.2739 |
| Found : | 550.2742 |

$^1$H-NMR(300MHz, $CDCl_3$, δppm):

2.26(6H,s),2.65(3H,d,J=4.1Hz),2.81(1H,dd, J=10.0Hz,13.4Hz),2.95-3.10(1H,m),3.17(1H,dd, J=2.9Hz,13.4Hz), 3.40-3.58(3H,m),3.74(3H,s),3.87 (3H,s),3.90(3H,s),4.22-4.35(1H,m),4.86-5.17(2H, m),5.02(1H,d,J=11.1Hz),5.31(1H, d,J=11.1Hz),6.45 (1H,s),6.68(2H,d,J=8.6Hz),6.75(2H,d,J=8.6Hz), 7.30-7.50(4H,m)

EXAMPLE 42

Preparation of Compound 42

2-(tert-Butyldimethylsiloxy)ethylamine (20.0 mg, 0.114 mmole) was dissolved in tetrahydrofuran (1 ml), and 1,1'-carbonyldiimidazole (18.5 mg, 0.114 mmole) was added at room temperature under an argon atmosphere, and the mixture was stirred for 30 minutes. To the mixture was added a solution of the compound obtained in Example 30-(1) (30.0 mg, 0.0629 mmole) in tetrahydrofuran (1 ml), and the mixture was stirred at room temperature for 6 hours. The reaction mixture was diluted with ethyl acetate, washed successively with water and brine. The organic layer was dried over $MgSO_4$ and concentrated under reduced pressure. The residue was purified by dry column flash chromatography (silica gel/chloroform: methanol=20:1) to give a pale yellow oil (32.5 mg). This product was dissolved in tetrahydrofuran (2 ml),

and a 1.0 M solution of tetrabutylammonium fluoride in tetrahydrofuran (60 µl) was added at 0°C, and the mixture was stirred for 2 hours. The reaction mixture was diluted with ethyl acetate, washed successively with water and brine, and dried over $MgSO_4$. The solvent was evaporated, and the residue was purified by preparative thin-layer chromatography (Merck, silica gel $60F_{254}$/chloroform:methanol=8:1) to give the captioned compound (20.9 mg) as a colorless oil.

IR(neat, $cm^{-1}$): 3382,2944,2782,1614,1518,1467,1248, 1122,1086,1032,753

| High Resolution FAB-MS(m/z, ($C_{32}H_{41}N_3O_6$+H)$^+$): | |
|---|---|
| Calcd.: | 564.3074 |
| Found : | 564.3055 |

$^1$H-NMR(300MHz, $CDCl_3$, δppm):

2.24(6H,s),2.55-2.67(1H,m),2.72(1H,dd,J=10.4Hz, 13.7Hz),2.74-2.96(3H,m),3.08(1H,dd,J=2.7Hz, 13.7Hz),3.23 (1H,ddd,J=4.5Hz,11.5Hz,13.3Hz),3.33 (2H,t,J=5.1Hz),3.44(1H,d,J=12.7Hz),3.53(1H,d, J=12.7Hz),3.68-3.74(2H,m), 3.75(3H,s),3.87(3H,s), 3.94(3H,s),4.30-4.42(1H,m),4.46-4.58(1H,m),5.00 (1H,d,J=10.9Hz),5.36(1H,d,J=10.9Hz), 6.46(1H,s), 6.70(2H,d,J=8.5Hz),6.80(2H,d,J=8.5Hz),7.31-7.55 (4H,m)

EXAMPLE 43

Preparation of Compound 43

To a solution of the compound obtained in Example 30-(1) (50.3 mg, 0.106 mmole) in dichloromethane (2 ml), were added 4-dimethylaminopyridine (8.0 mg, 0.065 mmole) and dimethylcarbamoyl chloride (15 µl, 0.163 mmole) at 0°C under an argon atmosphere, and the mixture was stirred at room temperature for 6 hours. The reaction mixture was concentrated under reduced pressure, and the residue was purified by dry column flash chromatography (silica gel/chloroform:methanol=30:1) to give the captioned compound (47.8 mg) as a pale yellow oil.

IR(neat, $cm^{-1}$): 1644,1515,1497,1461,1248,1122,1095, 1032

| High Resolution FAB-MS(m/z, ($C_{32}H_{41}N_3O_5$+H)$^+$): | |
|---|---|
| Calcd.: | 548.3124 |
| Found : | 548.3094 |

$^1$H-NMR(300MHz, $CDCl_3$, δppm):

2.25(6H,s),2.41(6H,s),2.58-2.69(1H,m),2.80(1H, dd,J=10.7Hz,14.2Hz),2.80-3.00(1H,m),3.06(1H,dd, J=3.2Hz, 14.2Hz),3.48-3.52(1H,m),3.47(2H,s),3.75-3.88(1H,m),3.75(3H,s),3.85(3H,s),3.90(3H,s),5.06 (1H,d,J=10.9Hz),5.33 (1H,d,J=10.9Hz),5.16(1H,dd, J=3.2Hz,10.7Hz),6.43(1H,s),6.68(2H,d,J=8.6Hz), 6.89(2H,d,J=8.6Hz),7.30-7.53(4H,m)

EXAMPLE 44

Preparation of Compound 44

The compound obtained in Example 30-(1) (29.8 mg, 0.0625 mmole) was dissolved in acetonitrile (2 ml), and chlorosulfonyl isocyanate (12 µl, 0.139 mmole) was added under nitrogen at 0°C, and the mixture was stirred at room temperature for 3 hours. To the mixture was added water (0.2 ml), and the reaction mixture was stirred for 1 hour. The resulting mixture was diluted with ethyl acetate, washed successively with a saturated aqueous sodium hydrogencarbonate solution and brine, and dried over $MgSO_4$. The organic layer was concentrated under reduced pressure and the residue was purified by preparative thin-layer chromatography (Merck, silica gel $60F_{254}$/chloroform:methanol=7:1) to give the captioned compound (5.5 mg) as a pale yellow oil.

IR(neat, $cm^{-1}$): 1584,1515,1497,1467,1344,1248,1122, 1032,753

| High Resolution FAB-MS(m/z, ($C_{30}H_{37}N_3O_5$+H)$^+$): | |
|---|---|
| Calcd.: | 520.2812 |
| Found : | 520.2787 |

$^1$H-NMR(300MHz, $CDCl_3$, δppm):

2.32+2.61(6H,s×2),2.40-3.35(3H,m),2.72(1H,dd, J=10.0Hz,13.7Hz),3.09(1H,dd,J=3.0Hz,13.7Hz),3.59 (2H,s), 3.75+3.84(3H,s×2),3.86+3.87(3H,s×2), 3.91+3.95(3H,s×2),4.25-4.45(1H,m),4.60-4.72(1H, m),5.03+5.06(1H,d×2, J=11.2Hz,J=11.2Hz),5.36+ 5.45(1H,d×2,J=11.2Hz,J=11.2Hz),6.38+6.44(1H,s× 2),6.67(2H,d,J=8.8Hz),6.77(2H,d, J=8.8Hz),7.35-7.60(4H,m)

Compounds 45 and 46 (Examples 45 and 46) were prepared using compound 26 obtained in Example 26, in the same manner as described in Example 30-(1) and Examples 40,30-(2).

EXAMPLE 45

Compound 45

appearance: pale yellow amorphous
IR(KBR, cm$^{-1}$): 1638,1605,1539,1497,1461,1260,1122

| High Resolution FAB-MS(m/z, ($C_{31}H_{39}N_3O_5$+H)$^+$): | |
|---|---|
| Calcd.: | 534.2968 |
| Found : | 534.2996 |

$^1$H-NMR(300MHz, $CDCl_3$, δppm):

2.18(6H,s),2.32(3H,d,J=4.6Hz),2.59(1H,td, J=3.3Hz,16.2Hz),2.76(1H,dd,J=10.0Hz,13.3Hz),2.87 (1H,td, J=5.3Hz,16.2Hz),3.10(1H,dd,J=3.0Hz, 13.3Hz),3.23(1H,ddd,J=3.3Hz,5.3Hz,13.2Hz),3.14-3.19(1H,m),3.43(1H,d, J=12.9Hz),3.56(1H,d, J=12.9Hz),3.65(3H,s),3.87(3H,s),3.90(3H,s),4.24-4.35(1H,m),4.59-4.69(1H,m),5.17(1H,d, J=11.7Hz), 5.55(1H,d,J=11.7Hz),6.36(1H,d,J=7.8Hz),6.45(1H, s),6.47(1H,d,J=2.0Hz),6.68(1H,dd,J=2.0Hz,7.8Hz), 7.04(1H,t,J=7.8Hz),7.30-7.42(3H,m),7.55-7.62(1H, m)

EXAMPLE 46

Compound 46

appearance: colorless oil
IR(neat, cm$^{-1}$): 1650,1605,1497,1458,1266,1122,1083, 756

| High Resolution FAB-MS(m/z, ($C_{31}H_{38}N_2O_6$+H)$^+$): | |
|---|---|
| Calcd.: | 535.2808 |
| Found : | 535.2803 |

$^1$H-NMR(300MHz, $CDCl_3$, δppm):

2.21+2.24(6H,s×2),2.70-3.02(3H,m),2.77(1H,d, J=14.8Hz),3.09(1H,dd,J=3.0Hz,13.7Hz),3.17-3.37 (1H,m),3.35-3.60(2H,m),3.52(1H,d,J=14.8Hz),3.64 (3H,s),3.84+3.88(3H,s×2),3.89+3.92(3H,s×2), 4.45+5.90(1H,dd×2,J=3.0Hz, 10.5Hz,J=4.8Hz, 7.2Hz),4.65-4.76(1H,m),5.14+5.21(1H,d×2, J=11.7Hz,J=11.7Hz),5.39+5.62(1H,d×2,J=11.7Hz,

J=11.7Hz),6.34(1H,d,J=7.6Hz),6.41(1H,d,J=2.0Hz), 6.47(1H,s),6.69+6.70(1H,ddx2,J=2.0Hz,7.6Hz, J=2.0Hz,7.6Hz), 7.03+7.05(1H,tx2,J=7.6Hz, J=7.6Hz),7.30-7.66(4H,m)

EXAMPLE 47

(1) Preparation of 2-chloroacetyl-6,7-dimethoxy-8-(3-dimethylaminomethylbenzyloxy)-1-(4-methoxybenzyl)-1,2,3, 4-tetrahydroisoquinoline

The compound obtained in Example 30-(1) (210 mg, 0.440 mmole) was dissoved in dichloromethane (3 ml), and a solution of chloroacetic acid (92 mg, 0.538 mmole) in dichloromethane (0.5 ml) was added at room temperature under an argon atmosphere, and the mixture was stirred for 30 minutes. The reaction mixture was diluted with ethyl acetate, washed successively with a saturated aqueous sodium hydrogencarbonate solution and brine. The organic layer was dried over $MgSO_4$ and concentrated under reduced pressure to give the desired compound (206 mg) as a pale yellow oil.

FAB-MS(m/z, $(C_{31}H_{37}N_2O_5Cl+H)^+$): 553

(2) Preparation of compound 47

The compound obtained in (1) (100 mg, 0.187 mmole) was dissolved in dimethylformamide, and thioacetic S-acid (20 µl, 0.280 mmole) was added at room temperature under an argon atmosphere, and the mixture was stirred for 6 hours. The reaction mixture was concentrated under reduced pressure, and the residue was purified by preparative thin-layer chromatography (Merck, silica gel $60F_{254}$/chloroform:methanol=10:1) to give the captioned compound (50.1 mg) as a colorless oil.

IR(neat, $cm^{-1}$): 2944,2820,1695,1647,1515,1431,1248, 1122,1089,1032,960,835

| High Resolution FAB-MS(m/z, $(C_{33}H_{40}N_2O_6S+H)^+$): | |
|---|---|
| Calcd.: | 593.2686 |
| Found : | 593.2710 |

$^1$H-NMR(300MHz, $CDCl_3$, δppm):

2.24+2.26(6H,sx2),2.30+2.35(3H,sx2),2.34(1H,d, J=15.4Hz),2.65-2.97(2H,m),2.74(1H,dd,J=10.6Hz, 13.6Hz), 3.12(1H,dd,J=2.2Hz,13.6Hz),3.13-3.27(1H, m),3.28(1H,d,J=15.4Hz),3.49+3.56(2H,sx2),3.75+ 3.76(3H,sx2), 3.86+3.88(3H,sx2),3.94(3H,s), 4.70-4.80(1H,m),4.91+5.95(1H,ddx2,J=2.2Hz, 10.6Hz,J=4.7Hz,8.6Hz),5.02+ 5.09(1H,dx2, J=10.9Hz,J=10.7Hz),5.16+5.43(1H,dx2,J=10.9Hz, J=10.7Hz),6.41+6.46(1H,sx2),6.63-6.85(4H,m), 7.34-7.57(4H,m)

EXAMPLE 48

Preparation of Compound 48

The compound obtained in Example 47-(1) (43.0 mg, 0.077 mmole) was dissolved in dimethyl sulfoxide (0.5 ml), and a 15% aqueous sodium methyl sulfide solution (55 µl) was added at room temperature under an argon atmosphere, and the mixture was stirred overnight. The reaction mixture was diluted with water and extracted with ethyl acetate. The combined organic layers were dried over $MgSO_4$, and concentrated under reduced pressure. The residue was purified by preparative thin-layer chromatography (Merck, silica gel $60F_{254}$/chloroform:methanol=10:1) to give the captioned compound (18.2 mg) as a pale yellow oil.

| High Resolution FAB-MS(m/z, $(C_{32}H_{40}N_2O_5S+H)^+$): | |
|---|---|
| Calcd.: | 565.2736 |
| Found : | 565.2746 |

$^1$H-NMR(300MHz, $CDCl_3$, δppm):

1.92(3H,s),2.29+2.33(6H,s×2),2.61(2H,s),2.73 (1H,dd,J=10.3Hz,13.4Hz),2.80-2.95(1H,m),3.09(1H, dd, =2.8Hz,13.6Hz),3.12-3.24(1H,m),3.53(2H,s), 3.55-3.68(1H,m),3.73+3.75(3H,s×2),3.87+3.88(3H, s×2),3.93+3.95(3H, s×2),4.90+5.95-6.02(1H,dd+m, J=2.0Hz,10.3Hz),4.72-4.81(1H,m),4.98+5.06(1H,d× 2,J=10.8Hz,J=10.8Hz),5.22+5.45 (1H,d×2,J=10.8Hz, J=10.8Hz),6.42+6.47(1H,s×2),6.63-6.73+6.83-6.90 (4H,m×2),7.38-7.55(4H,m)

EXAMPLE 49

Preparation of Compound 49

The compound obtained in Example 47-(1) (70.5 mg, 0.127 mmole) was dissolved in ethanol, and morpholine (350 μl, 4.01 mmole) was added under an argon atmosphere, and the mixture was heated under reflux for 3 hours. The reaction mixture was concentrated under reduced pressure, and ethyl acetate was added to the residue. The mixture was washed with brine, dried over $MgSO_4$ and concentrated under reduced pressure. The residue was purified by preparative thin-layer chromatography (merck, silica gel $60F_{254}$/chloroform:methanol=10:1) to give the captioned compound (67.2 mg) as a colorless oil,

IR(neat, $cm^{-1}$): 1644,1515,1458,1344,1248,1119,1092, 1029

| High Resolution FAB-MS(m/z, $(C_{35}H_{45}N_3O_6+H)^+$): | |
|---|---|
| Calcd.: | 604.3387 |
| Found : | 604.3367 |

$^1$H-NMR(300MHz, $CDCl_3$, δppm):

2.05(1H,d,J=14.4Hz),2.14(4H,t,J=4.6Hz),2.26+2.31 (6H,s×2),2.44(1H,d,J=14.4Hz),2.60-3.00(3H,m), 3.07(1H, dd,J=2.5Hz,13.8Hz),3.14-3.30(1H,m),3.45-3.65(6H,m),3.72+3.75(3H,s ×2),3.86+3.88(3H,s× 2),3.88+3.91(3H,s×2), 4.76+6.06(1H,dd×2, J=5.9Hz,11.9Hz,J=3.5Hz,10.0Hz),5.06+5.20(1H,d× 2,J=10.7Hz,J=11.0Hz),5.24+5.35(1H,d×2, J=10.7Hz, J=11.0Hz),5.10-5.20(1H,m),6.42+6.46(1H,s×2), 6.66+6.69(2H,d×2,J=8.8Hz,J=8.8Hz),6.76+6.87(2H, d×2, J=8.8Hz,J=8.8Hz),7.36-7.57(4H,m)

Compounds 50 and 51 (Examples 50 and 51) were prepared using the compound obtained in Example 34-(1), in the same manner as described in Examples 48 and 47. Compound 52 (Example 52) was prepared using intermediate 5 obtained in Referential Example 5, in the same manners as described in Examples 26,30-(1) and 47. Compound 53 (Example 53) was prepared using compound 20 obtained in Example 20, in the same manners as described in Examples 30-(1) and 47.

EXAMPLE 50

Compound 50

appearance: pale yellow oil

IR(neat, $cm^{-1}$): 1644,1605,1497,1458,1437,1266,1122, 1089

| High Resolution FAB-MS(m/z, $(C_{32}H_{40}N_2O_5S+H)^+$): | |
|---|---|
| Calcd.: | 565.2736 |
| Found : | 565.2765 |

$^1$H-NMR(300MHz, $CDCl_3$, δppm):

1.91(3H,s),2.26+2.28(6H,s×2),2.33(1H,d, J=14.1Hz),2.41(1H,d,J=14.1Hz),2.55-2.80(1H,m), 2.79(1H,dd, J=10.4Hz,13.4Hz),2.80-2.97(1H,m),3.15 (1H,dd,J=2.5Hz,13.4Hz),3.15-3.29(1H,m),3.50(2H, s),3.65+3.66(3H,s×2), 3.86+3.88(3H,s×2),3.87+ 3.94(3H,s×2),4.71-4.82(1H,m),4.99+6.06(1H,dd× 2,J=2.5Hz,10.4Hz,J=4.1Hz,8.8Hz), 5.03+5.08(1H,d× 2,J=10.8Hz,J=10.8Hz),5.21+5.43(1H,d×2,J=10.8Hz, J=10.8Hz),6.48-6.60(3H,m),6.67+6.70 (1H,dd×2, J=2.1Hz,7.5Hz,J=2.1Hz,7.5Hz),7.04+7.05(1H,t×2, J=7.5Hz,J=7.5Hz),7.35-7.55(4H,m)

EXAMPLE 51

Compound 51

appearance: brown oil

IR(neat, $cm^{-1}$): 1698,1650,1605,1497,1458,1437,1269, 1122,1089,1032

| High Resolution FAB-MS(m/z, $(C_{33}H_{40}N_2O_6S+H)^+$): | |
|---|---|
| Calcd.: | 593.2686 |
| Found : | 593.2690 |

$^1$H-NMR(300MHz, $CDCl_3$, δppm):

2.24+2.29(6H,s×2),2.24+2.35(3H,s×2),2.44(1H,d, J=15.4Hz),2.65-2.77(1H,m),2.80(1H,dd,J=10.7Hz, 13.4Hz), 2.75-2.95(1H,m),3.13-3.30(2H,m),3.28(1H, d,J=15.4Hz),3.47+3.54(2H,s×2),3.65+3.68(3H,s× 2),3.85+3.88(3H,s×2), 3.86+3.93(3H,s×2),4.69-4.79(1H,m),4.95-5.03+6.01(1H,m+dd,J=5.8Hz, 8.1Hz),5.01+5.13(1H,d×2,J=10.9Hz,J=10.9 Hz), 5.14+5.39(1H,d×2,J=10.9Hz,J=10.9Hz),6.40-6.55 (3H,m),6.68+6.71(1H,dd×2,J=2.3Hz,7.8Hz,J=2.3Hz, 7.8Hz), 7.04+7.08(1H,t×2,J=7.8Hz,J=7.8Hz),7.30-7.50(4H,m)

EXAMPLE 52

Compound 52

appearance: colorless oil

IR(neat, $cm^{-1}$): 2944,2836,2770,1695,1647,1506,1464, 1344,1227,1122,1089,966,753

| High Resolution FAB-MS(m/z, $(C_{34}H_{42}N_2O_4S+H)^+$): | |
|---|---|
| Calcd.: | 623.2791 |
| Found : | 623.2783 |

$^1$H-NMR(300MHz, $CDCl_3$, δppm):

2.21+2.26(6H,s×2),2.25+2.33(3H,s×2),2.76-3.21 (4H,m),3.36-3.85(5H,m),3.53+3.55(3H,s×2),3.57+ 3.59(3H, s×2),3.80+3.85(3H,s×2),3.86+3.88(3H,s× 2),4.47(1H,ddd,J=3.4Hz,5.9Hz,13.0Hz),5.11+5.25 (1H,d×2,J=11.8Hz, J=11.9Hz),5.19+6.06(1H,dd×2, J=3.0Hz,8.7Hz,J=5.1Hz,9.0Hz),5.32+5.46(1H,d×2, J=11.8Hz,J=11.9Hz),6.33-6.52 (1H,m),6.45+6.50(1H, s×2),6.60-6.70(2H,m),7.22-7.49(3H,m),7.63-7.74 (1H,m)

EXAMPLE 53

Compound 53

appearance: yellow oil

IR(neat, $cm^{-1}$): 2920,2812,1695,1647,1482,1440,1263, 1209,1116,1041,966,753

| High Resolution FAB-MS(m/z, $(C_{34}H_{38}N_2O_7S+H)^+$): | |
|---|---|
| Calcd.: | 619.2478 |
| Found : | 619.2474 |

$^1$H-NMR(300MHz, $CDCl_3$, δppm):

2.23+2.34(3H,s×2),2.33-2.49(4H,m),2.52(1H,d, J=15.4Hz),2.61-2.95(3H,m),3.08-3.27(2H,m),3.38 (1H,d, J=15.4Hz),3.50(2H,s),3.57-3.73(4H,m), 3.66+3.75(3H,s×2),4.63-4.74(1H,m),5.03+5.95-6.05(1H,dd+m,J=2.2Hz, 10.3Hz),5.22+5.35(1H,s+d, J=11.0Hz),5.22+5.44(1H,s+d,J=11.0Hz),5.92+5.97 (1H,d+s,J=7.8Hz),5.93+5.97(1H,d+s, J=7.8Hz),6.31+ 6.37(1H,s×2),6.45-6.56(2H,m),6.64-6.77(1H,m), 7.00-7.12(1H,m),7.26-7.52(4H,m)

EXAMPLE 54

(1) Preparation of 2-chloroacetyl-6,7-dimethoxy-8-(2-dimethylaminomethylbenzyloxy)-1-(3-methoxybenzyl)-1,2,3, 4-tetrahydroisoquinoline

The desired compound was prepared as a pale yellow oil (yield: 83 mg) using compound 26 obtained in Example 26 (104 mg), in the same manner as described in Example 30-(1) and Example 47-(1).

FAB-MS(m/z, $(C_{31}H_{37}N_2O_5Cl+H)^+$): 553

(2) Preparation of compound 54

To a suspension of sodium hydride (6.5 mg, 0.162 mmole,60%) in dimethylformamide (1 ml) was added 2-pyrrolidone (12 µl, 0.158 mmole), and the mixture was stirred at 70°C under an argon atmosphere for 10 minutes. The mixture was cooled to room temperature. To the mixture was added dropwise a solution of the compound obtained in (1) (83.0 mg, 0.150 mmole) in dimethylformamide (1 ml), and the reaction mixture was stirred at room temperature for 1 hour. Water was added to the mixture, and the mixture was extracted with ethyl acetate. The combined organic layers were washed with brine, dried over $MgSO_4$ and concentrated under reduced pressure. The residue was purified by preparative thin-layer chromatography (Merck, silica gel $60F_{254}$/chloroform:methanol=10:1), and was triturated in ether to give the captioned compound (65.4 mg) as a colorless powder.

IR(KBr, $cm^{-1}$): 2944,1692,1659,1605,1497,1458,1269, 1122

| High Resolution FAB-MS(m/z, $(C_{35}H_{43}N_3O_6+H)^+$): | |
|---|---|
| Calcd.: | 602.3230 |
| Found : | 602.3230 |

$^1$H-NMR(300MHz, $CDCl_3$, δppm):

1.80-2.05(2H,m),2.13(1H,d,J=16.3Hz),2.22(6H,s), 2.20-2.40(2H,m),2.55(1H,dt,J=5.4Hz,8.4Hz),2.60-3.00(2H, m),2.81(1H,dd,J=10.7Hz,13.2Hz),3.13(1H, dd,J=2.6Hz,13.2Hz),3.15-3.30(2H,m),3.45-3.60(2H, m),3.62+3.65(3H, s×2),3.84+3.89(3H,s×2), 3.87+3.91(3H,s×2),4.02(1H,d,J=16.3Hz),4.70-4.78+5.98(1H,m+dd,J=4.8Hz,14.9Hz),4.72-4.81(1H, m),5.12+5.31(1H,d×2,J=11.9Hz,J=11.9Hz), 5.39+5.59(1H,d×2,J=11.9Hz,J=11.9Hz),6.32(1H,d, J=7.6Hz), 6.35-6.55(1H,m),6.46(1H,s),6.65+6.71 (1H,dd×2,J=1.8Hz,7.8Hz,J=1.8Hz,7.8Hz),7.04(1H, t,J=7.8Hz),7.25-7.45(3H, m),7.59-7.70(1H,m)

EXAMPLE 55

Preparation of Compound 55

To a suspension of 6,7-dimethoxy-1-(4-methoxybenzyl)-1,2,3,4-tetrahydroisoquinolin-8-ol (50.3 mg, 0.153 mmole) in dichloromethane (1.5 ml) were added triethylamine (25 µl, 0.18 mmole) and methyl chloroformate (14 µl, 0.18 mmole) at 0°C, and the reaction mixture was stirred at room temperature for 4 hours. The reaction mixture was diluted with dichloromethane, washed successively with 10% aqueous citric acid and brine. The organic layer was dried over $MgSO_4$ and concentrated under reduced pressure to give a yellow oil. Using this product, the captioned compound was prepared as a pale yellow oil (22.5 mg) in the same manner as described in Example 1-(1).

IR(neat, $cm^{-1}$): 2944,2770,1704,1608,1518,1458,1368, 1248,1119,1032,840,792,759

| High Resolution FAB-MS(m/z, ($C_{31}H_{38}N_2O_6$+H)$^+$): | |
|---|---|
| Calcd.: | 535.2808 |
| Found : | 535.2841 |

$^1$H-NMR(300MHz, $CDCl_3$, δppm):

2.24(6H,s),2.40-2.91(2H,m),2.69(1H,dd,J=10.3Hz, 13.7Hz),3.06(1H,dd,J=3.2Hz,13.7Hz),3.22+3.58(3H, s×2), 3.28-3.42(1H,m),3.46(2H,s),3.74(3H,s),3.87 (3H,s),3.86+3.90(3H,s×2),4.20(1H,ddd,J=2.7Hz, 6.2Hz,8.7Hz),5.04 (1H,d,J=10.8Hz),5.18+5.30(1H,d ×2,J=10.8Hz,J=10.8Hz),5.24+5.57(1H,dd×2, J=3.2Hz,10.3Hz,J=3.2Hz,10.3Hz), 6.40+6.45(1H,s× 2),6.68(2H,d,J=8.8Hz),6.78+6.84(2H,d×2,J=8.8Hz, J=8.8Hz),7.30-7.50(4H,m)

Compounds 56 and 57 (Examples 56 and 57) were prepared using methanesulfonyl chloride, acetyl chloride in the same manner as described in Example 55.

EXAMPLE 56

Compound 56

appearance: pale yellow oil

| High Resolution FAB-MS(m/z, ($C_{30}H_{38}N_2O_6S$+H)$^+$): | |
|---|---|
| Calcd.: | 555.2529 |
| Found : | 555.2511 |

$^1$H-NMR(300MHz, $CDCl_3$, δppm):

2.04(3H,s),2.25(6H,s),2.57-2.68(1H,m),2.65(1H, dd,J=10.9Hz,13.9Hz),2.94-3.08(1H,m),3.13(1H,dd, J=3.4Hz, 13.9Hz),3.38-3.45(1H,m),3.47(2H,s),3.75 (3H,s),3.84-3.89(1H,m),3.86(3H,s),3.91(3H,s), 5.02(1H,dd,J=3.4Hz, 10.9Hz),5.04(1H,d,J=10.9Hz), 5.32(1H,d,J=10.9Hz),6.43(1H,s),6.67-6.72(2H,m), 6.79-6.83(2H,m),7.34-7.51(4H,m)

EXAMPLE 57

Compound 57

appearance: colorless oil

IR(neat, $cm^{-1}$): 2938,1650,1608,1518,1458,1428,1368, 1364,1248,1122,1098,1029,834,753

| High Resolution FAB-MS(m/z, ($C_{31}H_{38}N_2O_5$+H)$^+$): | |
|---|---|
| Calcd.: | 519.2859 |
| Found : | 519.2854 |

$^1$H-NMR(300MHz, $CDCl_3$, δppm):

1.19(3H,s),2.22+2.25(6H,s×2),2.62-2.92(2H,m), 2.73(1H,dd,J=10.7Hz,13.7Hz),3.06(1H,dd,J=2.7Hz, 13.7Hz), 3.11-3.28(1H,m),3.42(2H,s),3.74+3.75(3H, s×2),3.85+3.88(3H,s×2),3.93(3H,s),4.72+5.96-6.05(1H,dd+m,J=2.7Hz, 10.7Hz),4.69-4.80(1H,m), 5.03+5.08(1H,d×2,J=11.1Hz,J=11.1Hz),5.16+5.40 (1H,d×2,J=11.1Hz,J=11.1Hz),6.40+6.45 (1H,s×2), 6.65-6.83(4H,m),7.30-7.50(4H,m)

EXAMPLE 58

(1) Preparation of 6,7-dimethoxy-1-(4-methoxybenzyl)-2-(N,N-dimethylaminoacetyl)-1,2,3,4-tetrahydroisoquinolin-8-ol

To a suspension of 6,7-dimethoxy-1-(4-methoxybenzyl)-1,2,3,4-tetrahydroisoquinolin-8-ol (103 mg, 0.312 mmole) in dichloromethane (2 ml) were added N,N-dimethylglysine hydrochloride (52.2 mg, 0.374 mmole) and HOBt (57.3 mg, 0.374 mmole) at room temperature under nitrogen. Then triethylamine (56.5 µl, 0.405 mmole) and EDCI (71.7 mg, 0.374 mmole) were added to the mixture at 0°C. The reaction mixture was stirred at 0°C for 1.5 hours and at room temperature for 5 hours. The reaction mixture was concentrated under reduced pressure, and water was added to the residue. The mixture was extracted with chloroform. The combined extracts were successively washed with a saturated aqueous sodium hydrogencarbonate solution and brine, dried over $MgSO_4$, and concentrated under reduced pressure. The residue was recrystallized from methanol to give the desired compound (78.4 mg) as colorless crystals.

m.p.: 185-187°C

FAB-MS(m/z, $(C_{23}H_{30}N_2O_5+H)^+$): 415

(2) Preparation of compound 58

The captioned compound was prepared as a colorless oil (yield: 35.6 mg) using the compound obtained in (1) in the same manner as described in Example 1-(1).

IR(neat, $cm^{-1}$): 2938,2776,1611,1500,1344,1122,1035

| High Resolution FAB-MS(m/z, $(C_{33}H_{43}N_3O_5+H)^+$): | |
|---|---|
| Calcd.: | 562.3281 |
| Found : | 562.3257 |

$^1$H-NMR(300MHz, $CDCl_3+D_2O$, δppm):

1.96+2.08(6H,s×2),1.99(1H,d,J=14.4Hz),2.25+2.26 (6H,s×2),2.40(1H,d,J=14.4Hz),2.77(1H,dd, J=10.6Hz, 13.6Hz),2.60-2.95(2H,m),3.10(1H,dd, J=2.6Hz,13.6Hz),3.06-3.25(1H,m),3.48+3.49(2H,s× 2),3.72+3.75(3H,s×2), 3.86+3.88(3H,s×2),3.87+ 3.92(3H,s×2),4.67-4.75(1H,m),5.04(1H,d, J=10.9Hz),5.22-5.26(1H,m),5.36(1H,d,J=10.9Hz), 6.41+6.47(1H,s×2),6.66-6.73(2H,m),6.79-6.89(2H, m),7.33-7.49(4H,m)

Compounds 59 and 60 (Examples 59 and 60) were prepared by the reaction of 6,7-dimethoxy-1-(3-methoxybenzyl)-1,2,3,4-tetrahydroisoquinolin-8-ol with the corresponding chlorides, 3-chloromethyl-N,N-dimethyl-benzylamine hydrochloride, N-(3-chloromethylbenzyl)-N′-methylpiperazine dihydrochloride, in the same manner as described in Example 55.

EXAMPLE 59

Compound 59

appearance: pale yellow oil

IR(neat, $cm^{-1}$): 2944,1704,1605,1497,1455,1341,1251, 1119,1032

| High Resolution FAB-MS(m/z, $(C_{31}H_{38}N_2O_6+H)^+$): | |
|---|---|
| Calcd.: | 535.2808 |
| Found : | 535.2834 |

$^1$H-NMR(300MHz, $CDCl_3$, δppm):

2.24(6H,s),2.40-2.53+2.63(1H,m+td,J=2.9Hz, 14.6Hz),2.76(1H,dd,J=10.3Hz,13.5Hz),2.75-2.92 (1H,m),3.11 (1H,dd,J=3.2Hz,13.5Hz),3.24+3.60(3H,s ×2),3.28-3.45(1H,m),3.45(2H,s),3.66+3.67(3H,s× 2),3.85+3.89(3H,s×2), 3.87(3H,s),4.19(1H,ddd, J=2.9Hz,6.4Hz,13.2Hz),5.05+5.07(1H,d×2, J=11.0Hz,J=11.0Hz),5.17+5.28(1H,d×2,J=11.0Hz, J=11.0Hz),5.30+5.62(1H,dd×2,J=3.2Hz,10.3Hz, J=3.2Hz,10.3Hz),6.41+6.45(1H,s×2),6.45-6.60(2H, m),6.67(1H,dd,J=1.1Hz,8.1Hz),7.06(1H,t,J=8.1Hz), 7.25-7.50(4H,m)

EXAMPLE 60

Compound 60

appearance: colorless oil
IR(neat, $cm^{-1}$): 2944,2800,1704,1605,1497,1458,1341, 1248,1164,1119,1014

| High Resolution FAB-MS(m/z, ($C_{34}H_{43}N_3O_6$+H)$^+$): | |
|---|---|
| Calcd.: | 590.3230 |
| Found : | 590.3209 |

$^1$H-NMR(300MHz, $CDCl_3$, δppm):
2.28(3H,s),2.33-2.60(8H,m),2.50-2.70(1H,m),2.75 (1H,dd,J=10.1Hz,13.4Hz),2.75-2.91(1H,m),3.10(1H, dd, J=3.6Hz,13.4Hz),3.24+3.60(3H,s×2),3.25-3.46 (1H,m),3.53(2H,s),3.66+3.67(3H,s×2),3.85+3.90 (3H,s×2),3.87 (3H,s),4.13-4.24(1H,m),5.04+5.07 (1H,d×2,J=11.1Hz,J=11.1Hz),5.15+5.26(1H,d×2, J=11.1Hz,J=11.1Hz),5.29+5.61 (1H,dd×2,J=3.6Hz, 10.1Hz,J=4.7Hz,8.2Hz),6.42+6.45(1H,s×2),6.47-6.59(2H,m),6.68(1H,d,J=8.2Hz,),7.06(1H,t, J=8.2Hz),7.30-7.50(4H,m)

EXAMPLE 61

(1) Preparation of 6,7-dimethoxy-1-(3-methoxybenzyl)-2-(N-methylcarbamoyl)-1,2,3,4-tetrahydroisoquinolin-8-ol

To a solution of 6,7-dimethoxy-1-(3-methoxybenzyl)-1,2,3,4-tetrahydroisoquinolin-8-ol (350 mg, 1.06 mmole) in dichloromethane (11 ml) was added methyl isocyanate (69 μl, 1.17 mmole) at room temperature under an argon atmosphere, and the reaction mixture was stirred for 3.5 hours. The reaction mixture was concentrated under reduced pressure, and the residue was purified by column chromatography (silica gel/chloroform:methanol=20:1) to give the desired compound (410 mg) as a colorless powder.
m.p.: 75-80°C
FAB-MS (m/z, ($C_{21}H_{26}N_2O_5$+H)$^+$): 387

(2) Preparation of compound 61

The captioned compound was prepared as a yellow oil (yield: 58.1 mg) using the compound obtained in (1) (57.2 mg, 0.148 mmole) and 2-chloromethylpyridine hydrochloride (32.0 mg, 0.195 mmole) in the same manner as described in Example 1-(1).
IR(neat, $cm^{-1}$): 3376,2938,1635,1608,1542,1497,1461, 1440,1374,1344,1260,1194,1152,1122, 1086, 1044,1005,759

| High Resolution FAB-MS(m/z, ($C_{27}H_{31}N_3O_5$+H)$^+$): | |
|---|---|
| Calcd.: | 478.2342 |
| Found : | 478.2359 |

$^1$H-NMR(300MHz, $CDCl_3$, δppm):
2.44(3H,d,J=4.7Hz),2.56-2.68(1H,m),2.70-2.92(2H, m),3.10(1H,dd,J=3.2Hz,13.4Hz),3.24-3.36(1H,m), 3.69 (3H,s),3.86(3H,s),3.90(3H,s),4.03-4.10(1H, m),4.29-4.37(1H,m),5.10-5.18(1H,m),5.11(1H,d, J=12.0Hz),5.41(1H,d, J=12.0Hz),6.46(1H,s),6.52-6.60(2H,m),6.68-6.71(1H,m),7.08(1H,t,J=7.8Hz), 7.25-7.31(1H,m),7.53(1H,d,J=8.5Hz), 7.76(1H,t, J=8.5Hz),8.55-8.60(1H,m)

Compounds 62 and 63 (Examples 62 and 63) were prepared using 3-chloromethylpyridine hydrochloride, 4-chloromethylpyridine hydrochloride in the same manner as described in Example 61-(2). Compounds 64~67 (Examples 64~67) were prepared using the compound obtained in Example 61-(1), in the same manner as described in Example 22-(1) followed by the reaction with the corresponding amines, methylamine, piperazine, N-methylpiperazine, morpholine in the same manner as described in Example 22-(2).

EXAMPLE 62

Compound 62

appearance: yellow oil

IR(neat, $cm^{-1}$): 3376,2944,1635,1605,1584,1539,1497, 1470,1437,1374,1341,1263,1221,1152, 1122, 1086,1029,756

| High Resolution FAB-MS(m/z, $(C_{27}H_{31}N_3O_5+H)^+$): | |
|---|---|
| Calcd.: | 478.2342 |
| Found : | 478.2365 |

$^1$H-NMR(300MHz, $CDCl_3$, δppm):

2.42(3H,d,J=4.9Hz),2.75-2.92(1H,m),2.82(1H,dd, J=9.7Hz,13.1Hz),3.07(1H,dd,J=3.7Hz,13.1Hz),3.23-3.33 (1H,m),3.34-3.42(1H,m),3.69(3H,s),3.86(3H, s),3.87(3H,s),4.08-4.18(1H,m),4.76-4.84(1H,m), 5.12(1H,d,J=11.6Hz), 5.23(1H,d,J=11.6Hz),6.47(1H, s),6.48-6.56(2H,m),6.68-6.74(1H,m),7.11(1H,t, J=8.1Hz),7.33(1H,dd,J=4.9Hz,7.6Hz), 7.77-7.82(1H, m),8.58-8.64(1H,m),8.71-8.77(1H,m)

EXAMPLE 63

Compound 63

appearance: pale yellow oil

| High Resolution FAB-MS(m/z, $(C_{27}H_{31}N_3O_5+H)^+$): | |
|---|---|
| Calcd.: | 478.2342 |
| Found : | 478.2320 |

$^1$H-NMR(300MHz, $CDCl_3$, δppm):

2.47(3H,d,J=4.6Hz),2.50-2.70(1H,m),2.78-2.95(2H, m),3.08(1H,dd,J=4.1Hz,12.2Hz),3.29-3.42(1H,m), 3.49-3.60(1H,m),3.66(3H,s),3.78(3H,s),3.87(3H, s),3.94-4.10(1H,m),5.01(1H,dd,J=4.1Hz,8.7Hz), 5.11(2H,s),6.48(1H,s), 6.50-6.70(2H,m),6.66-6.76 (1H,m),7.10(1H,t,J=7.7Hz),7.41(2H,d,J=5.4Hz), 8.12-8.30(2H,m)

EXAMPLE 64

Compound 64

appearance: pale yellow powder

IR(KBr, $cm^{-1}$): 2944,1608,1545,1497,1470,1260,1122, 753

| High Resolution FAB-MS(m/z, ($C_{30}H_{37}N_3O_5$+H)$^+$): | |
|---|---|
| Calcd.: | 520.2812 |
| Found : | 520.2804 |

$^1$H-NMR(300MHz, $CDCl_3$, δppm):

2.46(3H,s),2.58(3H,d,J=4.4Hz),2.50-2.64(1H,m), 2.73(1H,dd,J=9.5Hz,13.4Hz),2.75-2.87(1H,m),2.98 (1H,dd, J=4.0Hz,13.4Hz),3.40-3.51(1H,m),3.52-3.70 (1H,m),3.68(3H,s),3.86(3H,s),3.92(3H,s),3.88(1H, d,J=12.1Hz),4.11 (1H,d,J=12.1Hz),4.94(1H,d, J=11.8Hz),5.41(1H,d,J=11.8Hz),5.26-5.40(1H,m), 6.36(1H,d,J=7.7Hz),6.40(1H,d, J=2.1Hz),6.42(1H,s),6.68(1H,dd,J=2.1Hz,7.7Hz),7.07(1H,t,J=7.7Hz), 7.35(1H,d,J=7.6Hz),7.39(1H,t,J=7.6Hz),7.58 (1H,d, J=7.6Hz),7.61(1H,s)

EXAMPLE 65

Compound 65

appearance: colorless powder

IR(KBr, $cm^{-1}$): 1635,1605,1539,1497,1461,1341, 1263, 1122,753

| High Resolution FAB-MS(m/z, ($C_{33}H_{42}N_4O_5$+H)$^+$): | |
|---|---|
| Calcd.: | 575.3234 |
| Found : | 575.3244 |

$^1$H-NMR(300MHz, $CDCl_3$, δppm):

2.33(3H,d,J=4.6Hz),2.38-2.50(4H,m),2.56(1H,td, J=4.0Hz,16.1Hz),2.74(1H,dd,J=10.0Hz,13.3Hz), 2.75-2.90 (1H,m),2.85-2.97(4H,m),3.11(1H,dd, J=2.8Hz,13.3Hz),3.16-3.30(1H,m),3.52(2H,s),3.69 (3H,s),3.87(3H,s),3.91 (3H,s),4.15-4.25(1H,m), 4.63(1H,dd,J=2.8Hz,10.0Hz),5.13(1H,d,J=11.2Hz), 5.29(1H,d,J=11.2Hz),6.44(1H,s),6.46 (1H,d, J=7.7Hz),6.51(1H,d,J=2.4Hz),6.70(1H,dd,J=2.4Hz, 7.7Hz),7.07(1H,t,J=7.7Hz),7.25-7.45(4H,m)

EXAMPLE 66

Compound 66

appearance: pale yellow powder

IR(KBr, $cm^{-1}$): 1635,1605,1539,1497,1461,1344,1260, 1122

| High Resolution FAB-MS(m/z, ($C_{34}H_{44}N_4O_5$+H)$^+$): | |
|---|---|
| Calcd.: | 589.3390 |
| Found : | 589.3385 |

$^1$H-NMR(300MHz, $CDCl_3$, δppm):

2.30(3H,s),2.33(3H,d,J=4.6Hz),2.35-2.60(8H,m), 2.57(1H,td,J=3.6Hz,16.2Hz),2.76(1H,dd,J=10.1Hz, 13.3Hz), 2.79-2.92(1H,m),3.12(1H,dd,J=3.0Hz, 13.3Hz),3.15-3.30(1H,m),3.50(2H,s),3.69(3H,s), 3.87(3H,s),3.91(3H,s),4.23-4.33(1H,m),4.62(1H, dd,J=3.0Hz,10.1Hz),5.11(1H,d,J=11.1Hz),5.29(1H, d,J=11.1Hz),6.45(1H,s),6.48(1H,d,J=7.5Hz), 6.52 (1H,d,J=2.5Hz),6.69(1H,dd,J=2.5Hz,7.5Hz),7.07 (1H,t,J=7.5Hz),7.31-7.45(4H,m)

EXAMPLE 67

Compound 67

appearance: colorless powder

IR(KBr, $cm^{-1}$): 2944,1638,1605,1536,1497,1458,1341, 1266,1119

| High Resolution FAB-MS(m/z, ($C_{33}H_{41}N_3O_6$+H)$^+$): | |
|---|---|
| Calcd.: | 576.3074 |
| Found : | 576.3058 |

$^1$H-NMR(300MHz, $CDCl_3$, δppm):

2.33(3H,d,J=4.6Hz),2.40(4H,t,J=4.6Hz),2.57(1H, td,J=3.4Hz,15.9Hz),2.76(1H,dd,J=10.0Hz,13.3Hz), 2.75-2.90(1H,m),3.12(1H,dd,J=10.0Hz,13.3Hz), 3.15-3.30(1H,m),3.46(1H,d,J=13.4Hz),3.50(1H,d, J=13.4Hz),3.66 (4H,t,J=4.6Hz),3.69(3H,s),3.87(3H, s),3.90(3H,s),4.20-4.32(1H,m),4.62(1H,dd, J=2.8Hz,10.0Hz),5.12(1H,d, J=11.2Hz),5.29(1H,d, J=11.2Hz),6.45(1H,s),6.48(1H,d,J=7.6Hz),6.53(1H, d,J=2.2Hz),6.70(1H,dd,J=2.2Hz,7.6Hz), 7.07(1H,t, J=7.6Hz),7.30-7.48(4H,m)

EXAMPLE 68

Preparation of Compound 68

To a solution of compound 30 obtained in Example 30-(2) (25.4 mg, 0.0475 mmole) in dichloromethane (0.6 ml) were added 4-dimethylaminopyridine (5.8 mg, 0.047 mmole) and acetic anhydride (5.3 µl, 0.056 mmole), and the reaction mixture was stirred at room temperature for 2 hours. The reaction mixture was concentrated under reduced pressure, and the residue was purified by preparative thin-layer chromatography (Merck, silica gel $60F_{254}$/chloroform:methanol=10:1) to give the captioned compound (20.4 mg) as a colorless oil.

IR(neat, $cm^{-1}$): 2944,2820,2765,1755,1671,1614,1515, 1458,1371, 1248,1122,1032,838,785

| High Resolution FAB-MS(m/z, ($C_{33}H_{40}N_2O_7$+H)$^+$): | |
|---|---|
| Calcd.: | 577.2914 |
| Found : | 577.2945 |

$^1$H-NMR(300MHz, $CDCl_3$, δppm):

2.03+2.16(3H,sx2),2.24+2.29(6H,sx2),2.64-2.96 (2H,m),2.74(1H,dd,J=10.5Hz,13.7Hz),3.02(1H,d, J=14.4Hz), 3.07(1H,dd,J=2.0Hz,13.7Hz),3.20(1H,dt, J=4.5Hz,12.5Hz),3.46+3.55(2H,sx2),3.74+3.76(3H, sx2),3.85+3.88(3H,sx2), 3.86+3.93(3H,sx2),4.17 (1H,d,J=14.4Hz),4.55+5.90(1H,ddx2,J=2.0Hz, 10.5Hz,J=5.5Hz,7.3Hz),4.67-4.77(1H,m), 5.03+5.09 (1H,dx2,J=11.0Hz,J=11.0Hz),5.41(1H,d,J=11.0Hz), 6.39+6.45(1H,sx2),6.64-6.82(4H,m),7.31-7.50(4H, m)

EXAMPLE 69

Preparation of Compound 69

The captioned compound was prepared as a colorless oil (yield: 21.8 mg) using compound 30 obtained in Example 30-(2) (55.4 mg, 0.104 mmole), in the same manner as described in Example 30-(2).

IR(neat, $cm^{-1}$): 3412,2944,2836,1755,1645,1611,1515, 1457,1240,1179,1122,1080,1032,753

High Resolution FAB-MS(m/z, ($C_{33}H_{40}N_2O_8$+H)$^+$): 593

$^1$H-NMR(300MHz, $CDCl_3$, δppm):

2.24+2.29(6H,sx2),2.43-2.95(3H,m),2.74(1H,dd, J=10.5Hz,13.9Hz),3.06(1H,dd,J=2.3Hz,13.9Hz), 3.11-3.29 (1H,m),3.16(1H,d,J=14.2Hz),3.47(2H,s), 3.76+3.77(3H,sx2),3.86+3.88(3H,sx2),3.93+3.94 (3H,sx2),4.08(1H,d, J=14.2Hz),4.07-4.35(2H,m), 4.50+5.83(1H,ddx2,J=2.3Hz,10.5Hz,J=5.7Hz, 7.8Hz),4.60-4.71(1H,m),5.14+5.16(1H,d×2, J=11.2Hz,J=11.2Hz),5.39+5.40(1H,d×2,J=11.2Hz, J=11.2Hz),6.39+6.46(1H,s×2),6.63-6.71(4H,m), 7.29-7.48(4H,m)

EXAMPLE 70

Preparation of Compound 70

To a solution of compound 47 obtained in Example 47-(2) (19.5 mg, 0.0329 mmole) in methanol (0.5 ml) was added an aqueous potassium hydroxide solution (1N, 65 μl) under an argon atmosphere, and the mixture was stirred at room temperature for 4 hours. The reaction mixture was concentrated under reduced pressure, and the residue was purified by preparative thin-layer chromatography (Merck, silica gel $60F_{254}$/chloroform: methanol=10:1) to give the captioned compound (14.5 mg) as a colorless oil.

IR(neat, $cm^{-1}$): 2938,2820,1644,1515,1458,1434, 1248,1122,1089,1032,753

| High Resolution FAB-MS(m/z, $(C_{31}H_{38}N_2O_5S+H)^+$): | |
|---|---|
| Calcd.: | 551.2579 |
| Found : | 551.2584 |

$^1$H-NMR(300MHz, $CDCl_3$, δppm):

2.27+2.30(6H,s×2),2.28-2.57(2H,m),2.59-2.98(3H, m),3.01-3.39(3H,m),3.42-3.63(2H,m),3.69+3.72(3H, s×2), 3.76+3.86(3H,s×2),3.89+3.95(3H,s×2),4.57-4.71(1H,m),4.71-4.86+5.96-5.97(1H,m ×2),4.93-5.11(1H,m),5.33-5.51 (1H,m),6.39+6.47(1H,s ×2), 6.56-6.87(4H,m),7.33-7.58(4H,m)

EXAMPLE 71

Preparation of Compound 71

The captioned compound was prepared as a yellow oil (yield: 48.3 mg) using intermediate 2 obtained in Referential Example 2 (90.0 mg, 0.252 mmole) and 3-nitrobenzyl chloride (56.1 mg, 0.327 mmole) in the same manner as described in Example 1-(1).

FAB-MS(m/z, $(C_{27}H_{28}N_2O_7+H)^+$): 493

$^1$H-NMR(300MHz, $CDCl_3$, δppm):

2.60-3.00(2H,m),3.09(1H,dd,J=2.7Hz,13.7Hz),3.14-3.28(1H,m),3.45-3.53(1H,m),3.69(3H,s),3.88(3H, s),3.89 (3H,s),4.43(1H,ddd,J=2.5Hz,6.3Hz,12.6Hz), 4.55+5.71-5.77(1H,dd+m.J=2.7Hz,10.5Hz),5.19+5.25 (1H,d×2,J=12.0Hz, 12.0Hz),5.39(1H,d,J=12.0Hz), 6.39+6.44(1H,s×2),6.47-6.60(2H,m),6.69-6.80(1H, m),7.08+7.12(1H,t×2,J=7.7Hz, 7.7Hz),7.43+8.00 (1H,s×2),7.58+7.60(1H,t×2,J=7.8Hz,7.8Hz),7.78-7.88(1H,m),8.19-8.30(1H,m),8.36-8.42(1H,m)

EXAMPLE 72

Preparation of Compound 72

To a solution of Compound 71 obtained in Example 71 (48.0 mg, 0.0975 mmole) in ethanol (2 ml) was added tin(II) chloride (93.2 mg, 0.492 mmole) under nitrogen, and the reaction mixture was stirred at 70°C for 1 hour. To the mixture was added an aqueous sodium hydrogen-carbonate solution, and the mixture was extracted with chloroform. The combined extracts were dried over $MgSO_4$, and concentrated under reduced pressure. The residue was purified by preparative thin-layer chromatography (Merck, silica gel $60F_{254}$/chloroform: methanol=20:1) to give the captioned compound (29.2 mg) as a yellow oil.

IR(neat, $cm^{-1}$): 3370,2938,1668,1605,1497,1458,1437, 1374,1344,1296,1266,1236,1188,1155, 1122,1083, 1032,1005,963,861,753,699

| High Resolution FAB-MS(m/z, ($C_{27}H_{30}N_2O_5$+H)$^+$): | |
|---|---|
| Calcd.: | 463.2233 |
| Found : | 463.2270 |

$^1$H-NMR(300MHz, $CDCl_3$, δppm):
2.71(1H,dd,J=11.0Hz,13.6Hz),2.62-2.92(2H,m),3.15 (1H,dd,J=2.4Hz,13.6Hz),3.10-3.21(1H,m),3.69+3.73 (3H, s×2),3.85+3.88(3H,s×2),3.89+3.93(3H,s×2), 4.33-4.42+5.85-5.90(2H,m×2),5.01+5.02(1H,d×2, J=11.0Hz,J=11.0Hz), 5.23+5.24(1H,d×2,J=11.0Hz, J=11.0Hz),6.39+6.46(1H,s×2),6.51-6.60(2H,m), 6.62-6.68(1H,m),6.69-6.80(3H,m),7.07-7.20(2H,m), 7.22+7.97(1H,s×2)

EXAMPLE 73

Preparation of Compound 73

To a solution of compound 24 (20.0 mg, 0.0355 mmole) in acetone (2 ml) was added an aqueous oxone solution (2 ml, 0.0223 mmole), and the reaction mixture was stirred at room temperature for 15 hours. Water was added to the mixture, and the mixture was extracted with chloroform. The combined extracts were dried over $MgSO_4$, and concentrated under reduced pressure. The residue was purified by preparative thin-layer chromatography (Merck, silica gel $60F_{254}$/chloroform: methanol=20:1) to give the captioned compound (14.3 mg) as a colorless oil.
IR(neat, $cm^{-1}$): 2938,2830,1671,1605,1584,1497,1458, 1437,1368,1344,1269,1236,1188,1155, 1122,1083, 1056,1032,960,753,699

| High Resolution FAB-MS(m/z, ($C_{32}H_{38}N_2O_6S$+H)$^+$): | |
|---|---|
| Calcd.: | 579.2529 |
| Found : | 579.2535 |

$^1$H-NMR(300MHz, $CDCl_3$, δppm):
2.56-3.32(13H,m),3.53(2H,s),3.67+3.71(3H,s×2), 3.86+3.88(3H,s×2),3.93(3H,s),4.29-4.42+5.72-5.78(2H,m ×2),5.12+5.16(1H,d×2,J=11.4Hz, J=11.4Hz),5.28+5.32(1H,d×2,J=11.4Hz,J=11.4Hz), 6.40-6.54(3H,m),6.66-6.73(1H, m),7.05+7.10(1H,× 2,J=7.9Hz,J=7.9Hz),7.19-7.53(4H,m),7.35-7.97(1H, s×2)

EXAMPLE 74

(1) Preparation of (-) optical isomer of Compound 1

The captioned compound was prepared as a colorless oil using (-)-6,7-dimethoxy-1-(4-methoxybenzyl)-1,2,3,4-tetrahydroisoquinolin-8-ol obtained in Referential Example 11-(1), in the same manner as described in Referential Example 1 and Example 1-(1).
Optical Rotation: $[\alpha]_D^{25}$ =-83.0° (C=0.684, chloroform)

(2) Preparation of (+) optical isomer of compound 1

The captioned compound was prepared as a colorless oil using (+)-6,7-dimethoxy-1-(4-methoxybenzyl)-1,2,3,4-tetrahydroisoquinolin-8-ol obtained in Referential Example 11-(2), in the same manner as described in Referential Example 1 and Example 1-(1).
Optical Rotation: $[\alpha]_D^{25}$ =+82.1° (C=0.702, chloroform)
Compound 75 (Example 75) was prepared using compound 4 obtained in Example 4, in the same manner as described in Example 30.

EXAMPLE 75

Compound 75

appearance: yellow oil

IR(neat, $cm^{-1}$): 2932,2854,2776,1650,1611,1584,1518, 1500,1461,1401,1374,1344,1305,1248, 1179, 1122,1083,1032,831

| High Resolution FAB-MS(m/z, ($C_{31}H_{38}N_2O_6$+H) | |
|---|---|
| Calcd.: | 535.2808 |
| Found : | 535.2826 |

$^1$H-NMR(300MHz, $CDCl_3$, δppm):

2.27+2.28(6H,sx2),2.37-2.60(1H,m),2.67(1H,d, J=15.0Hz),2.73(1H,dd,J=10.5Hz,13.7Hz),2.79-2.98 (1H,m), 3.05(1H,dd,J=2.5Hz,13.7Hz),3.18-3.36(1H, m),3.42(1H,d,J=15.0Hz),3.49(2H,s),3.74+3.75(3H,s ×2),3.86+3.88(3H, sx2),3.93(3H,s),4.34(1H,dd+m, J=2.5Hz,10.5Hz),4.65-4.74(1H,m),5.06+5.07(1H,d× 2,J=10.9Hz,10.9Hz),5.16+5.38 (1H,d×2,J=10.9Hz, 10.9Hz),6.40+6.45(1H,sx2),6.69+6.70(2H,d×2, J=8.6Hz,8.6Hz),6.77+6.79(2H,d×2,J=8.6Hz, 8.6Hz), 7.32-7.55(4H,m)

## Claims

1. A compound represented by general formula (I):

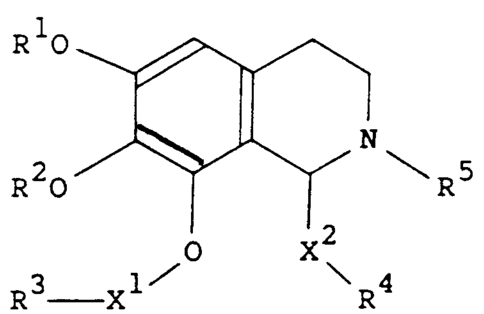

(I)

wherein each of $R^1$ and $R^2$ independently represents a $C_1$ to $C_6$ alkyl group or both are combined together to form a methylene group;

$X^1$ represents a divalent alkylene chain having 1 to 5 carbon atoms which may be substituted with a $C_1$ to $C_6$ alkyl group (one optional methylene group in which alkylene chain may be replaced by one group selected from the group consisting of an oxy group, a thio group, a sulfinyl group, a sulfonyl group or a group shown by formula: $-NR^6-$ wherein $R^6$ represents a hydrogen atom or a $C_1$ to $C_6$ alkyl group); provided that the said methylene group is not the methylene group adjacent to the oxygen atom at the 8-position of the isoquinoline ring;

$X^2$ represents a divalent alkylene chain having 1 to 4 carbon atoms which may be substituted with a $C_1$ to $C_6$ alkyl group;

each of $R^3$ and $R^4$ represents independently an aryl group or a heteroaryl group, each of which group may be substituted with 1 to 3 substituents which may be the same or different and are selected from the group consisting of a $C_1$ to $C_6$ alkyl group, a $C_1$ to $C_6$ alkoxy group, a methylenedioxy group, a halogen atom, a nitro group, a hydroxy group, a cyano group, a $C_1$ to $C_6$ alkoxycarbonyl group, a $C_1$ to $C_6$ alkanoyl group, an amino group, an N-mono-$C_1$ to $C_6$ alkylamino group, an N,N-di-$C_1$ to $C_6$ alkylamino group, a carbamoyl group, an N-mono-$C_1$ to $C_6$ alkylcarbamoyl group, an N,N-di-$C_1$ to $C_6$ alkylcarbamoyl group, an amino-$C_1$ to $C_6$ alkyl group, an N-mono-$C_1$ to $C_6$ alkylamino-$C_1$ to $C_6$ alkyl group, an N,N-di-$C_1$ to $C_6$ alkylamino-$C_1$ to $C_6$ alkyl group, an N-(hydroxy-$C_1$ to $C_6$ alkyl)amino-$C_1$ to $C_6$ alkyl group, an N-$C_1$ to $C_6$-alkyl-N-(hydroxy-$C_1$ to $C_6$ alkyl)amino-$C_1$ to $C_6$ alkyl group, an N,N-di(hydroxy-$C_1$ to $C_6$ alkyl)amino-$C_1$ to $C_6$ alkyl group, an N-($C_1$ to $C_6$ alkoxy-$C_1$ to $C_6$ alkyl)amino-$C_1$ to $C_6$ alkyl group, an N-$C_1$ to $C_6$ alkyl-N-($C_1$ to $C_6$ alkoxy-$C_1$ to $C_6$ alkyl)amino-$C_1$ to $C_6$ alkyl group, an N,N-di($C_1$ to $C_6$ alkoxy-$C_1$ to $C_6$ alkyl)amino-$C_1$ to $C_6$ alkyl group and a nitrogen-containing saturated heterocyclic $C_1$ to $C_6$ alkyl group;

and

$R^5$ represents a $C_1$ to $C_6$ alkoxycarbonyl group, a $C_1$ to $C_6$ alkylsulfonyl group, a group shown by formula:

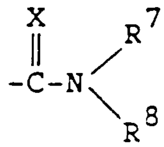

wherein each of $R^7$ and $R^8$ independently represents a hydrogen atom or a $C_1$ to $C_6$ alkyl group which may be substituted with 1 or 2 substituents, which may be the same or different and are selected from the group consisting of a hydroxy group and a $C_1$ to $C_6$ alkoxy group, or both are combined together with the nitrogen atom adjacent thereto to form a saturated nitrogen-containing heterocyclic group; and X represents an oxygen atom or a sulfur atom, or

a $C_1$ to $C_6$ alkanoyl group which may be substituted with 1 or 2 substituents, which may be the same or different and are selected from the group consisting of a $C_1$ to $C_6$ alkylsulfinyl group, a group shown by formula: $R^9S$- wherein $R^9$ represents a hydrogen atom, a $C_1$ to $C_6$ alkyl group, a $C_1$ to $C_6$ alkanoyl group, a carbamoyl group, an N-mono-$C_1$ to $C_6$ alkylcarbonyl group, an N,N-di-$C_1$ to $C_6$ alkylcarbamoyl group or a $C_1$ to $C_6$ alkoxycarbonyl group; a group shown by formula: $R^{10}O$- wherein $R^{10}$ represents a hydrogen atom, a $C_1$ to $C_6$ alkyl group or a $C_1$ to $C_6$ alkanoyl group which may be substituted with a hydroxy group; and a group shown by formula:

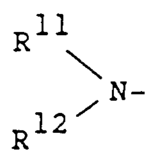

wherein each $R^{11}$ and $R^{12}$ independently represents a hydrogen atom, a $C_1$ to $C_6$ alkyl group or a $C_1$ to $C_6$ alkanoyl group, or both are combined together to form a 5- to 7-membered nitrogen-containing saturated heterocyclic ring having 3 to 6 carbon atoms together with the nitrogen atom adjacent thereto, wherein one methylene group not adjacent to the nitrogen atom for forming the ring may be replaced by an oxy group or a thio group and one methylene group adjacent to the nitrogen atom may be replaced by a carbonyl group, and
a pharmaceutically acceptable salt thereof.

2. A compound or a pharmaceutically acceptable salt thereof according to claim 1, wherein each of $R^1$ and $R^2$ independently represents a $C_1$ to $C_6$ alkyl group or both are combined together to form a methylene group; $R^4$ represents a phenyl group wherein 1 to 3 optional hydrogen atoms on the benzene ring may be replaced by 1 to 3 substituents selected from the group consisting of a $C_1$ to $C_6$ alkoxy group and a methylenedioxy group, or a pyridyl group; $X^2$ represents $CH_2$ or $CH_2CH_2$; $R^3$ represents a group represented by formula:

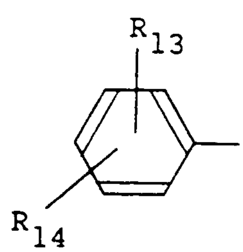

wherein $R^{13}$ represents a hydrogen atom or a $C_1$ to $C_6$ alkoxy group; and $R^{14}$ represetns an amino group, an N-mono-$C_1$ to $C_6$ alkylamino group or an N,N-di-$C_1$ to $C_6$ alkylamino group or represents a group shown by formula:

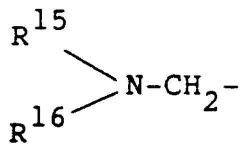

wherein each of $R^{15}$ and $R^{16}$ independently represents a hydrogen atom or a $C_1$ to $C_6$ alkyl group wherein a hydrogen atom on the carbon atom not adjacent to the nitrogen atom may be replaced by a hydroxy group or a $C_1$ to $C_6$ alkoxy group; or both $R^{15}$ and $R^{16}$ are combined together with the nitrogen atom adjacent thereto to form a 5- to 7-membered nitrogen-containing saturated heterocyclic ring having 3 to 6 carbon atoms and in this case, one optional methylene group not adjacent to the nitrogen atom may be replaced by one group selected from the group consisting of an oxy group, a thio group, a sulfinyl group, a sulfonyl group or a group shown by formula: $-NR^{17}-$ wherein $R^{17}$ represents a hydrogen atom or a $C_1$ to $C_6$ alkyl group, or

a pyridyl group wherein 1 or 2 hydrogen atoms on the pyridine ring may be replaced by 1 or 2 substituents selected from the group consisting of a $C_1$ to $C_6$ alkoxy group and an N,N-di-$C_1$ to $C_6$ alkylaminomethyl group.

3. A compound or a pharmaceutically acceptable salt thereof according to claim 1 for use as a pharmaceutically active ingredient.

4. A pharmaceutical composition, which comprises a compound or a pharmaceutically acceptable salt thereof according to claim 1, as an active ingredient.

5. A pharmaceutical composition according to claim 4, which is for the treatment of arrhythmia, myocardiac infarction or angina pectoris.

6. Use of a compound or a pharmaceutically acceptable salt thereof according to claim 1 for the preparation of a pharmaceutical composition for the treatment of arrhythmia, myocardiac infarction or angina pectoris.

**Patentansprüche**

1. Verbindung der allgemeinen Formel (I):

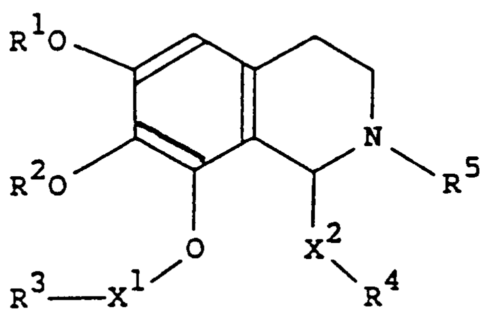

(I)

worin bedeuten:

**$R^1$** und **$R^2$** jeweils unabhängig voneinander eine $C_1$-$C_6$-Alkylgruppe oder beide zusammen eine Methylengruppe;
**$X^1$** eine zweiwertige Alkylenkette mit 1 bis 5 Kohlenstoffatom(en), die durch eine $C_1$-$C_6$-Alkylgruppe substituiert sein kann (eine beliebige Methylengruppe in der Alkylenkette kann durch eine Gruppe, ausgewählt aus einer Sauerstoffgruppe, einer Thiogruppe, einer Sulfinylgruppe, einer Sulfonylgruppe oder einer Gruppe der Formel $-NR^6-$ mit $R^6$ gleich einem Wasserstoffatom oder einer $C_1$-$C_6$-Alkylgruppe ersetzt sein), wobei gilt, daß es sich bei der betreffenden Methylengruppe nicht um die dem Sauerstoffatom in 8-Stellung des Isochinolinringes benachbarte Methylengruppe handelt;
**$X^2$** eine zweiwertige Alkylenkette mit 1 bis 4 Kohlenstoffatom(en), die durch eine $C_1$-$C_6$-Alkylgruppe substituiert sein kann;
**$R^3$** und **$R^4$** jeweils unabhängig voneinander eine Arylgruppe oder eine Heteroarylgruppe, von denen jede durch 1

bis 3 Substituent(en), die gleich oder verschieden sein können, ausgewählt aus einer $C_1$-$C_6$-Alkylgruppe, einer $C_1$-$C_6$-Alkoxygruppe, einer Methylendioxygruppe, einem Halogenatom, einer Nitrogruppe, einer Hydroxygruppe, einer Cyanogruppe, einer $C_1$-$C_6$-Alkoxycarbonylgruppe, einer $C_1$-$C_6$-Alkanoylgruppe, einer Aminogruppe, einer N-Mono-$C_1$-$C_6$-alkylaminogruppe, einer N,N-Di-$C_1$-$C_6$-alkylaminogruppe, einer Carbamoylgruppe, einer N-Mono-$C_1$-$C_6$-alkylcarbamoylgruppe, einer N,N-Di-$C_1$-$C_6$-alkylcarbamoylgruppe, einer Amino-$C_1$-$C_6$-alkylgruppe, einer N-Mono-$C_1$-$C_6$-alkylamino-$C_1$-$C_6$-alkylgruppe, einer N,N-Di-$C_1$-$C_6$-alkylamino-$C_1$-$C_6$-alkylgruppe, einer N-(Hydroxy-$C_1$-$C_6$-alkyl) amino-$C_1$-$C_6$-alkylgruppe, einer N-$C_1$-$C_6$-Alkyl-N-(hydroxy-$C_1$-$C_6$-alkyl)amino-$C_1$-$C_6$-alkylgruppe, einer N,N-Di(hydroxy-$C_1$-$C_6$-alkyl)amino-$C_1$-$C_6$-alkylgruppe, einer N-($C_1$-$C_6$-Alkoxy-$C_1$-$C_6$-alkyl) amino-$C_1$-$C_6$-alkylgruppe, einer N-$C_1$-$C_6$-Alkyl-N-($C_1$-$C_6$-alkoxy-$C_1$-$C_6$-alkyl)amino-$C_1$-$C_6$-alkylgruppe, einer N,N-Di($C_1$-$C_6$-alkoxy-$C_1$-$C_6$-alkyl)amino-$C_1$-$C_6$-alkylgruppe und einer stickstoffhaltigen gesättigten heterocyclischen $C_1$-$C_6$-Alkylgruppe, substituiert sein kann und

**$R^5$** eine $C_1$-$C_6$-Alkoxycarbonylgruppe, eine $C_1$-$C_6$-Alkylsulfonylgruppe, eine Gruppe der Formel:

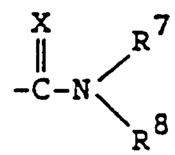

worin $R^7$ und $R^8$ jeweils unabhängig voneinander ein Wasserstoffatom oder eine $C_1$-$C_6$-Alkylgruppe, die mit 1 oder 2 Substituent(en), die gleich oder verschieden sein können und unter Hydroxygruppen und $C_1$-$C_6$-Alkoxygruppen ausgewählt sind, substituiert sein können, oder zusammen mit dem benachbarten Stickstoffatom eine gesättigte stickstoffhaltige heterocyclische Gruppe darstellen und X für ein Sauerstoffatom oder ein Schwefelatom steht, oder eine $C_1$-$C_6$-Alkanoylgruppe, die durch 1 oder 2 Substituent(en), die gleich oder verschieden sein können und unter $C_1$-$C_6$-Alkylsulfinylgruppen, Gruppen der Formel $R^9S$- mit $R^9$ gleich einem Wasserstoffatom, einer $C_1$-$C_6$-Alkylgruppe, einer $C_1$-$C_6$-Alkanoylgruppe, einer Carbamoylgruppe, einer N-Mono-$C_1$-$C_6$-alkylcarbonylgruppe, einer N,N-Di-$C_1$-$C_6$-alkylcarbamoylgruppe oder einer $C_1$-$C_6$-Alkoxycarbonylgruppe, Gruppen der Formel $R^{10}O$- mit $R^{10}$ gleich einem Wasserstoffatom, einer $C_1$-$C_6$-Alkylgruppe oder einer $C_1$-$C_6$-Alkanoylgruppe, die mit einer Hydroxygruppe substituiert sein kann, und Gruppen der Formel

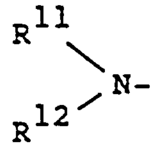

worin $R^{11}$ und $R^{12}$ unabhängig voneinander jeweils ein Wasserstoffatom, eine $C_1$-$C_6$-Alkylgruppe oder eine $C_1$-$C_6$-Alkanoylgruppe, oder zusammen mit dem benachbarten Stickstoffatom einen 5-7-gliedrigen stickstoffhaltigen gesättigten heterocyclischen Ring mit 3 bis 6 Kohlenstoffatomen darstellen, wobei eine nicht dem Stickstoffatom zur Bildung des Rings benachbarte Methylengruppe durch eine Oxogruppe oder eine Thiogruppe und eine dem Stickstoffatom benachbarte Methylengruppe durch eine Carbonylgruppe ersetzt sein können, ausgewählt sind, substituiert sein kann, und ein pharmazeutisch akzeptables Salz derselben.

**2.** Verbindung oder ein pharmazeutisch akzeptables Salz derselben nach Anspruch 1, wobei $R^1$ und $R^2$ unabhängig voneinander jeweils für eine $C_1$-$C_6$-Alkylgruppe stehen oder zusammen eine Methylengruppe bilden; $R^4$ eine Phenylgruppe, bei der 1 bis 3 beliebige(s) Wasserstoffatom(e) am Benzolring durch 1 bis 3 Substituent(en), ausgewählt aus einer $C_1$-$C_6$-Alkoxygruppe und einer Methylendioxygruppe ersetzt sein kann (können), oder eine Pyridylgruppe darstellt; $X^2$ $CH_2$ oder $CH_2CH_2$ entspricht und $R^3$ für eine Gruppe der Formel

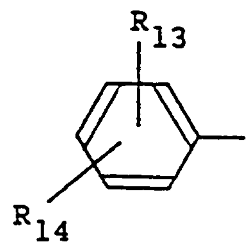

mit $R^{13}$ gleich einem Wasserstoffatom oder einer $C_1$-$C_6$-Alkoxygruppe und $R^{14}$ gleich einer Aminogruppe, einer N-Mono-$C_1$-$C_6$-alkylaminogruppe oder einer N,N-Di-$C_1$-$C_6$-alkylaminogruppe oder einer Gruppe der Formel:

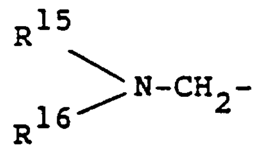

in welcher $R^{15}$ und $R^{16}$ unabhängig voneinander jeweils einem Wasserstoffatom oder einer $C_1$-$C_6$-Alkylgruppe entsprechen, wobei ein Wasserstoffatom an einem nicht dem Stickstoffatom benachbarten Kohlenstoffatom durch eine Hydroxygruppe oder eine C1-C6-Alkoxygruppe ersetzt sein kann, oder $R^{15}$ und $R^{16}$ zusammen mit dem benachbarten Stickstoffatom einen 5- bis 7-gliedrigen stickstoffhaltigen 3 bis 6 Kohlenstoffatome enthaltenden gesättigten heterocyclischen Ring bilden, wobei in diesem Falle eine beliebige nicht dem Stickstoffatom benachbarte Methylengruppe durch eine Gruppe, ausgewählt aus einer Oxygruppe, einer Thiogruppe, einer Sulfinylgruppe, einer Sulfonylgruppe oder einer Gruppe der Formel:
$-NR^{17}-$ mit $R^{17}$ gleich einem Wasserstoffatom oder einer $C_1$-$C_6$-Alkylgruppe, ersetzt sein kann, oder
eine Pyridylgruppe, in welcher 1 oder 2 Wasserstoffatom(e) am Pyridinring durch 1 oder 2 Substituent(en), ausgewählt aus $C_1$-$C_6$-Alkoxygruppen und N,N-Di-$C_1$-$C_6$-alkylaminomethylgruppen, ersetzt sein kann (können), steht.

3. Eine Verbindung oder ein pharmazeutisch akzeptables Salz derselben nach Anspruch 1 zur Verwendung als pharmazeutisch aktiver Bestandteil.

4. Arzneimittelzubereitung, enthaltend eine Verbindung oder ein pharmazeutisch akzeptables Salz derselben nach Anspruch 1 als aktiven Bestandteil.

5. Arzneimittelzubereitung nach Anspruch 4 zur Behandlung von Arrhythmie, Herzinfarkt oder Angina Pectoris.

6. Verwendung einer Verbindung oder eines pharmazeutisch akzeptablen Salzes derselben nach Anspruch 1 zur Herstellung einer Arzneimittelzubereitung zur Behandlung von Arrhythmie, Herzinfarkt oder Angina Pectoris.

## Revendications

1. Composé représenté par la formule générale (I)

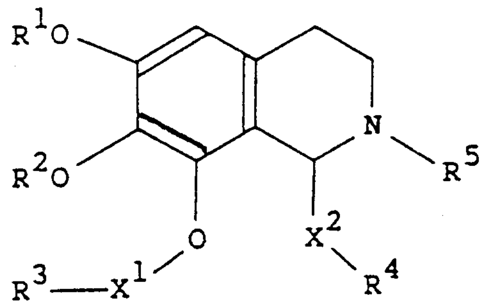

(I)

dans laquelle $R^1$ et $R^2$ représentent chacun, indépendamment l'un de l'autre, un groupement alkyle en $C_1$-$C_6$ ou sont combinés ensemble pour former un groupement méthylène;

$X^1$ représente une chaîne alkylène bivalente à 1-5 atomes de carbone qui peut être substituée par un groupement alkyle en $C_1$-$C_6$ (un éventuel groupement méthylène dans ladite chaîne alkylène peut être remplacé par un groupement choisi dans le groupe constitué par un groupement oxy, un groupement thio, un groupement sulfinyle, un groupement sulfonyle ou un groupement représenté par la formule $-NR^6-$ dans laquelle $R^6$ est mis pour un atome d'hydrogène ou un groupement alkyle en $C_1$-$C_6$), étant spécifié que ledit groupement méthylène n'est pas le groupement méthylène voisin de l'atome d'oxygène en position 8 du noyau isoquinoléine;

$X^2$ représente une chaîne alkylène bivalente à 1-4 atomes de carbone qui peut être substituée par un groupement alkyle en $C_1$-$C_6$;

$R^3$ et $R^4$ représentent chacun, indépendamment l'un de l'autre, un groupement aryle ou un groupement hétéroaryle, dont chacun peut être substitué par 1 à 3 substituants qui peuvent être identiques ou différents et sont choisis dans le groupe constitué par un groupement alkyle en $C_1$-$C_6$, un groupement alcoxy en $C_1$-$C_6$, un groupement méthylènedioxy, un atome d'halohène, un groupement nitro, un groupement hydroxy, un groupement cyano, un groupement (alcoxy en $C_1$-$C_6$)carbonyle, un groupement alcanoyle en $C_1$-$C_6$, un groupement amino, un groupement N-mono(alkyl en $C_1$-$C_6$)amino, un groupement N,N-di(alkyl en $C_1$-$C_6$)amino, un groupement carbamoyle, un groupement N-mono(alkyl en $C_1$-$C_6$)carbamoyle, un groupement N,N-di(alkyl en $C_1$-$C_6$)carbamoyle, un groupement aminoalkyle en $C_1$-$C_6$, un groupement N-mono(alkyl en $C_1$-$C_6$)aminoalkyle en $C_1$-$C_6$, un groupement N,N-di(alkyl en $C_1$-$C_6$)aminoalkyle en $C_1$-$C_6$, un groupement N-(hydroxyalkyl en $C_1$-$C_6$)aminoalkyle en $C_1$-$C_6$, un groupement N-(alkyl en $C_1$-$C_6$)-N-(hydroxyalkyl en $C_1$-$C_6$)aminoalkyle en $C_1$-$C_6$, un groupement N,N-di(hydroxyalkyl en $C_1$-$C_6$)aminoalkyle en $C_1$-$C_6$, un groupement N-(alcoxy en $C_1$-$C_6$-alkyl en $C_1$-$C_6$)aminoalkyle en $C_1$-$C_6$, un groupement N-(alkyl en $C_1$-$C_6$)-N-(alcoxy en $C_1$-$C_6$-alkyl en $C_1$-$C_6$)aminoalkyle en $C_1$-$C_6$, un groupement N,N-di(alcoxy en $C_1$-$C_6$-alkyl en $C_1$-$C_6$)aminoalkyle en $C_1$-$C_6$, un groupement N,N-di(alcoxy en $C_1$-$C_6$-alkyl en $C_1$-$C_6$)aminoalkyle en $C_1$-$C_6$ et un groupement alkyle en $C_1$-$C_6$ hétérocyclique saturé contenant de l'azote; et

$R^5$ représente un groupement (alcoxy en $C_1$-$C_6$)carbonyle, un groupement (alkyl en $C_1$-$C_6$)sulfonyle, un groupement représenté par la formule

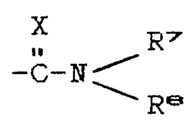

dans laquelle $R^7$ et $R^8$ représentent chacun, indépendamment l'un de l'autre, un atome d'hydrogène ou un groupement alkyle en $C_1$-$C_6$ qui peut être substitué par 1 ou 2 substituants qui peuvent être identiques ou différents et sont choisis dans le groupe constitué par un groupement hydroxy et un groupement alcoxy en $C_1$-$C_6$, ou bien $R^7$ et $R^8$ sont combinés ensemble, avec l'atome d'azote qui les relie, pour former un groupement hétérocyclique saturé contenant de l'azote; et X représente un atome d'oxygène ou un atome de soufre, ou

un groupement alcanoyle en $C_1$-$C_6$ qui peut être substitué par 1 ou 2 substituants qui peuvent être identiques ou différents et sont choisis dans le groupe constitué par un groupement (alkyl en $C_1$-$C_6$)sulfinyle, un groupement représenté par la formule $R^9S-$ (où $R^9$ représente un atome d'hydrogène, un groupement alkyle en $C_1$-$C_6$, un groupement alcanoyle en $C_1$-$C_6$, un groupement carbamoyle, un groupement N-mono(alkyl en $C_1$-$C_6$)carbo-

nyle, un groupement N,N-di(alkyl en $C_1$-$C_6$)carbamoyle ou un groupement (alcoxy en $C_1$-$C_6$)carbonyle); un groupement représenté par la formule $R^{10}O$- (où $R^{10}$ représente un atome d'hydrogène, un groupement alkyle en $C_1$-$C_6$ ou un groupement alcanoyle en $C_1$-$C_6$ qui peut être substitué par un groupement hydroxy); et un groupement représenté par la formule

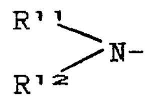

dans laquelle $R^{11}$ et $R^{12}$ représentent chacun, indépendamment l'un de l'autre, un atome d'hydrogène, un groupement alkyle en $C_1$-$C_6$ ou un groupement alcanoyle en $C_1$-$C_6$, ou bien ils sont combinés ensemble pour former un noyau hétérocyclique saturé à 5-7 chaînons contenant de l'azote, comportant 3 à 6 atomes de carbone conjointement à l'atome d'azote adjacent, dans lequel un groupement méthylène non voisin de l'atome d'azote entrant dans la constitution du noyau peut être remplacé par un groupement oxy ou un groupement thio et un groupement méthylène voisin de l'atome d'azote peut être remplacé par un groupement carbonyle, et

ses sels pharmaceutiquement acceptables.

2. Composé ou ses sels pharmaceutiquement acceptables selon la revendication 1, dans lequel $R^1$ et $R^2$ représentent chacun, indépendamment l'un de l'autre, un groupement alkyle en $C_1$-$C_6$ ou sont combinés ensemble pour former un groupement méthylène; $R^4$ représente un groupement phényle dans lequel 1 à 3 atomes d'hydrogène au choix sur le noyau benzène peuvent être remplacés par 1 à 3 substituants choisis dans le groupe constitué par un groupement alcoxy en $C_1$-$C_6$ et un groupement méthylènedioxy, ou un groupement pyridyle; $X^2$ représente $CH_2$ ou $CH_2CH_2$; $R^3$ représente un groupement représenté par la formule

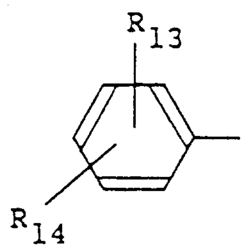

dans laquelle $R^{13}$ représente un atome d'hydrogène ou un groupement alcoxy en $C_1$-$C_6$; et $R^{14}$ représente un groupement amino, un groupement N-mono(alkyl en $C_1$-$C_6$)amino ou un groupement N,N-di(alkyl en $C_1$-$C_6$)amino, ou il représente un groupement représenté par la formule

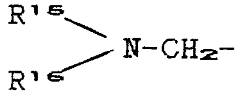

dans laquelle $R^{15}$ et $R^{16}$ représentent chacun, indépendamment l'un de l'autre, un atome d'hydrogène ou un groupement alkyle en $C_1$-$C_6$ dans lequel un atome d'hydrogène sur l'atome de carbone non voisin de l'atome d'azote peut être remplacé par un groupement hydroxy ou un groupement alcoxy en $C_1$-$C_6$; ou bien $R^{15}$ et $R^{16}$ sont combinés ensemble, avec l'atome d'azote qui les relie, pour former un noyau hétérocyclique saturé à 5-7 chaînons contenant de l'azote et 3 à 6 atomes de carbone et, dans ce cas, un éventuel groupement méthylène non voisin de l'atome d'azote peut être remplacé par un groupement choisi dans le groupe constitué par un groupement oxy, un groupement thio, un groupement sulfinyle, un groupement sulfonyle ou un groupement représenté par la formule -$NR^{17}$- (où $R^{17}$ représente un atome d'hydrogène ou un groupement alkyle en $C_1$-$C_6$), ou

un groupement pyridyle dans lequel 1 ou 2 atomes d'hydrogène sur le noyau pyridine peuvent être remplacés par 1 ou 2 substituants choisis dans le groupe constitué par un groupement alcoxy en $C_1$-$C_6$ et un groupement N,N-di(alkyl en $C_1$-$C_6$)aminométhyle.

3. Composé ou ses sels pharmaceutiquement acceptables selon la revendication 1, destiné à être utilisé comme ingrédient à activité pharmaceutique.

4. Composition pharmaceutique, qui comprend, comme ingrédient actif, un composé ou un de ses sels pharmaceutiquement acceptables selon la revendication 1.

5. Composition pharmaceutique selon la revendication 4, qui sert au traitement de l'arythmie, de l'infarctus du myocarde ou de l'angine de poitrine.

6. Utilisation d'un composé ou d'un de ses sels pharmaceuti-quement acceptables selon la revendication 1 pour la préparation d'une composition pharmaceutique pour le traitement de l'arythmie, de l'infarctus du myocarde ou de l'angine de poitrine.